# EUROPÄISCHE PATENTSCHRIFT

(11) **EP 3 083 603 B1**
(45) Veröffentlichungstag und Bekanntmachung des Hinweises auf die Patenterteilung: **13.09.2017**
(21) Anmeldenummer: 14814837.2
(22) Anmeldetag: 16.12.2014
(51) Int. Cl.: C07D 403/04, A61K 31/4184, A61P 5/00

(54) **BENZIMIDAZOLDERIVATE ALS EP4-LIGANDEN**
BENZIMIDAZOLYL DERIVATIVES AS EP4 LIGANDS
DÉRIVÉS DE BENZIMIDAZOLE EN TANT QUE LIGANDS DE EP4

(30) Priorität: 19.12.2013 EP 13198448
(43) Veröffentlichungstag der Anmeldung: 26.10.2016
(73) Patentinhaber: Bayer Pharma Aktiengesellschaft, 13353 Berlin (DE)
(72) Erfinder: PETERS, Olaf, 99891 Tabarz (DE); BRÄUER, Nico, 14612 Falkensee (DE); BOTHE, Ulrich, 13187 Berlin (DE); KOPPITZ, Marcus, 13467 Berlin (DE); NAGEL, Jens, 55442 Daxweiler (DE); LANGER, Gernot, 14612 Falkensee (DE)
(74) Vertreter: BIP Patents
(86) Internationale Anmeldenummer: PCT/EP2014/077955
(87) Internationale Veröffentlichungsnummer: WO 2015/091475

(56) Entgegenhaltungen:
- WO-A1-2011/102149
- WO-A1-2014/086739

## Beschreibung

Die vorliegende Erfindung betrifft neuartige Liganden des humanen Prostanoidrezeptors Subtyp EP4 und ihre Verwendung zur Herstellung eines Arzneimittels zur Behandlung und/oder Prophylaxe von Erkrankungen sowie ihre Verwendung als Arzneimittel und pharmazeutische Präparate, die die neuen Benzimidazol-5-carbonsäurederivate enthalten.

Das Krankheitsbild der Endometriose ist umfassend untersucht und beschrieben, wenn auch die pathogenen Mechanismen noch nicht vollständig bekannt sind. Charakteristisch für Endometriose ist eine persistierende Ansiedlung von Endometriumsgewebe außerhalb der Uterushöhle, die zur Bildung typischer Endometrioseherde führt. Diese Läsionen können in unterschiedlicher Verteilung und Ausprägung im muskulären Bereich des Uterus (Endometriosis interna, Adenomyose), an verschiedenen Stellen des Bauchraums, z.B. den Ligamenten, am parietalen Peritoneum des Douglas Raumes (peritoneale Endometriose), der Darmwand, am Ovar (sogenannte Endometrioma) oder rektovaginal (rektovaginale, häufig auch tiefinfiltrierende, Endometriose) nachgewiesen werden. Das neu angesiedelte Gewebe behält wesentliche Eigenschaften des Ursprungsgewebes (Uterus, Endometrium). Die Endometriose hat einen entzündlichen Charakter und äußert sich häufig durch verschiedene Formen von Unterleibschmerzen. Man geht davon aus, dass 10-20% der Frauen im reproduktiven Alter von Endometriose betroffen sind. Kernsymptome der Endometriose sind chronische Unterleibsschmerzen, Dysmenorrhoe, Dyspareunie, Dysurie, Blutungsstörungen und Unfruchtbarkeit. Die Symptome treten zumeist kombiniert auf.
Es wird vermutet, dass Endometriumsgewebe durch retrograde Menstruation über den Eileiter in den Peritonealraum gelangt und sich anschließend im Peritonealgewebe einnisten kann und zu den bei der Endometriose beobachteten Läsionen führt (Stratton & Berkley, Giudice 2010). Diese Läsionen rufen im Verlaufe der Erkrankung fortschreitende lokale Entzündungen hervor und sind durch Hochregulation von COX₂ Enzym und eine vermehrte Prostaglandinsynthese gekennzeichnet (Chishima et al 2002; Sales and & Jabbour 2003).

Die Wirkungen der Prostaglandine werden über spezifische G-Protein-gekoppelten Rezeptoren vermittelt, die in der Zellmembran lokalisiert sind. Von besonderem Interesse ist das Prostaglandin E2 (PGE2), das unterschiedlichste zelluläre Wirkungen erzielt, indem es an funktionell verschiedene Rezeptorsubtypen, nämlich EP1, EP2, EP3 und EP4, bindet.

Der Prostaglandin Rezeptorsubtyp EP4 (PTGER4) ist einer der 4 humanen Rezeptoren, die durch endogen gebildetes Prostaglandin E2 (PGE2) aktiviert werden. EP4 gehört zur Familie der membranständigen G-Protein gekoppelten Rezeptoren (GPCR) und aktiviert über die Kopplung an ein heterotrimeres G-Protein (Gs) in erster Linie die Bildung des intrazellulären Signalmoleküls cAMP mittels Stimulation membranständiger Adenylatzyklasen (Yokoyama et al, 2013; The Prostanoid EP4 Receptor and Its Signaling Pathway; Utako Yokoyama, Kousaku Iwatsubo, Masanari Umemura,Takayuki Fujita and Yoshihiro Ishikawa; Pharmacological Reviews, July 2013, Vol. 65, No 3, 1010-1052; (http://pharmrev.aspetjournals.org/content/65/3/1010.long#title15*)*

Die Expression des Rezeptors wurde auf periphären Nervenendigungen von Nozizeptoren, auf Makrophagen und Neutrophilen nachgewiesen. Für diese Zelltypen wurde eine große Bedeutung im Zusammenhang mit Endometriose nachgewiesen. Es wird angenommen, dass die lokale Entzündung der Endometriose-Läsionen einen wesentlichen Beitrag zu der Genese der beobachteten Schmerzsymptome leistet (Stratton & Berkley 2010; Giudice 2010).

Derzeitige Therapieansätze für die Behandlung einer diagnostizierten Endometriose sind sehr eingeschränkt.

So kann die Endometriose durch ein operatives Entfernen der endometriotischen Läsionen in einem laparoskopischen Eingriff behandelt werden. Dabei werden Endometrioseherde operativ mit Hitze (Elektrokauterisation) oder durch Ausschneiden (Exstirpation) entfernt. Zusätzlich können hierbei eventuell vorhandene Verwachsungen gelöst, Endometriosezysten entfernt und bei Kinderwunsch die Durchgängigkeit der Eileiter mittels Chromopertubation geprüft werden. Die Rückfallquote nach einem solchen Eingriff ist allerdings sehr hoch (25-30%). Die Hysterektomie, also das komplette Entfernen des Uterus, besteht in solchen besonders hartnäckigen Fällen als finale Therapieoption.

Bei besonders schweren Erkrankungen bringt manchmal erst die Entfernung beider Eierstöcke und Eileiter (beidseitige Salpingo-Oophorektomie, Adnexektomie) eine definitive Therapie.

Die Regelschmerzen und verlängerte bzw. verstärkte Blutungen, die von einer Endometriose in der Gebärmuttermuskulatur (adenomyosis uteri) ausgehen, können auch mit einer Gebärmutterentfernung erfolgreich behandelt werden.

Diese Eingriffe führen jedoch zu Unfruchtbarkeit und einer verfrühten Menopause mit den damit verbundenen Problemen, weshalb der Nutzen gut gegen die Nachteile abgewogen werden muss.

Neben invasiven operativen Eingriffen kann auch eine medikamentöse Therapie in Betracht gezogen werden. Diese kommt häufig bei großflächigem, eventuell nicht komplett operablem Befall, zur Anwendung, wird aber auch bei leichter bis mittlerer Erkrankung eingesetzt. Neben vorwiegend symptomatischer Schmerztherapie mit nichtsteroidalen Analgetika (NSAID), kommen bisher im Prinzip vier Substanzgruppen zur Anwendung:
(a) kombinierte orale Kontrazeptiva (bestehend aus Estrogen und Gestagen) (OCs)
(b) Gestagene
(c) GnRH-Analoge (GnRH = Gonadotropin-Releasing-Hormone) und
(d) Danazol®

Die kombinierten oralen Kontrazeptiva (a) regulieren den Zyklusverlauf und reduzieren die Menstruationsstärke. Daraus folgt vermutlich deren Wirksamkeit in Endometriose-Patientinnen. Allerdings ist die Patientinnen-Zufriedenheit mit dieser Behandlungsform gering, was vermutlich auf Nebenwirkungen durch Beeinflussung des Hormonhaushaltes und eine unzulängliche Schmerzkontrolle zurück zu führen ist. Zudem weisen neue Studien darauf hin, dass eine langjährige Verwendung der hormonellen Wirkstoffe mit erhöhter Rate an tiefinfiltrierender Endometriose assoziiert zu sein scheint, einer besonders schmerzhaften Endometrioseform.

Der Einsatz von OCs bei der Behandlung der Endometriose wird auch in der Patentliteratur beschrieben. So offenbart die EP 1257280, dass mikronisiertes Drospirenon zur Behandlung der Endometriose geeignet ist. Es wird dort in Absatz [0045] beschrieben, dass Zusammensetzungen von Drospirenon mit einem niedrigen Gehalt an Estrogen oder auch ohne jegliches Estrogen u.a. zur Behandlung der Endometriose geeignet sind. Dieses erklärt sich aus der gestagenen Eigenschaft des Drospirenons. In der EP1257280 werden Mengen von 0,5 bis 10 mg Drospirenon als wirksam beschrieben. Über die Dauer der Behandlung der Endometriose mit Drospirenon ist in dieser Schrift nichts offenbart.

In der WO2008/107373 werden Mineralokortikoid-Rezeptor-Antagonisten zur Herstellung eines Arzneimittels für die Behandlung der Endometriose beschrieben. Neben der Verwendung von Verbindungen mit reiner antimineralokortikoiden Wirkung werden dort auch Verbindungen vorgeschlagen, die darüber hinaus auch eine Wirkung am Progesteron-Rezeptor, am Estrogen-Rezeptor, am Glucokortikoid-Rezeptor und/oder am Androgen-Rezeptor zeigen. Insbesondere die in der WO2008/107373 offenbarten Verbindungen Spironolacton und das zuvor genannte Drospirenon weisen auch eine gestagene Wirkung auf.

Die in der WO2008/107373 genannte Verbindung Eplerenon als reiner MR Antagonist zeigt eine relativ schwache *in vitro* Potenz. Bevorzugt sind MR Antagonisten, die in *in vitro* Transaktivierungsassays eine mindestens 10-fach geringere IC₅₀ verglichen mit Eplerenon aufweisen.

Gestagene (b) werden ebenfalls bei Endometriose eingesetzt. Ansatzpunkt ist hier zum einen die Unterdrückung der Funktion der Eierstöcke und zum anderen das Herbeiführen der terminalen Differenzierung des Endometriums, der Dezidualisierung, die letztlich zum Gewebsuntergang führt.

Die Gestagene täuschen dem Körper eine Schwangerschaft vor und schaffen so eine veränderte hormonelle Situation. Es findet kein Eisprung mehr statt und die Gebärmutterschleimhaut bildet sich zurück. In der Regel lassen die Endometriosebeschwerden dann innerhalb von 6 bis 8 Wochen nach.

Depot-MPA (Medroxyprogesteronacetat) und Visanne© (Dienogest) sind für die Endometriosebehandlung zugelassen. Eine deutliche analgetische Wirkung von Visanne© tritt erst nach mehreren Wochen Behandlung ein (Petraglia et al 2011). Es gibt keine Hinweise auf die allgemein erwünschte zügige Schmerzlinderung. Bei MPA kann es aufgrund der antiestrogenen Wirkung der Verbindung bereits nach einer Anwendungsdauer von 6 Monaten zu einer Verringerung der Knochenmasse kommen. Es soll deshalb keinesfalls über einen längeren Zeitraum als von 2 Jahren angewendet werden. Unter Behandlung mit Visanne kann es als Nebenwirkung der gestagenen Eigenschaften zu einer unerwünschten Beeinflussung des Blutungsprofils kommen. (Fachinfo-Nebenwirkungen).

Gestagene beeinflussen im Allgemeinen neben dem Hormonzyklus auch das Blutungsprofil, mit Blutungsstörungen als einer häufigen Nebenwirkung von Gestagenen. Das betrifft auch Substanzen, die an anderen Hormonrezeptoren aktiv sind und gleichzeitig eine gestagene Aktivität aufweisen, wie beispielsweise Spironolacton. Durch eine fehlerhafte Angiogenese (Gefäßneubildung, ein Vorgang der zyklisch im Endometrium stattfindet) während der Dezidualisierung des Endometriums werden die Gefäßwände brüchig und es kommt zu den sogenannten Durchbruchblutungen, die unabhängig von einer Menstruationsblutung stattfinden und charakteristisch für die chronische Behandlung mit Gestagenen sind.

Die Gonadotropin-Releasing-Hormon-Analoga (GnRH) (c) stellen derzeit den Goldstandard der zugelassenen Therapeutika gegen alle Stadien der Endometriose dar. GnRH-Analoga blockieren die Hirnanhangsdrüse vollständig. Der Menstruationszyklus findet nicht mehr statt. Diese Substanzen versetzen den Körper der Frau somit vorübergehend künstlich in die Wechseljahre und das Endometriosegewebe kann daher auch nicht mehr mitbluten. Das Gewebe wird hypotroph.

Aufgrund des Nebenwirkungsprofils ist dieser Therapieansatz jedoch ebenfalls nur für den kurzfristigen Einsatz (bis zu 6 Monaten) geeignet. So induzieren GnRH-Agonisten postmenopausale Symptome, wie Hitzewallungen (80-90%), Schlafstörungen (60-90%), Trockenheit der Scheide (30%), Kopfschmerzen (20-30%), Stimmungsveränderungen (10%) und Abnahme der Knochendichte mit einhergehendem erhöhtem Osteoporoserisiko.

Abgesehen von den genannten Nebenwirkungen stellt sich nach Beendigung der Behandlung innerhalb von zwei bis drei Monaten der normale Zyklus wieder ein. Bei über 60% der betroffenen Frauen, kehren dann auch die Beschwerden der Endometriose zurück, so dass ein erneuter Behandlungszyklus erwogen werden muss.

Aufgrund der genannten Nachteile haben GnRH-Analoga bislang keine breite Anwendung bei der Behandlung der Endometriose erlangt, wenngleich diese, die in den 70er Jahren etablierte Standardtherapie mit Danazol®, einem gestagenen Androgen, aufgrund des etwas besseren Nebenwirkungsprofils, verdrängt haben.

Danazol® (d) war das erste "klassische" Therapeutikum der Endometriose und bis in die 70er-Jahre der Goldstandard. Danazol® führt bei längerer Anwendung, ähnlich wie das männliche Geschlechtshormon Testosteron, zu einer Vermännlichung der Frau. Als weitere Nebenwirkungen treten die für Androgene bekannten Wirkungen, wie Akne, Hyperandrogenismus, Hirsutismus und (irreversible) Stimmlagenveränderung auf (Hinweis Fachinfo).

Danazol® wirkt, wie auch die GnRH-Agonisten, auf die Hirnanhangsdrüse, die für die Produktion von Hormonen verantwortlich ist, die die Eierstöcke stimulieren. Dadurch wird die Produktion von Estrogenen in den Eierstöcken eingestellt.

Es besteht daher ein dringender Bedarf an alternativen Präparaten, die eine nicht invasive Behandlung der Endometriose erlauben und die nicht die Nachteile des Standes der Technik aufweisen. Hier bietet sich die spezifische Blockade der Funktionen des humanen EP4-Rezeptors mittels geeigneter Modulatoren an.

In diesem Zusammenhang sind EP4-Antagonisten bekannt aber noch nicht als Arzneimittel zugelassen. Es sind aber EP4-Rezeptor antagoniste Eigenschaften werden für verschiedene Strukturklassen beschrieben, die sich signifikant von den erfindungsgemäßen Verbindungen unterscheiden, indem sie nicht deren Carbazolylbenzimidazol-Struktur aufweisen. So werden in den WO2005/0121508, WO2005102389 und WO2005/105733 (Pfizer) beispielsweise *N*-Benzyl-aryl-amide, *N-*Benzyl-heteroarylamide und [(1H-Benzimidazol-1-yl)phenylethyl]aryl- und [(1H-Benzimidazol-1-yl)phenylethyl]heteroaryl-sulfonylcarbamate für die Anwendung bei Schmerz, Entzündungen, Osteoarthritis und Rheumatoider Arthritis beschrieben. Pfizer beschreibt in WO2002032422, WO2002032900 und WO2003086371 auch Strukturen, die generisch Benzimidazole einschließen, jedoch an Position 2 nicht durch einen kondensierten Tricyclus, wie Carbazol substituiert sein können. Thiophen-*N*-benzylamide in WO2008017164 und WO2009020588, Indol-*N*-benzylamide in WO2007121578 und N-{[(6,8-dihydro-7H-pyrrolo[3,4-g]quinolin-7-yl)-aryl]methyl}sulfonylamide in WO2008104055 werden für nahezu das gleiche Indikationsspektrum durch Merck-Frosst adressiert. In WO2010019796 (Chemietek) werden generisch sehr breit mehrfach substituierte Heterobicyclen beansprucht, wobei die typischen Einheiten der erfindungsgemäßen Verbindungen, Carbazol und Benzimidazol, nicht in den sehr wenigen Beispielen vorkommen und tricyclische Substituenten wie das Carbazol auch generisch nicht adressiert werden. In WO2004067524 (Pharmagene Laboratories) werden Furanderivate für die Behandlung von Kopfschmerz und Migräne beschrieben, bei denen der Furanring linear mit zwei weiteren Aryl- oder Heteroaryleinheiten, jeweils mit sechs Ringatomen, verbunden ist.

EP2172447 (Astellas Pharma) beansprucht generisch in sehr breiter Weise Verbindungen, die aus zwei direkt miteinander verbundenen Heterocyclen bestehen können, von denen aber einer durch eine Aminocarbonylgruppe substituiert sein muss und die Aminogruppe durch einen Substituenten weiter substituiert sein muss, der eine Carboxylgruppe oder ein Carboxylsurrogat trägt für die Indikationen renale Insuffizienz und diabetische Nephropathie.

Es werden auch Verbindungen beschrieben, die keine EP4-Antagonisten sind, aber strukturell in Beziehung zu den erfindungsgemäßen Verbindungen stehen. US2004/0122046 (Pfizer) adressiert Carbazole, die direkt über Position 3 mit einem Heterocyclus verbunden sind, der auch Benzimidazol sein kann, als NPY-Rezeptorantagonisten zur Behandlung von Adipositas. Im Unterschied zu den erfindungsgemäßen Verbindungen ist das NH der Benzimidazoleinheit aber zwingend unsubstituiert und die beiden Sechsringe der Carbazoleinheit können keinen weiteren Substituenten tragen. WO03/000254 bzw. EP1162196 (Japan Tobacco) beansprucht in breiter Weise generisch Heterobicyclen, die direkt mit einem Heterocyclus verbunden sein können als Therapeutikum für Hepatitis C. Falls es sich bei dem Heterocyclus um ein Benzimidazol handelt, muss dieser im Unterschied zu den erfindungsgemäßen Verbindungen an Position 1 zwingend direkt durch eine Bindung mit einer Cycloalkyl- oder Cycloalkenyleinheit verbunden sein. Paratek beschreibt in WO2010/124097 substituierte Benzimidazole als Antünfektiva. Jedoch trägt das Benzimidazol anders als in den erfindungsgemäßen Verbindungen an Position 4 zwingend eine Alkylgruppe die terminal mit einer Carbonsäure-, Phosphonsäure oder Sulfonsäurefunktion bzw. deren Derivaten substituiert ist; des Weiteren ist an Position 2 als direkter cyclischer Substituent Heterocycloalkyl, nicht aber Heteroaryl zugelassen. Ausgehend vom beschriebenen Stand der Technik bestand daher keine Veranlassung, die Strukturen des Standes der Technik erfindungsgemäß abzuwandeln, um Strukturen zu erhalten, die antagonistisch am EP4-Rezeptor wirken.

Aufgabe der vorliegenden Erfindung ist es, *in vivo* verfügbare und somit wirksame und stabile Verbindungen, als potente und selektive EP4-Rezeptorliganden mit antagonistischer Wirkung, bereitzustellen, die sich daher zur Behandlung und/oder Prophylaxe von Erkrankungen wie z.B. Endometriose eignen.

Diese Aufgabe wurde durch die Verbindungen der allgemeinen Formel I gelöst, 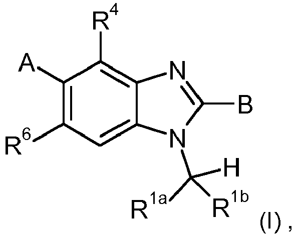 in der
- A: für Wasserstoff, Brom, Cyano, Formyl, C₁-C₃-Alkyl, 4-6-gliedriges Heterocyclyl, 5-6-gliedriges Heteroaryl, R¹¹O-C(=O)-(CH₂)ₚ-, R⁵R^{5'}N-C(=O)-(CH₂)ₚ-, R¹¹O-S(=O)₂-(CH₂)ₚ-, R⁵R^{5'}N-S(=O)₂-(CH₂)ₚ-, (C₁-C₆-Alkyl)-S(=O)₂-, (C₁-C₆-Alkyl)-S(=O)(=NH)- oder (C₃-C₆-Cycloalkyl)-S(=O)(=NH)- steht,
wobei Heteroaryl bevorzugt aus der Gruppe bestehend aus Pyrazolyl, Imidazolyl, Triazolyl, Tetrazolyl, Oxazolyl, Isoxazolyl und Oxadiazolyl ausgewählt wird,
   und
wobei Alkylreste gegebenenfalls einfach oder mehrfach, gleich oder verschieden substituiert sein können mit Halogen oder Hydroxy,
   und
wobei Heterocyclylreste gegebenenfalls einfach oder mehrfach, gleich oder verschieden substituiert sein können mit C₁-C₃-Alkyl, Trifluormethyl oder Hydroxy,
   und
wobei Heterarylreste gegebenenfalls einfach oder mehrfach, gleich oder verschieden substituiert sein können mit C₁-C₃-Alkyl, Trifluormethyl oder Hydroxy,
- B: aus den folgenden Strukturen ausgewählt wird, 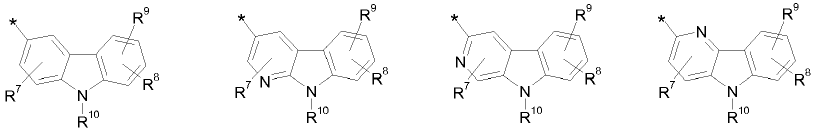 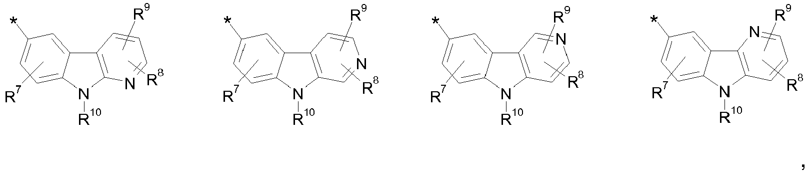 worin * die Verknüpfungsstelle im Molekül bedeutet,
- R^{1a}, R^{1b}: unabhängig voneinander für Wasserstoff, Cyano, C₁-C₅-Alkyl, C₂-C₅-Alkenyl, C₂-C₅-Alkinyl, (C₃-C₆-Cycloalkyl)-(CH₂)ₘ-, (4-6-gliedriges Heterocyclyl)-(CH₂)ₙ-, (C₁-C₅-Alkoxy)-(C₁-C₃-alkyl)-, (C₃-C₆-Cycloalkoxy)-(C₁-C₃-alkyl)-, H₂N-(C₁-C₃-Alkyl)-, (C₁-C₅-Alkyl)NH-(C₁-C₃-alkyl)- oder (C₁-C₅-Alkyl)₂N-(C₁-C₃-alkyl)- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
   und
wobei Alkylreste, Cycloalkylreste und Heterocyclylreste gegebenenfalls einfach oder mehrfach, gleich oder verschieden substituiert sein können mit Halogen, C₁-C₅-Alkyl, Hydroxy, C(=O)OH, HO-C(=O)-(C₁-C₅-Alkyl)-, (C₁-C₅-Alkyl)O-C(=O)-(C₁-C₅-alkyl)- oder (C₁-C₅-Alkyl)-S(=O)₂-,
- R⁴: für Wasserstoff, Fluor, Chlor, C₁-C₂-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen oder Hydroxy,
- R⁵, R^{5'}: unabhängig voneinander für Wasserstoff, C₁-C₇-Alkyl, (C₁-C₇-Alkoxy)-(C₂-C₅-alkyl)-, (4-6-gliedriges Heterocyclyl)-(CH₂)ᵣ-, (C₁-C₇-Alkyl)-C(=O)-, (C₃-C₇-Cycloalkyl)-C(=O)-, Phenyl-(CH₂)ᵣ-C(=O)-, Pyridyl-(CH₂)ᵣ-C(=O)-, (C₁-C₇-Alkyl)-S(=O)₂-, (C₃-C₇-Cycloalkyl)-S(=O)₂-, Phenyl-(CH₂)ᵣ-S(=O)₂- oder Pyridyl-(CH₂)ᵣ-S(=O)₂- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
   und
wobei R⁵ und R^{5'} unabhängig voneinander gegebenenfalls einfach oder mehrfach, gleich oder verschieden substituiert sein können mit Halogen, Hydroxy, C₁-C₂-Alkyl, Trifluoromethyl, (C₁-C₅-Alkyl)NH-, (C₁-C₅-Alkyl)₂N-, C₁-C₂-Alkoxy oder Trifluormethoxy,
oder
- R⁵, R^{5'}: zusammen mit dem Stickstoffatom, an das sie gebunden sind, einen 4-6-gliedrigen heterocyclischen Ring bilden, der optional, einfach oder mehrfach, gleich oder verschieden mit Oxo, Hydroxy, Carboxy, C₁-C₂-Alkyl oder
C₁-C₂-Alkoxy substituiert sein kann,
wobei ein 6-gliedriger heterocyclischer Ring optional als weiteres Ringatom ein Heteroatom, das aus der Gruppe bestehend aus O und N ausgewählt wird, enthalten kann,
- R⁶: für Wasserstoff, Fluor, Chlor, Methyl, Trifluormethyl, Methoxy oder Trifluormethoxy steht,
- R⁷: für Wasserstoff, Fluor, Chlor, Cyano, SF₅, C₁-C₃-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen,
- R⁸: für Fluor, Chlor, Brom, Cyano, SF₅, C₁-C₃-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen,
- R⁹: für Fluor, Chlor, Brom, Cyano, C₁-C₃-Alkyl oder C₁-C₂-Alkoxy steht,
wobei Alkylreste gegebenenfalls einfach oder mehrfach, gleich oder verschieden substituiert sein können mit Halogen,
oder
- R⁹: für Brom steht und gleichzeitig R⁸ für Wasserstoff steht,
- R¹⁰: für C₁-C₅-Alkyl, C₃-C₅-Alkenyl, C₃-C₅-Alkinyl, (C₃-C₆-Cycloalkyl)-(CH₂)ₙ-, (4-6-gliedriges Heterocyclyl)-(CH₂)ₙ-, oder (C₁-C₇-Alkoxy)-(C₂-C₅-alkyl)-steht,
wobei Heterocyclyl bevorzugt aus der Gruppe bestehend aus Oxetanyl, Tetrahydrofuranyl, Tetrahydropyranyl, Morpholinyl, Pyrrolidinyl und Piperidinyl ausgewählt wird,
   und
wobei Alkylreste, Cycloalkylreste und Heterocyclylreste gegebenenfalls einfach oder mehrfach, gleich oder verschieden substituiert sein können mit Halogen, C₁-C₂-Alkyl, C₁-C₂-Alkoxy oder C(=O)OH,
- R¹¹: für Wasserstoff, C₁-C₇-Alkyl, C₃-C₇-Cycloalkyl, Phenyl-(CH₂)_{q}- oder (C₁-C₇-Alkoxy)-(C₂-C₅-alkyl)- steht,
wobei Phenyl gegebenenfalls einfach oder mehrfach, gleich oder verschieden substituiert sein kann mit C₁-C₃-Alkyl, Trifluormethyl oder Hydroxy,
- m: 0, 1, 2 oder 3 ist,
- n: 0, 1, 2 oder 3 ist,
- p: 0, 1 oder 2 ist,
- q: 1, 2 oder 3 ist, und
- r: 0, 1, 2 oder 3 ist,
sowie deren Diastereomere, Enantiomere, Solvate und Salze oder Cyclodextrin-Clathrate, für die Herstellung von Arzneimitteln.

Erfindungsgemäße Verbindungen sind die Verbindungen der Formel (I) und deren Stereoisomere, Tautomere, N-Oxide, Hydrate, Salze, Solvate und Solvate der Salze, sowie die von Formel (I) umfassten, nachfolgend als Ausführungsbeispiele genannten Verbindungen und deren Stereoisomere, Tautomere, N-Oxide, Hydrate, Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchio¬metrische Zusammensetzung eines solchen Salzes, wie es nach dem jeweiligen Herstell- und/oder Reinigungsverfahren erhalten wurde, in der Regel nicht bekannt. Sofern nicht genauer spezi¬fiziert, sind daher Namens- und Struktur¬formel-Zusätze wie beispielsweise "Hydrochlorid", "Trifluoracetat", "Natrium-Salz" bzw. "x HCl", "x CF3COOH", "x Na⁺" bei solchen Salzen nicht stöchio¬metrisch zu verstehen, son¬dern haben allein deskriptiven Charak¬ter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I, Bestimmte isotopische Varianten einer erfindungsgemäßen Verbindung, wie insbesondere solche, bei denen ein oder mehrere radioaktive Isotope inkorporiert sind, können von Nutzen sein, beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Die erfindungsgemäßen Verbindungen sind neu und haben eine antagonistische Wirkung am EP4-Rezeptor und dienen unter anderem der Behandlung der Endometriose.

Im Rahmen der vorliegenden Erfindung haben die Substituenten, soweit nicht anders spezifiziert, die folgende Bedeutung:
Alkyl steht für einen linearen oder verzweigten, gesättigten, monovalenten Kohlenwasserstoffrest mit mindestens 1 und höchstens 7 Kohlenstoffatomen (C₁-C₇-Alkyl). Eine gegebenenfalls vorgenommene Einschränkung des Bereiches für die Zahl der Kohlenstoffatome lässt sich direkt aus dem Präfix vor 'Alkyl' erkennen, beispielsweise bedeutet C₁-C₃-Alkyl, dass nur Alkylgruppen mit 1, 2 oder 3 Kohlenstoffatomen zugelassen sind.
Beispielhaft seien genannt: Methyl, Ethyl, Propyl, Butyl, Pentyl, Hexyl, iso-Propyl, isoButyl, sec-Butyl, tert-Butyl, iso-Pentyl, 2-Methylbutyl, 1-Methylbutyl, 1-Ethylpropyl, 1,2-Dimethylpropyl, neo-Pentyl, 1,1-Dimethylpropyl, 4-Methylpentyl, 3-Methylpentyl, 2-Methylpentyl, 1-Methylpentyl, 2-Ethylbutyl, 1-Ethylbutyl, 3,3-Dimethylbutyl, 2,2-Dimethylbutyl, 1,1-Dimethylbutyl, 2,3-Dimethylbutyl, 1,3-Dimethylbutyl, 1,2-Dimethylbutyl. Die Alkylreste können gegebenenfalls ein- oder mehrfach durch Fluor substituiert sein.

Alkenyl und Alkinyl bezeichnen lineare oder verzweigte, ungesättigte, monovalente Kohlenwasserstoffreste, die sich von den zuvor genannten Alkylgruppen dadurch ableiten, dass der Rest mindestens zwei Kohlenstoffatome aufweist und dass eine Einfachbindung zwischen zwei mit der geeigneten Zahl von Wasserstoffatomen versehenen Kohlenstoffatomen durch eine Doppelbindung oder eine Dreifachbindung ersetzt ist.
Beispielhaft seien genannt: Vinyl, Allyl, Buten-1-yl für Alkenyl und Ethinyl, Propargyl, Pentin-1-yl für Alkinyl. Die Zahl der Kohlenstoffatome ergibt sich aus dem Präfix, z.B. bedeutet C₂-C₅-Alkenyl eine Alkenylgruppe mit 2 bis 5 Kohlenstoffatomen.

Alkoxy steht für einen linearen oder verzweigten, gesättigten Alkyletherrest der Formel Alkyl-O mit mindestens 1 und höchstens 7 Kohlenstoffatomen (C₁-C₇-Alkoxy). Beispielhaft seien genannt: Methoxy, Ethoxy, n-Propoxy, Isopropoxy, n-Butoxy, sec-Butoxy, Isobutoxy, tert-Butoxy, Pentyloxy, Hexyloxy und Heptyloxy.
Die Alkoxyreste können gegebenenfalls ein- oder mehrfach durch Fluor substituiert sein.

Alkoxyalkyl steht für einen mit Alkoxy substituierten Alkylrest, wobei Cₙ-Alkoxy-Cₘ-Alkyl bedeutet, dass dabei der Alkoxyteil n Kohlenstoffatome und der Alkylteil, über den der Rest gebunden ist, m Kohlenstoffatome aufweist. Beispielsweise bedeutet (C₁-C₅-Alkoxy)-(C₁-C₃-alkyl), dass für die Alkoxygruppe 1, 2, 3, 4 oder 5 Kohlenstoffatome und für die Alkylgruppen 1, 2 oder 3 Kohlenstoffatome zugelassen sind.
Beispielhaft seien genannt: Methoxymethyl, Ethoxymethyl, Propoxymethyl, Isopropoxymethyl, 2-Methoxyethyl, 2-Ethoxyethyl, 2-Propoxyethyl, 2-Isopropoxyethyl.

C₃-C₇-Cycloalkyl bezeichnet monocyclische Alkylreste mit 3 bis 7 Kohlenstoffatomen, wobei die Zahl der Ringatome modifiziert sein kann, wie dann in den Indizes ausgewiesen (z.B. bedeutet C₄-C₅-Cycloalkyl 4 oder 5 Ringatome).

Beispielhaft seien genannt: Cyclopropyl, Cyclobutyl, Cyclopentyl und Cyclohexyl. Die Cycloalkylreste können gegebenenfalls ein- oder mehrfach durch Fluor substituiert sein.

Cycloalkoxy steht für einen Rest C₃-C₆-Cycloalkyl-O, wobei C₃-C₆-Cycloalkyl die vorstehend angegebene Bedeutung besitzt.
Beispielhaft seien genannt: Cyclopropyloxy, Cyclobutyloxy, Cyclopentyloxy und Cyclohexyloxy.

Cycloalkoxyalkyl steht für einen mit Cycloalkoxy substituierten Alkylrest, wobei Cₙ-Cycloalkoxy-Cm-Alkyl bedeutet, dass dabei der Cycloalkoxyteil n Kohlenstoffatome und der Alkylteil, über den der Rest gebunden ist, m Kohlenstoffatome aufweist. Beispielsweise bedeutet (C₃-C₆-Cycloalkoxy)-(C₁-C₃-alkyl), dass für die Cycloalkoxygruppe 3, 4, 5 oder 6 Kohlenstoffatome und für die Alkylgruppen 1, 2 oder 3 Kohlenstoffatome zugelassen sind.
Beispielhaft seien genannt: (Cyclopropyloxy)methyl, (Cyclobutyloxy)methyl, 2-(Cyclopropyloxy)ethyl, 2-(Cyclobutyloxy)ethyl.

Heterocycloalkyl oder Heterocyclyl bezeichnet monocyclische oder bicyclische, nicht-aromatische heterocyclische Reste mit in der Regel 4 bis 10 Ringatomen, und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Die Bindungsvalenz kann an einem beliebigen Kohlenstoffatom oder an einem Stickstoffatom sein. 4-6 gliedriges Heterocyclyl bezeichnet nicht-aromatische heterocyclische Reste mit 4, 5 oder 6 Ringatomen und 1 oder 2 Heteroatomen und/oder Heterogruppen aus der Reihe N, O, S, SO, SO₂.
Beispielhaft seien genannt: Azetidinyl, Pyrrolidinyl, Piperidinyl, Piperazinyl, 1,4-Diazepanyl, Morpholinyl, Thiomorpholinyl, Oxetanyl, Tetrahydrofuranyl, Tetrahydropyranyl, 1,4-Dioxanyl, 2-Oxo-oxazolidinyl. Die Heterocyclylreste können gegebenenfalls ein- oder mehrfach durch Fluor, Hydroxy, Methoxy beziehungsweise mit Oxo substituiert sein.

Unter Halogen ist jeweils Fluor, Chlor oder Brom zu verstehen.

Heteroaryl bezeichnet ein mono- oder bicyclisches aromatisches Ringsystem, das jeweils 5 - 10 Ringatome enthalten kann und das anstelle des Kohlenstoffs ein- oder mehrere, gleiche oder verschiedene Heteroatome, wie Sauerstoff, Schwefel oder Stickstoff enthält. Die Bindungsvalenz kann an einem beliebigen Kohlenstoffatom oder an einem Stickstoffatom sein. 5-6 gliedriges Heteroaryl bezeichnet ein monocyclisches aromatisches Ringsystem, das 5 oder 6 Ringatome enthält und das anstelle des Kohlenstoffs ein- oder mehrere, gleiche oder verschiedene Heteroatome, wie Sauerstoff, Schwefel oder Stickstoff enthält. Die Bindungsvalenz kann an einem beliebigen Kohlenstoffatom oder an einem Stickstoffatom sein.
Beispielhaft seien genannt: Thienyl, Thiazolyl, Furyl, Pyrrolyl, Oxazolyl, Imidazolyl, Pyrazolyl, Isoxazolyl, Isothiazolyl, Oxadiazolyl, Triazolyl, Thiadiazolyl, Tetrazolyl, Pyridyl, Pyridazinyl, Pyrimidinyl, Pyrazinyl, Triazinyl, Phthalidyl, Thiophthalidyl, Indolyl, Isoindolyl, Indazolyl, Benzothiazolyl, Benzofuryl, Benzothienyl, Benzimidazolyl, Benzoxazolyl, Azocinyl, Indolizinyl, Purinyl, Isochinolinyl, Chinolinyl, Chinolizinyl, Chinazolinyl, Chinoxalinyl, Cinnolinyl, Phthalazinyl, 1,7- or 1,8-Naphthyridinyl, Pteridinyl. Der C₅-C₆-gliedrige Heteroarylrest kann gegebenenfalls einfach durch Fluor, Chlor, Hydroxy, C₁-C₃-Alkyl beziehungsweise eine Trifluormethylgruppe substituiert sein.

Ist eine basische Funktion enthalten, sind die physiologisch verträglichen Salze organischer und anorganischer Säuren geeignet, wie Salzsäure, Schwefelsäure, Phosphorsäure, Zitronensäure, Weinsäure u.a.

Bevorzugt sind Verbindungen der Formel I, wobei
- A: für Cyano, C₁-C₃-Alkyl, 5-gliedriges Heterocyclyl, 5-gliedriges Heteroaryl, R¹¹O-C(=O)-(CH₂)ₚ-, R⁵R^{5'}N-C(=O)-(CH₂)ₚ- oder R⁵R^{5'}N-S(=O)₂-(CH₂)ₚ-steht,
wobei Heteroaryl bevorzugt aus der Gruppe bestehend aus Triazolyl, Tetrazolyl und Oxadiazolyl ausgewählt wird,
   und
wobei Alkylreste gegebenenfalls einfach oder mehrfach, gleich oder verschieden substituiert sein können mit Hydroxy,
   und
wobei Heterarylreste gegebenenfalls einfach oder mehrfach, gleich oder verschieden substituiert sein können mit C₁-C₃-Alkyl, Trifluormethyl oder Hydroxy,
- B: für eine Gruppe 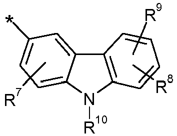 steht,
worin * die Verknüpfungsstelle im Molekül bedeutet,
- R^{1a}: für Wasserstoff oder C₁-C₅-Alkyl steht,
- R^{1b}: für Wasserstoff, C₁-C₅-Alkyl, C₂-C₅-Alkenyl, (C₃-C₆-Cycloalkyl)-(CH₂)ₘ-, (4-6-gliedriges Heterocyclyl)-(CH₂)ₙ-, (C₁-C₅-Alkoxy)-(C₁-C₃-Alkyl)- oder (C₁-C₅-Alkyl)₂N-(C₁-C₃-alkyl)- steht,
wobei Heterocyclyl bevorzugt aus der Gruppe bestehend aus Oxetanyl, Tetrahydrofuranyl, 1,4-Dioxanyl, Morpholinyl und Pyrrolidinyl ausgewählt wird,
   und
wobei Alkylreste und Cycloalkylreste gegebenenfalls einfach oder mehrfach, gleich oder verschieden substituiert sein können mit C₁-C₅-Alkyl, Hydroxy oder (C₁-C₅-Alkyl)-S(O)₂-,
- R⁴: für Wasserstoff, Fluor, Chlor, C₁-C₂-Alkyl oder C₁-C₂-Alkoxy steht,
- R⁵, R^{5'}: unabhängig voneinander für Wasserstoff, C₁-C₇-Alkyl, (4-6-gliedriges Heterocyclyl)-(CH₂)ᵣ-, (C₁-C₇-Alkyl)-S(O)₂-, (C₃-C₇-Cycloalkyl)-S(O)₂-, Phenyl-(CH₂)r-S(O)₂- oder Pyridyl-(CH₂)ᵣ-S(O)₂- stehen,
wobei Heterocyclyl bevorzugt aus der Gruppe bestehend aus Morpholinyl und Pyrrolidinyl ausgewählt wird,
   und
wobei R⁵ und R^{5'} unabhängig voneinander gegebenenfalls einfach oder mehrfach, gleich oder verschieden substituiert sein können mit Halogen, C₁-C₂-Alkyl, Trifluoromethyl, (C₁-C₅-Alkyl)₂N-, C₁-C₂-Alkoxy oder Trifluormethoxy,
oder
R⁵, R^{5'} zusammen mit dem Stickstoffatom, an das sie gebunden sind, einen 4-6-gliedrigen heterocyclischen Ring bilden, der optional einfach mit Oxo oder Hydroxy substituiert sein kann,
wobei ein 6-gliedriger heterocyclischer Ring optional als weiteres Ringatom ein Sauerstoffatom enthalten kann,

R⁶ für Wasserstoff, Fluor, Methyl oder Methoxy steht,
R⁷ für Wasserstoff, Fluor, Chlor, C₁-C₃-Alkyl oder C₁-C₂-Alkoxy steht,
R⁸ für Fluor, Chlor, Brom, C₁-C₃-Alkyl oder C₁-C₂-Alkoxy steht,
R⁹ für Fluor, Chlor, Brom, C₁-C₃-Alkyl oder C₁-C₂-Alkoxy steht,
oder
R⁹ für Brom steht und gleichzeitig R⁸ für Wasserstoff steht,
R¹⁰ für C₁-C₅-Alkyl, C₃-C₅-Alkenyl, C₃-C₅-Alkinyl, (C₃-C₆-Cycloalkyl)-(CH₂)ₙ-oder (C₁-C₇-Alkoxy)-(C₂-C₅-Alkyl)- steht,
R¹¹ für Wasserstoff oder C₁-C₇-Alkyl steht,
m 0 oder 1 ist,
n 0 oder 1 ist,
p 0 ist, und
r 0, 1 oder 2 ist,
sowie deren Diastereomere, Enantiomere, Solvate und Salze oder Cyclodextrin-Clathrate.

Bevorzugt sind Verbindungen der Formel I, wobei
- A: für 5-gliedriges Heteroaryl, R¹¹O-C(=O)-(CH₂)ₚ-, R⁵R^{5'}N-C(=O)-(CH₂)ₚ-oder R⁵R^{5'}N-S(=O)₂-(CH₂)ₚ- steht,
wobei Heteroaryl bevorzugt aus der Gruppe bestehend aus Triazolyl, Tetrazolyl und Oxadiazolyl ausgewählt wird,
   und
wobei Heterarylreste gegebenenfalls einfach oder mehrfach, gleich oder verschieden substituiert sein können mit C₁-C₃-Alkyl, Trifluormethyl oder Hydroxy,
- B: für eine Gruppe 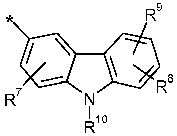 steht,
worin * die Verknüpfungsstelle im Molekül bedeutet,
- R^{1a}: für Wasserstoff oder Methyl steht,
- R^{1b}: für Wasserstoff, C₁-C₂-Alkyl, Vinyl, Cyclopropyl-(CH₂)ₘ-, Methoxy-(C₁-C₂-Alkyl)- oder (N,N-Dimethylamino)methyl steht,
wobei Alkylreste und Cycloalkylreste gegebenenfalls einfach oder mehrfach, gleich oder verschieden substituiert sein können mit Methyl, Hydroxy, oder Methylsulfonyl,
- R⁴: für Wasserstoff, Fluor, Chlor, Methyl oder Methoxy steht,
- R⁵, R^{5'}: unabhängig voneinander für Wasserstoff, C₁-C₂-Alkyl oder (5-6-gliedriges Heterocyclyl)-(CH₂)ᵣ- stehen,
wobei Heterocyclyl bevorzugt aus der Gruppe bestehend aus Morpholinyl und Pyrrolidinyl ausgewählt wird,
   und
wobei R⁵ und R^{5'} unabhängig voneinander gegebenenfalls einfach oder mehrfach, gleich oder verschieden substituiert sein können mit Chlor, Fluor, Methyl, Trifluoromethyl, N,N-Dimethylamino, Methoxy oder Trifluoromethoxy,
oder
- R⁵, R^{5'}: zusammen mit dem Stickstoffatom, an das sie gebunden sind, einen 4-6-gliedrigen heterocyclischen Ring bilden, der optional einfach mit Oxo oder Hydroxy substituiert sein kann,
wobei ein 6-gliedriger heterocyclischer Ring optional als weiteres Ringatom ein Sauerstoffatom enthalten kann,
- R⁶: für Wasserstoff, Fluor, Methyl oder Methoxy steht,
- R⁷: für Wasserstoff, Fluor, Chlor, Methyl oder Methoxy steht,
- R⁸: für Fluor, Chlor, Brom, Methyl oder Methoxy steht,
- R⁹: für Fluor, Chlor, Brom, Methyl oder Methoxy steht,
oder
- R⁹: für Brom steht und gleichzeitig R⁸ für Wasserstoff steht,
- R¹⁰: für C₁-C₃-Alkyl, Allyl, Propargyl, (C₃-C₄-Cycloalkyl)-(CH₂)ₙ- oder Methoxyethyl steht,
- R¹¹: für Wasserstoff oder C₁-C₃-Alkyl steht,
- m: 0 oder 1 ist,
- n: 0 oder 1 ist,
- p: 0 ist, und
- r: 0, 1 oder 2 ist,
sowie deren Diastereomere, Enantiomere, Solvate und Salze oder Cyclodextrin-Clathrate.

Bevorzugt sind Verbindungen der Formel I, wobei
- A: für R¹¹O-C(=O)-(CH₂)ₚ- steht,
- B: für eine Gruppe 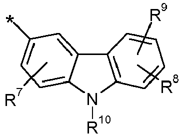 steht,
worin * die Verknüpfungsstelle im Molekül bedeutet,
- R^{1a}: für Wasserstoff steht,
- R^{1b}: für Methoxymethyl steht,
- R⁴: für Wasserstoff, Fluor oder Methyl steht,
- R⁶: für Wasserstoff steht,
- R⁷: für Wasserstoff steht,
- R⁸: für Fluor, Chlor oder Methyl steht,
- R⁹: für Fluor, Chlor, Brom oder Methyl steht,
oder
- R⁹: für Brom steht und gleichzeitig R⁸ für Wasserstoff steht,
- R¹⁰: für Ethyl steht,
- R¹¹: für Wasserstoff, Methyl oder Ethyl steht, und
- p: 0 ist,
sowie deren Diastereomere, Enantiomere, Solvate und Salze oder Cyclodextrin-Clathrate.

Bevorzugt sind Verbindungen der Formel I, in denen
- A: für Wasserstoff, Brom, Cyano, Formyl, C₁-C₃-Alkyl, 4-6-gliedriges Heterocyclyl, 5-6-gliedriges Heteroaryl, R¹¹O-C(=O)-(CH₂)ₚ-, R⁵R^{5'}N-C(=O)-(CH₂)ₚ-, R¹¹O-S(=O)₂-(CH₂)ₚ-, R⁵R^{5'}N-S(=O)₂-(CH₂)ₚ-, (C₁-C₆-Alkyl)-S(=O)₂-, (C₁-C₆-Alkyl)-S(=O)(=NH)- oder (C₃-C₆-Cycloalkyl)-S(=O)(=NH)- steht,
wobei Heteroaryl bevorzugt aus der Gruppe bestehend aus Pyrazolyl, Imidazolyl, Triazolyl, Tetrazolyl, Oxazolyl, Isoxazolyl und Oxadiazolyl ausgewählt wird,
   und
wobei Alkylreste gegebenenfalls einfach oder mehrfach, gleich oder verschieden substituiert sein können mit Halogen oder Hydroxy,
   und
wobei Heterocyclylreste gegebenenfalls einfach oder mehrfach, gleich oder verschieden substituiert sein können mit C₁-C₃-Alkyl, Trifluormethyl oder Hydroxy,
   und
wobei Heterarylreste gegebenenfalls einfach oder mehrfach, gleich oder verschieden substituiert sein können mit C₁-C₃-Alkyl, Trifluormethyl oder Hydroxy.

Bevorzugt sind Verbindungen der Formel I, in denen
- A: für Cyano, C₁-C₃-Alkyl, 5-gliedriges Heterocyclyl, 5-gliedriges Heteroaryl, R¹¹O-C(=O)-(CH₂)ₚ-, R⁵R^{5'}N-C(=O)-(CH₂)ₚ- oder R⁵R^{5'}N-S(=O)₂-(CH₂)ₚ-steht,
wobei Heteroaryl bevorzugt aus der Gruppe bestehend aus Triazolyl, Tetrazolyl und Oxadiazolyl ausgewählt wird,
   und
wobei Alkylreste gegebenenfalls einfach oder mehrfach, gleich oder verschieden substituiert sein können mit Hydroxy,
   und
wobei Heterarylreste gegebenenfalls einfach oder mehrfach, gleich oder verschieden substituiert sein können mit C₁-C₃-Alkyl, Trifluormethyl oder Hydroxy.

Bevorzugt sind Verbindungen der Formel I, in denen
- A: für 5-gliedriges Heteroaryl, R¹¹O-C(=O)-(CH₂)ₚ-, R⁵R^{5'}N-C(=O)-(CH₂)ₚ-oder R⁵R^{5'}N-S(=O)₂-(CH₂)ₚ- steht,
wobei Heteroaryl bevorzugt aus der Gruppe bestehend aus Triazolyl, Tetrazolyl und Oxadiazolyl ausgewählt wird,
   und
wobei Heterarylreste gegebenenfalls einfach oder mehrfach, gleich oder verschieden substituiert sein können mit C₁-C₃-Alkyl, Trifluormethyl oder Hydroxy.

Bevorzugt sind Verbindungen der Formel I, in denen
- A: für R¹¹O-C(=O)-(CH₂)ₚ- steht.

Bevorzugt sind Verbindungen der Formel I, in denen
- B: aus den folgenden Strukturen ausgewählt wird, 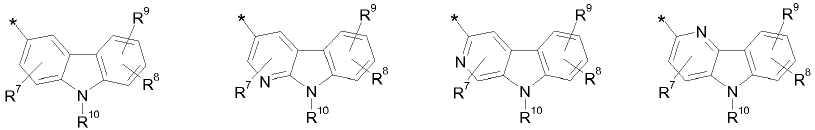 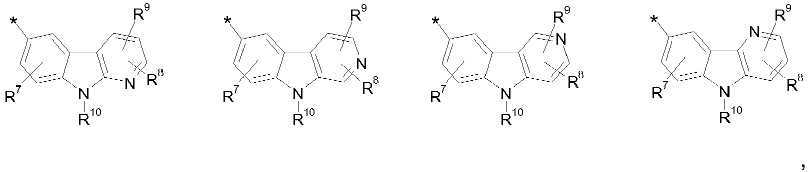 worin * die Verknüpfungsstelle im Molekül bedeutet.

Bevorzugt sind Verbindungen der Formel I, in denen
- B: für eine Gruppe 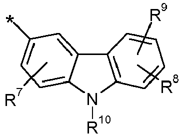
steht,
worin * die Verknüpfungsstelle im Molekül bedeutet.

Bevorzugt sind Verbindungen der Formel I, in denen
- R^{1a}, R^{1b}: unabhängig voneinander für Wasserstoff, Cyano, C₁-C₅-Alkyl, C₂-C₅-Alkenyl, C₂-C₅-Alkinyl, (C₃-C₆-Cycloalkyl)-(CH₂)ₘ-, (4-6-gliedriges Heterocyclyl)-(CH₂)ₙ-, (C₁-C₅-Alkoxy)-(C₁-C₃-alkyl)-, (C₃-C₆-Cycloalkoxy)-(C₁-C₃-alkyl)-, H₂N-(C₁-C₃-Alkyl)-, (C₁-C₅-Alkyl)NH-(C₁-C₃-alkyl)- oder (C₁-C₅-Alkyl)₂N-(C₁-C₃-alkyl)- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
   und
wobei Alkylreste, Cycloalkylreste und Heterocyclylreste gegebenenfalls einfach oder mehrfach, gleich oder verschieden substituiert sein können mit Halogen, C₁-C₅-Alkyl, Hydroxy, C(=O)OH,
   HO-C(=O)-(C₁-C₅-Alkyl)-, (C₁-C₅-Alkyl)O-C(=O)-(C₁-C₅-alkyl)-
   oder
(C₁-C₅-Alkyl)-S(=O)₂-.

Bevorzugt sind Verbindungen der Formel I, in denen
- R^{1a}: für Wasserstoff oder C₁-C₅-Alkyl steht.

Bevorzugt sind Verbindungen der Formel I, in denen
- R^{1a}: für Wasserstoff oder Methyl steht.

Bevorzugt sind Verbindungen der Formel I, in denen
- R^{1a}: für Methyl steht.

Bevorzugt sind Verbindungen der Formel I, in denen
- R^{1a}: Wasserstoff steht.

Bevorzugt sind Verbindungen der Formel I, in denen
- R^{1b}: für Wasserstoff, C₁-C₅-Alkyl, C₂-C₅-Alkenyl, (C₃-C₆-Cycloalkyl)-(CH₂)ₘ-, (4-6-gliedriges Heterocyclyl)-(CH₂)ₙ-, (C₁-C₅-Alkoxy)-(C₁-C₃-Alkyl)- oder (C₁-C₅-Alkyl)₂N-(C₁-C₃-alkyl)- steht,
wobei Heterocyclyl bevorzugt aus der Gruppe bestehend aus Oxetanyl, Tetrahydrofuranyl, 1,4-Dioxanyl, Morpholinyl und Pyrrolidinyl ausgewählt wird,
   und
wobei Alkylreste und Cycloalkylreste gegebenenfalls einfach oder mehrfach, gleich oder verschieden substituiert sein können mit C₁-C₅-Alkyl, Hydroxy oder (C₁-C₅-Alkyl)-S(O)₂-.

Bevorzugt sind Verbindungen der Formel I, in denen
- R^{1b}: für Wasserstoff, C₁-C₂-Alkyl, Vinyl, Cyclopropyl-(CH₂)ₘ-, Methoxy-(C₁-C₂-Alkyl)- oder (N,N-Dimethylamino)methyl steht,
wobei Alkyl-, Alkylen- und Cycloalkylreste gegebenenfalls einfach oder mehrfach, gleich oder verschieden substituiert sein können mit Methyl, Hydroxy, oder Methylsulfonyl.

Bevorzugt sind Verbindungen der Formel I, in denen
- R^{1b}: für Methoxymethyl steht.

Bevorzugt sind Verbindungen der Formel I, in denen
- R⁴: für Wasserstoff, Fluor, Chlor, C₁-C₂-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen oder Hydroxy.

Bevorzugt sind Verbindungen der Formel I, in denen
- R⁴: für Wasserstoff, Fluor, Chlor, C₁-C₂-Alkyl oder C₁-C₂-Alkoxy steht.

Bevorzugt sind Verbindungen der Formel I, in denen
- R⁴: für Wasserstoff, Fluor, Chlor, Methyl oder Methoxy steht.

Bevorzugt sind Verbindungen der Formel I, in denen
- R⁴: für Wasserstoff, Fluor oder Methyl steht.

Bevorzugt sind Verbindungen der Formel I, in denen
- R⁴: für Wasserstoff steht.

Bevorzugt sind Verbindungen der Formel I, in denen
- R⁴: für Fluor steht.

Bevorzugt sind Verbindungen der Formel I, in denen
- R⁴: für Methyl steht.

Bevorzugt sind Verbindungen der Formel I, in denen
- R⁵, R^{5'}: unabhängig voneinander für Wasserstoff, C₁-C₇-Alkyl, (C₁-C₇-Alkoxy)-(C₂-C₅-alkyl)-, (4-6-gliedriges Heterocyclyl)-(CH₂)ᵣ-, (C₁-C₇-Alkyl)-C(=O)-, (C₃-C₇-Cycloalkyl)-C(=O)-, Phenyl-(CH₂)ᵣ-C(=O)-, Pyridyl-(CH₂)ᵣ-C(=O)-, (C₁-C₇-Alkyl)-S(=O)₂-, (C₃-C₇-Cycloalkyl)-S(=O)₂-, Phenyl-(CH₂)ᵣ-S(=O)₂- oder Pyridyl-(CH₂)ᵣ-S(=O)₂- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
   und
wobei R⁵ und R^{5'} unabhängig voneinander gegebenenfalls einfach oder mehrfach, gleich oder verschieden substituiert sein können mit Halogen, Hydroxy, C₁-C₂-Alkyl, Trifluoromethyl, (C₁-C₅-Alkyl)NH-,
   (C₁-C₅-Alkyl)₂N-, C₁-C₂-Alkoxy oder Trifluormethoxy,
oder
- R⁵, R^{5'}: zusammen mit dem Stickstoffatom, an das sie gebunden sind, einen 4-6-gliedrigen heterocyclischen Ring bilden, der optional, einfach oder mehrfach, gleich oder verschieden mit Oxo, Hydroxy, Carboxy, C₁-C₂-Alkyl oder
C₁-C₂-Alkoxy substituiert sein kann,
wobei ein 6-gliedriger heterocyclischer Ring optional als weiteres Ringatom ein Heteroatom, das aus der Gruppe bestehend aus O und N ausgewählt wird, enthalten kann.

Bevorzugt sind Verbindungen der Formel I, in denen
- R⁵, R^{5'}: unabhängig voneinander für Wasserstoff, C₁-C₇-Alkyl, (C₁-C₇-Alkoxy)-(C₂-C₅-alkyl)-, (4-6-gliedriges Heterocyclyl)-(CH₂)ᵣ-, (C₁-C₇-Alkyl)-C(=O)-, (C₃-C₇-Cycloalkyl)-C(=O)-, Phenyl-(CH₂)ᵣ-C(=O)-, Pyridyl-(CH₂)ᵣ-C(=O)-, (C₁-C₇-Alkyl)-S(=O)₂-, (C₃-C₇-Cycloalkyl)-S(=O)₂-, Phenyl-(CH₂)ᵣ-S(=O)₂- oder Pyridyl-(CH₂)ᵣ-S(=O)₂- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
   und
wobei R⁵ und R^{5'} unabhängig voneinander gegebenenfalls einfach oder mehrfach, gleich oder verschieden substituiert sein können mit Halogen, Hydroxy, C₁-C₂-Alkyl, Trifluoromethyl, (C₁-C₅-Alkyl)NH-,
   (C₁-C₅-Alkyl)₂N-, C₁-C₂-Alkoxy oder Trifluormethoxy.

Bevorzugt sind Verbindungen der Formel I, in denen
- R⁵, R^{5'}: zusammen mit dem Stickstoffatom, an das sie gebunden sind, einen 4-6-gliedrigen heterocyclischen Ring bilden, der optional, einfach oder mehrfach, gleich oder verschieden mit Oxo, Hydroxy, Carboxy, C₁-C₂-Alkyl oder
C₁-C₂-Alkoxy substituiert sein kann,
wobei ein 6-gliedriger heterocyclischer Ring optional als weiteres Ringatom ein Heteroatom, das aus der Gruppe bestehend aus O und N ausgewählt wird, enthalten kann.

Bevorzugt sind Verbindungen der Formel I, in denen
- R⁵, R^{5'}: unabhängig voneinander für Wasserstoff, C₁-C₇-Alkyl, (4-6-gliedriges Heterocyclyl)-(CH₂)ᵣ-, (C₁-C₇-Alkyl)-S(O)₂-, (C₃-C₇-Cycloalkyl)-S(O)₂-, Phenyl-(CH₂)ᵣ-S(O)₂- oder Pyridyl-(CH₂)ᵣ-S(O)₂- stehen,
wobei Heterocyclyl bevorzugt aus der Gruppe bestehend aus Morpholinyl und Pyrrolidinyl ausgewählt wird,
   und
wobei R⁵ und R^{5'} unabhängig voneinander gegebenenfalls einfach oder mehrfach, gleich oder verschieden substituiert sein können mit Halogen, C₁-C₂-Alkyl, Trifluoromethyl, (C₁-C₅-Alkyl)₂N-, C₁-C₂-Alkoxy oder Trifluormethoxy,
oder
- R⁵, R^{5'}: zusammen mit dem Stickstoffatom, an das sie gebunden sind, einen 4-6-gliedrigen heterocyclischen Ring bilden, der optional einfach mit Oxo oder Hydroxy substituiert sein kann,
wobei ein 6-gliedriger heterocyclischer Ring optional als weiteres Ringatom ein Sauerstoffatom enthalten kann.

Bevorzugt sind Verbindungen der Formel I, in denen
- R⁵, R^{5'}: unabhängig voneinander für Wasserstoff, C₁-C₇-Alkyl, (4-6-gliedriges Heterocyclyl)-(CH₂)ᵣ-, (C₁-C₇-Alkyl)-S(O)₂-, (C₃-C₇-Cycloalkyl)-S(O)₂-, Phenyl-(CH₂)ᵣ-S(O)₂- oder Pyridyl-(CH₂)ᵣ-S(O)₂- stehen,
wobei Heterocyclyl bevorzugt aus der Gruppe bestehend aus Morpholinyl und Pyrrolidinyl ausgewählt wird,
   und
wobei R⁵ und R^{5'} unabhängig voneinander gegebenenfalls einfach oder mehrfach, gleich oder verschieden substituiert sein können mit Halogen, C₁-C₂-Alkyl, Trifluoromethyl, (C₁-C₅-Alkyl)₂N-, C₁-C₂-Alkoxy oder Trifluormethoxy.

Bevorzugt sind Verbindungen der Formel I, in denen
- R⁵, R^{5'}: zusammen mit dem Stickstoffatom, an das sie gebunden sind, einen 4-6-gliedrigen heterocyclischen Ring bilden, der optional einfach mit Oxo oder Hydroxy substituiert sein kann,
wobei ein 6-gliedriger heterocyclischer Ring optional als weiteres Ringatom ein Sauerstoffatom enthalten kann.

Bevorzugt sind Verbindungen der Formel I, in denen
- R⁵, R^{5'}: unabhängig voneinander für Wasserstoff, C₁-C₂-Alkyl oder (5-6-gliedriges Heterocyclyl)-(CH₂)ᵣ- stehen,
wobei Heterocyclyl bevorzugt aus der Gruppe bestehend aus Morpholinyl und Pyrrolidinyl ausgewählt wird,
   und
wobei R⁵ und R^{5'} unabhängig voneinander gegebenenfalls einfach oder mehrfach, gleich oder verschieden substituiert sein können mit Chlor, Fluor, Methyl, Trifluoromethyl, N,N-Dimethylamino, Methoxy oder Trifluoromethoxy.

Bevorzugt sind Verbindungen der Formel I, in denen
- R⁶: für Wasserstoff, Fluor, Chlor, Methyl, Trifluormethyl, Methoxy oder Trifluormethoxy steht.

Bevorzugt sind Verbindungen der Formel I, in denen
- R⁶: für Wasserstoff, Fluor, Methyl oder Methoxy steht.

Bevorzugt sind Verbindungen der Formel I, in denen
- R⁶: für Wasserstoff steht.

Bevorzugt sind Verbindungen der Formel I, in denen
- R⁷: für Wasserstoff, Fluor, Chlor, Cyano, SF₅, C₁-C₃-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen.

Bevorzugt sind Verbindungen der Formel I, in denen
- R⁷: für Wasserstoff, Fluor, Chlor, C₁-C₃-Alkyl oder C₁-C₂-Alkoxy steht.

Bevorzugt sind Verbindungen der Formel I, in denen
- R⁷: für Wasserstoff, Fluor, Chlor, Methyl oder Methoxy steht.

Bevorzugt sind Verbindungen der Formel I, in denen
- R⁷: für Wasserstoff steht.

Bevorzugt sind Verbindungen der Formel I, in denen
- R⁸: für Fluor, Chlor, Brom, Cyano, SF₅, C₁-C₃-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen.

Bevorzugt sind Verbindungen der Formel I, in denen
- R⁸: für Fluor, Chlor, Brom, C₁-C₃-Alkyl oder C₁-C₂-Alkoxy steht.

Bevorzugt sind Verbindungen der Formel I, in denen
- R⁸: für Fluor, Chlor, Brom, Methyl oder Methoxy steht.

Bevorzugt sind Verbindungen der Formel I, in denen
- R⁸: für Fluor, Chlor oder Methyl steht.

Bevorzugt sind Verbindungen der Formel I, in denen
- R⁸: für Fluor steht.

Bevorzugt sind Verbindungen der Formel I, in denen
- R⁸: für Chlor steht.

Bevorzugt sind Verbindungen der Formel I, in denen
- R⁸: für Methyl steht.

Bevorzugt sind Verbindungen der Formel I, in denen
- R⁹: für Fluor, Chlor, Brom, Cyano, C₁-C₃-Alkyl oder C₁-C₂-Alkoxy steht.

Bevorzugt sind Verbindungen der Formel I, in denen
- R⁹: für Fluor, Chlor, Brom, C₁-C₃-Alkyl oder C₁-C₂-Alkoxy steht.

Bevorzugt sind Verbindungen der Formel I, in denen
- R⁹: für Fluor, Chlor, Brom, Methyl oder Methoxy steht.

Bevorzugt sind Verbindungen der Formel I, in denen
- R⁹: für Fluor, Chlor, Brom oder Methyl steht.

Bevorzugt sind Verbindungen der Formel I, in denen
- R⁹: für Fluor steht.

Bevorzugt sind Verbindungen der Formel I, in denen
- R⁹: für Chlor steht,

Bevorzugt sind Verbindungen der Formel I, in denen
- R⁹: für Brom steht.

Bevorzugt sind Verbindungen der Formel I, in denen
- R⁹: für Methyl steht.

Bevorzugt sind Verbindungen der Formel I, in denen
- R⁹: für Brom steht und gleichzeitig R⁸ für Wasserstoff steht.

Bevorzugt sind Verbindungen der Formel I, in denen
- R¹⁰: für C₁-C₅-Alkyl, C₃-C₅-Alkenyl, C₃-C₅-Alkinyl, (C₃-C₆-Cycloalkyl)-(CH₂)ₙ-, (4-6-gliedriges Heterocyclyl)-(CH₂)ₙ-, oder (C₁-C₇-Alkoxy)-(C₂-C₅-alkyl)-steht,
wobei Heterocyclyl bevorzugt aus der Gruppe bestehend aus Oxetanyl, Tetrahydrofuranyl, Tetrahydropyranyl, Morpholinyl, Pyrrolidinyl und Piperidinyl ausgewählt wird,
   und
wobei Alkylreste, Cycloalkylreste und Heterocyclylreste gegebenenfalls einfach oder mehrfach, gleich oder verschieden substituiert sein können mit Halogen, C₁-C₂-Alkyl, C₁-C₂-Alkoxy oder C(=O)OH.

Bevorzugt sind Verbindungen der Formel I, in denen
- R¹⁰: für C₁-C₅-Alkyl, C₃-C₅-Alkenyl, C₃-C₅-Alkinyl, (C₃-C₆-Cycloalkyl)-(CH₂)ₙ-oder (C₁-C₇-Alkoxy)-(C₂-C₅-Alkyl)- steht.

Bevorzugt sind Verbindungen der Formel I, in denen
- R¹⁰: für C₁-C₃-Alkyl, Allyl, Propargyl, (C₃-C₄-Cycloalkyl)-(CH₂)ₙ- oder Methoxyethyl steht.

Bevorzugt sind Verbindungen der Formel I, in denen
- R¹⁰: für Ethyl steht.

Bevorzugt sind Verbindungen der Formel I, in denen
- R¹¹: für Wasserstoff, C₁-C₇-Alkyl, C₃-C₇-Cycloalkyl, Phenyl-(CH₂)_{q}- oder (C₁-C₇-Alkoxy)-(C₂-C₅-alkyl)- steht,
wobei Phenyl gegebenenfalls einfach oder mehrfach, gleich oder verschieden substituiert sein kann mit C₁-C₃-Alkyl, Trifluormethyl oder Hydroxy.

Bevorzugt sind Verbindungen der Formel I, in denen
- R¹¹: für Wasserstoff oder C₁-C₇-Alkyl steht.

Bevorzugt sind Verbindungen der Formel I, in denen
- R¹¹: für Wasserstoff oder C₁-C₃-Alkyl steht.

Bevorzugt sind Verbindungen der Formel I, in denen
- R¹¹: für Wasserstoff, Methyl oder Ethyl steht.

Bevorzugt sind Verbindungen der Formel I, in denen
- R¹¹: für Methyl oder Ethyl steht.

Bevorzugt sind Verbindungen der Formel I, in denen
- R¹¹: für Wasserstoff steht.

Bevorzugt sind Verbindungen der Formel I, in denen
- m: 0, 1, 2 oder 3 ist.

Bevorzugt sind Verbindungen der Formel I, in denen
- m: 0 oder 1 ist.

Bevorzugt sind Verbindungen der Formel I, in denen
- m: 1 ist.

Bevorzugt sind Verbindungen der Formel I, in denen
- m: 0 ist.

Bevorzugt sind Verbindungen der Formel I, in denen
- n: 0, 1, 2 oder 3 ist.

Bevorzugt sind Verbindungen der Formel I, in denen
- n: 0 oder 1 ist.

Bevorzugt sind Verbindungen der Formel I, in denen
- n: 1 ist.

Bevorzugt sind Verbindungen der Formel I, in denen
- n: 0 ist.

Bevorzugt sind Verbindungen der Formel I, in denen
- p: 0, 1 oder 2 ist.

Bevorzugt sind Verbindungen der Formel I, in denen
- p: 0 ist.

Bevorzugt sind Verbindungen der Formel I, in denen
- q: 1, 2 oder 3 ist.

Bevorzugt sind Verbindungen der Formel I, in denen
- r: 0, 1, 2 oder 3 ist.

Bevorzugt sind Verbindungen der Formel I, in denen
- r: 0, 1 oder 2 ist.

Die folgenden Verbindungen entsprechend der vorliegenden Erfindung sind ganz besonders bevorzugt:
1. Methyl-2-(6-brom-9-ethyl-9H-carbazol-3-yl)-1-(2-methoxyethyl)-4-methyl-1H-benzimidazol-5-carboxylat
2. 2-(6-Brom-9-ethyl-9H-carbazol-3-yl)-1-(2-methoxyethyl)-4-methyl-1H-benzimidazol-5-carbonsäure
3. Methyl-2-(9-ethyl-8-fluor-6-methyl-9H-carbazol-3-yl)-1-(2-methoxyethyl)-4-methyl-1H-benzimidazol-5-carboxylat
4. 2-(9-Ethyl-8-fluor-6-methyl-9H-carbazol-3-yl)-1-(2-methoxyethyl)-4-methyl-1H-benzimidazol-5-carbonsäure
5. Methyl-2-(9-ethyl-6-fluor-8-methyl-9H-carbazol-3-yl)-1-(2-methoxyethyl)-4-methyl-1H-benzimidazol-5-carboxylat
6. 2-(9-Ethyl-6-fluor-8-methyl-9H-carbazol-3-yl)-1-(2-methoxyethyl)-4-methyl-1H-benzimidazol-5-carbonsäure
7. 2-(9-Ethyl-6-fluor-8-methyl-9H-carbazol-3-yl)-4-fluor-1-(2-methoxyethyl)-1H-benzimidazol-5-carbonsäure
8. 2-(9-Ethyl-8-fluor-6-methyl-9H-carbazol-3-yl)-4-fluor-1-(2-methoxyethyl)-1H-benzimidazol-5-carbonsäure
9. Ethyl-2-(9-ethyl-8-fluor-6-methyl-9H-carbazol-3-yl)-1-(2-methoxyethyl)-1H-benzimidazol-5-carboxylat
10. 2-(9-Ethyl-8-fluor-6-methyl-9H-carbazol-3-yl)-1-(2-methoxyethyl)-1H-benzimidazol-5-carbonsäure
11. Methyl-2-(8-chlor-9-ethyl-6-methyl-9H-carbazol-3-yl)-1-(2-methoxyethyl)-4-methyl-1H-benzimidazol-5-carboxylat
12. 2-(8-Chlor-9-ethyl-6-methyl-9H-carbazol-3-yl)-1-(2-methoxyethyl)-4-methyl-1H-benzimidazol-5-carbonsäure
13. 2-(5-Chlor-9-ethyl-6-methyl-9H-carbazol-3-yl)-1-(2-methoxyethyl)-4-methyl-1H-benzimidazol-5-carbonsäure

Gegenstand der vorliegenden Erfindung sind die Verbindungen der Formel (I), zur Behandlung und/oder Prophylaxe von Krankheiten.

Die erfindungsgemäßen Verbindungen der allgemeinen Formel I sind Liganden des EP4-Rezeptors und haben antagonistische Wirkung.
Gegenstand der vorliegenden Erfindung sind daher Verbindungen der Formel (I), die antagonistisch am EP4-Rezeptor wirken, zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

Ebenfalls Gegenstand der vorliegenden Erfindung ist die Verwendung einer Verbindung gemäß Formel (I) zur Herstellung eines Arzneimittels zur Behandlung und/oder Prophylaxe von Krankheiten. Dabei kann es sich um die Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen handeln.

Die antagonistische Wirkung kann durch einen Antagonismustest (siehe Beispiel 3.2.1 der biologischen Beispiele) bestimmt werden. So inhibiert beispielsweise die erfindungsgemäße Verbindung 10 die über die Gabe von PGE2 zu EP4-Rezeptor exprimierenden Zellen induzierte Stimulation der cAMP Produktion mit einem IC₅₀-Wert von ca 1.5 nM.

Unter Antagonisten sind solche Moleküle zu verstehen, die an ihre entsprechenden Rezeptoren binden und welche die Initiierung des/der mit dem Rezeptor gekoppelten Signaltransduktionswege/s durch den oder die natürlich vorkommenden Liganden inhibieren. Üblicherweise kompetitieren die Antagonisten mit dem natürlich vorkommenden Liganden des Rezeptors um die Bindung an den Rezeptor. Aber auch andere Modifikationen des Rezeptors durch Moleküle, die verhindern, dass die mit dem Rezeptor gekoppelten Signaltransduktionswege durch den oder die natürlich vorkommenden Liganden aktiviert werden, sind möglich (z.B. nicht-kompetitive, allosterische Modifikationen des Rezeptors).

Bevorzugterweise binden die Antagonisten reversibel an ihre korrespondierenden Rezeptoren.

Die EP4-Rezeptor Liganden mit antagonistischer Wirkung haben eine bevorzugte Affinität für den Rezeptor EP4 gegenüber jedem anderen EP-Subtyp. Der Antagonismus wird in Gegenwart des natürlichen Agonisten gemessen (PGE2).

Ebenfalls Gegenstand der vorliegenden Erfindung auf Grund der antagonistischen Wirkung am Rezeptor EP4 sind Arzneimittel zur Behandlung und/oder Prophylaxe von Erkrankungen, zu denen infektiöse Erkrankungen, Krebs, Herz-/Kreislauf-Erkrankungen, angiogenetische Erkrankungen, Störungen der Uteruskontraktion, akuter und chronischer Schmerz, inflammatorische Erkrankungen, neuroinflammatorische Erkrankungen, neurodegenerative Erkrankungen, Autoimmunerkrankungen, immunabhängige Erkrankungen/Therapien, nephrologische Erkrankungen, ophthamologische Erkrankungen zählen.

Unter infektiösen Erkrankungen sind durch unizelluläre Parasiten hervorgerufene Erkrankungen (z.B. Klebsiella, Streptococcus) zu verstehen. Bei den infektiösen Erkrankungen können die Arzneimittel immunmodulatorisch so wirken, dass die Erkrankungen prophylaktisch (Verringerung der Infektionsgefahr, wie beispielsweise bei Knochenmarkstransplantationen) oder therapeutisch behandelt werden können. Unter Krebs sind solide Tumoren und Leukämien; unter viralen Infektionen z. B. Cytomegalus-Infektionen, Hepatitis, Hepatitis B und C und HIV- Erkrankungen; unter Herz-Kreislauf-Erkrankungen ischämische Reperfusionserkrankung, Stenosen, Arteriosklerosen, Restenosen, Arthritis, Kawasaki-Syndrom und Aneurysmen; unter angiogenetischen Erkrankungen sind neben Endometriose die Fibrose und Fibroide im Uterus; unter Störungen der Uteruskontraktion z.B. Menstruationsbeschwerden; unter Schmerz beispielsweise inflammatorische Hyperalgesie, Arthritis, Arthrose, neuropathischer Schmerz, Gicht, Eingeweideschmerz, Rückenschmerzen, Kopfschmerzen, Migräne, Zahnschmerzen, Schmerzen durch Sonnenbrand und Schmerzen durch Brandverletzungen, unter inflammatorischen Erkrankungen beispielsweise entzündliche Darmerkrankungen; unter neuroinflammatorischen und neurodegenerativen Erkrankungen z.B. Multiple Sklerose, Alzheimer, Parkinson, ALS, Schlaganfall; unter immunabhängigen Erkrankungen/Therapien z.B. Transplantationen, bei denen eine Immunmodulation den Therapieerfolg erhöht; unter Autoimmunerkrankungen beispielsweise die ophthamologische Erkrankung Morbus Basedow, und unter nephrologischen Erkrankungen polyzystische Nierenerkrankungen, Glomerulonephritis, zu verstehen.

Die erfindungsgemäßen Verbindungen können dabei mit den üblichen pharmazeutischen Hilfsstoffen vermengt werden. Die EP4-Rezeptor Liganden mit antagonistischer Wirkung werden in einer dem Fachmann an sich bekannten Weise formuliert.

Ebenfalls Gegenstand der vorliegenden Erfindung ist die Verwendung einer Verbindung gemäß Formel (I) zur Herstellung eines Arzneimittels.

Ebenfalls Gegenstand der vorliegenden Erfindung sind Arzneimittel, die die erfindungsgemäßen Verbindungen enthalten, mit geeigneten Formulierungs- und Trägerstoffen.

Die therapeutisch wirksame Dosis ist abhängig vom Körpergewicht, Applikationsweg, individuellem Verhalten, der Art der Zubereitung und Zeitpunkt bzw. Intervall, zu welchem die Applikation erfolgt. Ein typischer Dosisbereich für eine Frau mit 70 kg Körpergewicht liegt zwischen 1-500 mg/Tag, vorzugsweise zwischen 5 und 20 mg/ Tag. Ein weiterer Gegenstand der vorliegenden Erfindung betrifft Arzneimittel, enthaltend mindestens eine erfindungsgemäße Verbindung und mindestens einen oder mehrere weitere Wirkstoffe, insbesondere zur Behandlung und/oder Prophylaxe der Endometriose. Als geeignete Kombinationswirkstoffe seien beispielhaft und vorzugsweise genannt: selektive Estrogen Rezeptor Modulatoren (SERMs), Estrogenrezeptor (ER) Antagonisten, Aromataseinhibitoren, 17-β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen (Mkk3/6, Mek1/2, Erk1/2), Inhibitoren der Proteinkinase B (PKBa/β/γ; Akt1/2/3), Inhibitoren der Phosphoinositid-3-Kinase (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, Neurokinin1 Rezeptor Anatgonisten, Paracetamol, selektiven COX2-Inhibitoren und/oder nicht-selektiven COX1/COX2 Inhibitoren.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die zuvor genannten Indikationen.

Die Verbindungen können, sowohl nach oraler als auch parenteraler Gabe, für die zuvor genannten Indikationen eingesetzt werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Die Dosierung der Verbindungen der allgemeinen Formel I sollte in diesen Zubereitungen 0.01 % - 20 % betragen, um eine ausreichende pharmakologische Wirkung zu erzielen.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Die oben beschrieben Formulierungen und Darreichungsformen sind ebenfalls Gegenstand der vorliegenden Erfindung.

Sofern neben der erfindungsgemäßen Verbindung gemäß Formel I weitere Wirkstoffe enthalten sind, können diese in einer gemeinsamen Applikationsform formuliert sein oder gegebenenfalls auch als Kombinationspräparat verabreicht werden.

Für die orale Applikation eignen sich nach dem Stand der Technik funktionierende, die erfindungsgemäß zu verwendenden Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäß zu verwendenden Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Tinkturen, Vaginalkapseln und -zäpfchen, Tampons, Intrauterinpessare, wässrige Suspensionen (Lotionen, Schüttelmixturen), lipophile Suspensionen, Kristallsuspensionen, wässrige und ölige Injektionslösungen, Depotpräparationen, Salben, Fettsalben, Gele, Cremes, transdermale therapeutische Systeme (z.B. Pflaster), Milch, Pasten, Schäume, Streupuder, Implantate, intrauterine Spiralen, Vaginalringe oder Stents.

Bevorzugt sind die orale oder parenterale Applikation, insbesondere die orale und die intravenöse Applikation.

Die erfindungsgemäß zu verwendenden Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Bei oraler Applikation beträgt die Menge pro Tag etwa 0,01 bis 100 mg/kg Körpergewicht. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg/kg Körpergewicht je Tag betragen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Ebenfalls Gegenstand der vorliegenden Erfindung ist die Verwendung der erfindungsgemäßen Substanzen als EP4-Rezeptor Liganden mit antagonistischer Wirkung zur Herstellung eines Arzneimittels zur Prophylaxe und direkten Behandlung von Erkrankungen, die ursächlich im Zusammenhang mit dem EP4-Rezeptor stehen oder von Erkrankungen, die durch eine Beeinflussung des EP4-Rezeptors therapiert werden können.

Prostaglandine spielen eine wichtige Rolle bei der Angiogenese (Sales, Jabbour, 2003, Reproduction 126, 559 - 567; Kuwano et al., 2004, FASEB J. 18, 300-310; Kamiyama et al., 2006, Oncogene 25, 7019-7028; Chang et al., 2005, Prostaglandins & other Lipid Mediators 76, 48-58).

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567). Prostaglandine und hier speziell der EP4- und der EP2-Rezeptor sind in Zusammenhang mit starken Menstruationsblutungen gebracht worden (Smith et al., 2007 (Human Reproduction, Vol.22, No.5 pp. 1450-1456).

Gegenstand der vorliegenden Erfindung ist die Verwendung der Substanzen der allgemeinen Formel I zur Herstellung eines Arzneimittels für die Prophylaxe und Behandlung von Menstruationsbeschwerden und starken Menstruationsblutungen sowie Schmerzen während der Menstruation. Fibroide (Myome) sind gutartige Tumore im Uterus mit einer hohen Verbreitungsrate. Über die Stimulierung der Aromatase durch einen PGE2/cAMP-vermittelten Signalweg, sowie durch mögliche andere Mechanismen, besteht ein Zusammenhang zum Prostaglandinstoffwechsel (Imir et al., 2007, J Clin Endocrinol Metab 92, 1979-1982).

Gegenstand der vorliegenden Erfindung ist die Verwendung der Substanzen der allgemeinen Formel I zur Herstellung eines Arzneimittels für die Prophylaxe und Behandlung von Fibroiden (Myome).

Zunehmende Forschungsergebnisse belegen auch die Bedeutung der EP-Rezeptoren, bei einer Vielzahl von Krebsarten (z. B. Brustkrebs, Kolonkarzinom, Lungenkrebs, Prostatakrebs, Leukämie, Hautkrebs), was auf zukünftige Möglichkeiten des Einsatzes von Modulatoren (Antagonisten oder Agonisten) des EP4-Rezeptors für die Therapie und Vorbeugung (prophylaktisch und/oder adjuvant) von Krebs schließen lässt (Fulton et al., 2006, Cancer Res; 66(20): 9794-7; Hull et al., 2004, Mol Cancer Ther;3(8):1031-9; Wang et al., 2004, Seminars in Oncology, Vol 31, No 1, Suppl 3: pp 64-73).

Gegenstand der vorliegenden Erfindung ist die Verwendung der Substanzen der allgemeinen Formel I zur Herstellung eines Arzneimittels für die Behandlung und Vorbeugung von Krebserkrankungen.

Die Aktivierung von endothelialen Zellen spielt im pathogenen Prozess der Arteriosklerose eine wichtige Rolle. Neuere Forschungen zeigen eine Beteiligung des EP4-Rezeptors (Minami et al., 2008, J Biol Chem., Apr 11;283(15):9692-703. Epub 2008 Feb 12).

Gegenstand der vorliegenden Erfindung ist die Verwendung der Substanzen der allgemeinen Formel I zur Herstellung eines Arzneimittels für die Behandlung und Vorbeugung von Arteriosklerose.

Neuere wissenschaftliche Publikationen zeigen, dass bei neurodegenerativen, neuroentzündlichen und ischämischen Erkrankungen (Alzheimer, Parkinson, ALS, Schlaganfall) Prostaglandine und der EP4-Rezeptor wichtige Komponenten des Krankheitsgeschehens darstellen (Hoshino et al., 2007, J Biol Chem.; 282(45): 32676-88; Cimino et al., 2008, Current Medicinal Chemistry, 1863-1869).

Multiple Sklerose ist eine chronische Entzündung des Nervensystems. Prostaglandine, speziell PGE2 und über den EP4-Rezeptor vermittelte Effekte werden ursächlich mit den pathologischen Vorgängen bei Multipler Sklerose in Zusammenhang gebracht (Palumbo et al., 2011, Prostaglandins, Leukotrienes and Essential Fatty Acids 85: 29-35; Kihara et al., 2009, Proc Natl Acad Sci U. S. A, 106, Nr. 51: 21807-21812).

Gegenstand der vorliegenden Erfindung ist die Verwendung der Substanzen der allgemeinen Formel I zur Herstellung eines Arzneimittels für die Behandlung und Vorbeugung von neurodegenerativen, neuroentzündlichen und ischämischen Erkrankungen wie beispielsweise Alzheimer, Parkinson, ALS, Schlaganfall sowie für die Behandlung der Multiplen Sklerose.

Polyzystische Nierenerkrankungen stehen ebenfalls im Zusammenhang mit dem EP4-Rezeptor (Liu et al., 2012, Am J Physiol Renal Physiol. 2012 Aug 29. [Epub ahead of print.)

Gegenstand der vorliegenden Erfindung ist die Verwendung der Substanzen der allgemeinen Formel I zur Herstellung eines Arzneimittels für die Behandlung und Vorbeugung von polyzystischen Nierenerkrankungen.

Es gibt Hinweise, dass eine inflammatorische, gesteigerte Schmerzempfindlichkeit behandelt werden kann, indem gezielt EP4-Rezeptoren moduliert werden. Des Weiteren steht der EP4-Rezeptor in Zusammenhang mit weiteren Schmerzarten (Zeilhofer, 2007, Biochemical Pharmacology 73; 165- 174). Murase et al. (Eur J Pharmacol. 2008 Feb 2;580(1-2):116-21) berichten von einem Zusammenhang zwischen EP4-Rezeptor-Blockade und einer symptomatischen Erleichterung der Beschwerden, die bei Osteoarthritis und / oder rheumatoider Arthritis auftreten.

Gegenstand der vorliegenden Erfindung ist die Verwendung der erfindungsgemäßen Substanzen zur Herstellung eines Arzneimittels für die Behandlung und Vorbeugung von Schmerz verschiedenen Ursprungs wie beispielsweise der inflammatorischen Hyperalgesie oder der Arthritis.

Neuere wissenschaftliche Veröffentlichungen weisen auf eine Verwendung von EP4-Inhibitoren zur Vorbeugung und/oder Behandlung von Infektionen der Atmungswege hin. Serezani et al. (Am Respir Cell Mol Biol Vol 37. pp 562-570, 2007) beschreibt, dass über die Aktivierung des EP4-Rezeptors durch PGE2 Makrophagen des Atmungstraktes in ihrer Fähigkeit beeinträchtigt werden, Bakterien zu zerstören. Bakterieninfektionen führen zu einer vermehrten Produktion von Prostaglandinen, unter anderem PGE2, das über diesen Mechanismus die körpereigene Abwehr gegen Bakterien schwächt. Wie in dieser Publikation gezeigt, kann durch eine Inaktivierung des EP4-Rezeptors (und des EP2-Rezeptors) diese Fähigkeit der Bakterienbekämpfung wieder hergestellt werden.

Gegenstand der vorliegenden Erfindung ist die Verwendung der erfindungsgemäßen Substanzen zur Herstellung eines Arzneimittels für die Vorbeugung und Behandlung von Infektionskrankheiten der Lunge. Darmentzündliche Erkrankungen (z.B. Morbus Crohn) stehen ebenfalls im Zusammenhang mit dem Prostaglandin EP4-Rezeptor (Sheibanie et al., 2007, The Journal of Immunology, 178: 8138-8147).

Gegenstand der vorliegenden Erfindung ist die Verwendung der erfindungsgemäßen Substanzen zur Herstellung eines Arzneimittels für die Vorbeugung und Behandlung von darmentzündlichen Erkrankungen.

Bei Knochenmarkstransplantationen kommt es oft zu Komplikationen durch Infektionen, wobei eine Überproduktion von PGE2 im Zusammenhang mit einer verminderten Immunabwehr steht (Ballinger et al., 2006, The Journal of Immunology, 177: 5499-5508). Gegenstand der vorliegenden Erfindung ist die Verwendung der erfindungsgemäßen Substanzen zur Herstellung eines Arzneimittels für die Prophylaxe und Behandlung bei Knochenmarkstransplantationen.

Morbus Basedow (im Englischen "Graves Disease" genannt) ist eine Autoimmunerkrankung der Schilddrüse, bei der das klinische Bild auch pathologische Veränderungen im Auge umfassen kann (endokrine Orbitopathie; Hervortreten der Augäpfel (Exophtalmus)). Hierbei aktivieren einwandernde Lymphozyten vorhandene Fibroblasten, was unter anderem zu einer Anhäufung von Mucopolysachariden führt. Mögliche Folgen sind Beeinträchtigungen des Sehvermögens bis hin zur Blindheit. Untersuchungen zeigen, dass Interleukin-6 eine maßgebende Bedeutung für die pathologischen Mechansimen besitzt und über PGE2 wirkt (Wang et al., 1995, J Clin Endocrinol Metab 80: 3553-3560).

Gegenstand der vorliegenden Erfindung ist die Verwendung der erfindungsgemäßen Substanzen zur Herstellung eines Arzneimittels für die Prophylaxe und Behandlung bei Orbitopathie in Zusammenhang mit Morbus Basedow (Graves Disease) oder anderen pathologischen Erkrankungen des Auges.

Der natürliche Ligand (Agonist) des EP4-Rezeptors ist das PGE2, dessen Synthese über Cyclooxygenasen (COX)-Enzyme (COX-1, COX-2) vermittelt wird. Diese Enzyme sind in den erwähnten Krankheitsbildern, Indikationen und deren Entstehung meist über eine verstärkte Expression und Aktivität beteiligt. Deshalb ist bei allen erwähnten Anwendungsmöglichkeiten eine Kombination eines COX-Inhibitors (COX-2 und/oder COX-1) möglich, mit dem Ziel,
a) eine höhere und effektivere pharmakologische Wirksamkeit als mit einer Substanzklasse zu erreichen und
b) eine niedrigere Dosierung einer der beiden oder beider Substanzklassen zu ermöglichen, was zu einer Reduzierung möglicher Nebenwirkungen und einer besseren Verträglichkeit führt.

Gegenstand der vorliegenden Erfindung sind daher auch Arzneimittel enthaltend eine Verbindung der allgemeinen Formel (I) in Kombination mit einem COX-Inhibitor zur Behandlung von Erkrankungen (Kombinationspräparate). Als COX-Inhibitoren seien beispielsweise die nicht selektiven COX-Inhbitoren wie Aspirin, Naproxen, Indomethacin, Ibuprofen genannt oder die selektiven COX-Inhibitoren Meloxicam, Ketoprofen, Piroxicam, Tenoxicam, Nimesulide, Mefanemic Acid, Ketoralac, Celecoxib (4-[5-(4-methylphenyl)-3-(trifluormethyl)-1H-pyrazol-1-yl]benzenesulfonamide) Parecoxib (N-[4-(5-methyl-3-phenyl-4-isoxazolyl)phenyl]sulfonylpropionamide), Rofecoxib (4-(4-mesylphenyl)-3-phenylfuran-2(5H)-one), Valdecoxib (4-[5-methyl-3-phenyl-4-isoxazoyl]benzenesulfonamide), NS-398 (N-methyl-2-cyclohexanoxy-4-nitrobenzenesulfonamide), Lumiracoxib [2-(2'-chlor-6'-fluorphenyl)-amino-5-methylbenzeneacetic acid], Ceracoxib und Etoricoxib.

Diese Kombinationspräparate können für die Behandlung folgender Erkrankungen eingesetzt werden: infektiöse Erkrankungen, Krebs, Herz-/Kreislauf-Erkrankungen, angiogenetische Erkrankungen, Störungen der Uteruskontraktion, Schmerz, inflammatorische Erkrankungen, neuroinflammatorische Erkrankungen, neurodegenerative Erkrankungen, Autoimmunerkrankungen, immunabhängige Erkrankungen/Therapien, nephrologische Erkrankungen, ophthamologische Erkrankungen.

Im Folgenden sind die alternativen Reaktionsschemata dargestellt, nachdem die erfindungsgemäßen Verbindungen hergestellt werden können, jeweils in Abhängigkeit von der Verfügbarkeit der Ausgangsmaterialien. Für alle Schemata belegen Ausführungsbeispiele die Reaktionsführung detailliert.

Die in den Schemata 1-7 aufgeführten Reste R, R^{1a}, R^{1b}, R⁴, R⁵, R^{5'}, R⁶, R⁷, R⁸, R⁹, R¹⁰, A und B besitzen die im Anspruch aufgeführten Bedeutungen und dienen zur Illustration der Synthese, ohne den Umfang der beanspruchten Verbindungen auf diese Beispiele zu beschränken.

So können beispielsweise durch Umsetzung von substituierten o-Phenylendiaminen der allgemeinen Formel II oder XI mit Aldehyden der Formel III, XXIV oder XXI Benzimidazole der allgemeinen Struktur IV oder XII hergestellt werden. Dieses kann zum Beispiel dadurch erreicht werden, dass die Komponenten II und III in Gegenwart von Säuren und einem Oxidationsmittel erhitzt werden. Die so generierten Verbindungen IV und XII können dann nach in der Literatur bekannten Verfahren an den Stickstoffatomen des Imidazols substituiert werden, vorzugsweise mit Alkylhalogeniden, Oxiranen oder anderen Nucleophilen (Schema 1, Schema 3). Bei dieser Synthesevariante entstehen in der Regel die Isomere Va und Vb oder auch XIIa und Xllb, welche nach gängigen Methoden voneinander getrennt werden können. Gängige Methoden sind Trennverfahren wie beispielsweise Kristallisation, Chromatographie an Kieselgel oder auch Trennungen mittels Hochdruck- oder Hochleistungsflüssigchromatographie.

Carbonsäuren der Formel VI können mit einem Amin nach dem Fachmann bekannten Verfahren zu den erfindungsgemäßen Verbindungen der allgemeinen Formel I umgesetzt werden (Schema 1-4, allgemeine Formel VII).

Die Umsetzung zu Amiden der Formel VII findet beispielsweise statt, indem man eine Carbonsäure der Formel VI in Anwesenheit eines tertiären Amins, beispielsweise Triethylamin, mit Isobutylchloroformiat in ein gemischtes Anhydrid überführt wird, welches mit einem Alkalisalz des entsprechenden Amins in einem inerten Lösungsmittel beziehungsweise Lösungsmittelgemisch, zum Beispiel Tetrahydrofuran, N,N-Dimethylformamid, Dimethoxyethan bei Temperaturen zwischen -30°C und +60°C zu den Zielverbindungen der Formel I reagiert.

Es ist ebenfalls möglich, eine Carbonsäure VI mit Reagenzien wie beispielsweise Dicyclohexylcarbodiimid (DCC), 1-(3-Dimethylaminopropyl)-3-ethylcarbodiimid (EDCI), N-Hydroxybenzotriazol (HOBT), N-[(Dimethylamino)-(3H-[1,2,3]triazolo[4,5-b]pyridin-3-yloxy)methyliden]-N-methylmethanaminiumhexafluorophosphat (HATU) zu aktivieren. Zum Beispiel findet die Umsetzung mit HATU in einem inerten Solvens, beispielsweise N,N-Dimethylformamid, Dimethylsulfoxid in Anwesenheit des entsprechenden Amins und eines tertiären Amins, beispielsweise Triethylamin, Diisopropylethylamin bei Temperaturen zwischen -30°C und +60°C statt.
Es ist ebenfalls möglich, eine Carbonsäure der Formel VI mit einem anorganischen Säurechlorid, beispielsweise Phosphorpentachlorid, Phosphortrichlorid, Thionylchlorid in das entsprechende Carbonsäurechlorid und anschließend in Pyridin oder einem inerten Lösungsmittel, wie zum Beispiel N,N-Dimethylformamid in Anwesenheit des entsprechenden Amins und eines tertiären Amins, beispielsweise Triethylamin bei Temperaturen zwischen -30°C und +60°C in die Zielverbindungen der allgemeinen Formel I zu überführen.

Die erfindungsgemäßen Verbindungen der allgemeinen Formel I lassen sich ebenso aus Aminen der allgemeinen Formel XXXII durch Umsetzung mit Carbonsäuren, Carbonsäurechloriden oder Carbonsäureanhydriden erzielen.

Die erfindungsgemäßen Verbindungen der allgemeinen Formel I lassen sich ebenso aus Bromimidazolen der allgemeinen Formel XIII (XIIIa und/oder Xlllb) unter Palladium(0)-Katalyse durch Umsetzung mit einem entsprechenden Alkohol oder Amin und Kohlenmonoxid (CO) bzw. einer Kohlenmonoxidquelle, wie z.B. Molybdaenhexacarbonyl in einem geeigneten Lösungsmittel beziehungsweise - gemisch, zum Beispiel 1,4-Dioxan/Wasser oder Tetrahydrofuran, Zugabe einer Base wie beispielsweise Natriumcarbonat oder 1,8-Diazabicyclo(5.4.0)undec-7-en (DBU) sowie eines Katalysator-Liganden-Gemischs, zum Beispiel Palladium(II)-acetat oder trans-Bis(acetato)bis[o-(di-o-tolylphosphino)benzyl]dipalladium(II) / Tri-tert-butylphosphinotetrafluoroborat bei Temperaturen zwischen 80°C und 160°C (ggf. unter Mikrowellenbestrahlung zwischen 80-200 Watt), und im Falle der Verwendung von Kohlenmonoxid bei einem CO-Druck von 5-15bar, erhalten (Schema 3, Schema 4). Diese Methode ist nicht auf Methylester, d.h. auf die Verwendung von Methanol beschränkt, sondern auch auf andere Ester erweiterbar. So lassen sich beispielsweise durch Verwendung von Ethanol anstelle Methanol auf diese Art und Weise die entsprechenden Ethylester synthetisieren.

Die Carbonsäuren der allgemeinen Formel VI lassen sich beispielsweise aus Estern der Formel Va durch Esterverseifung in einem geeigneten Lösungsmittel beziehungsweise Lösungsmittelgemisch, zum Beispiel Methanol, Ethanol, Tetrahydrofuran, Wasser unter Zusatz einer wässrigen Lösung eines Alkalihydroxids, zum Beispiel Natriumhydroxid, Lithiumhydroxid bei Temperaturen zwischen 20°C und 60°C erhalten (Schema 1 -4).

Die Verbindungen der allgemeinen Formel XXIV lassen sich beispielsweise aus den entsprechenden Anilinen XX herstellen, indem XX nach dem Fachmann bekannten Verfahren zu Formel XXII cyclisiert und anschließend zu XXIII oxidiert werden. Die so generierten Verbindungen XXIII können nach in der Literatur bekannten Verfahren am Stickstoff des Carbazols alkyliert werden (Schema 5) und dann in Reaktionen eingesetzt werden, in denen die erfindungsgemäßen Benzimidazole der Formel I dargestellt werden.

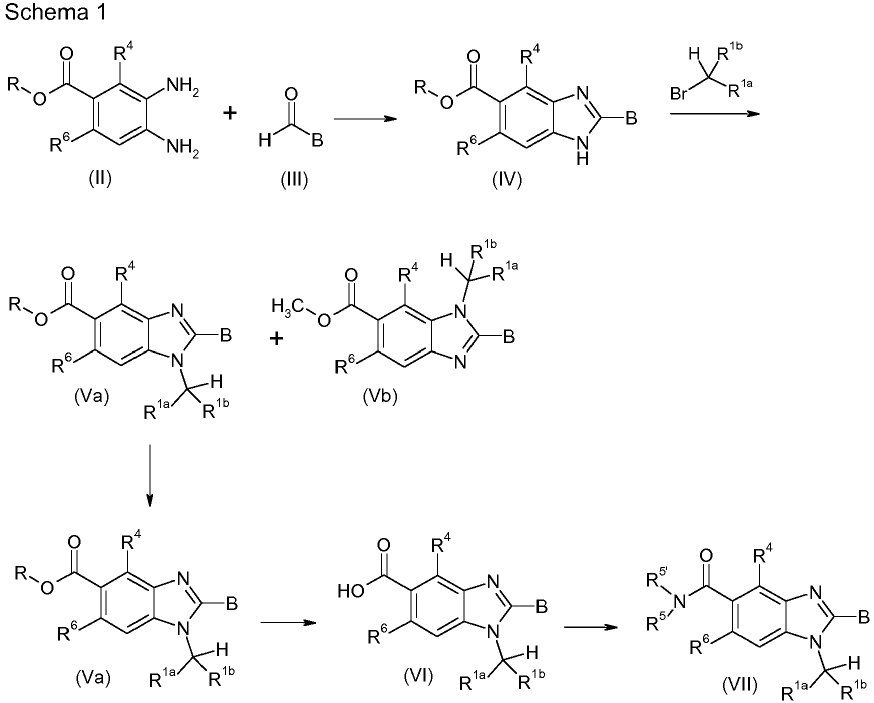

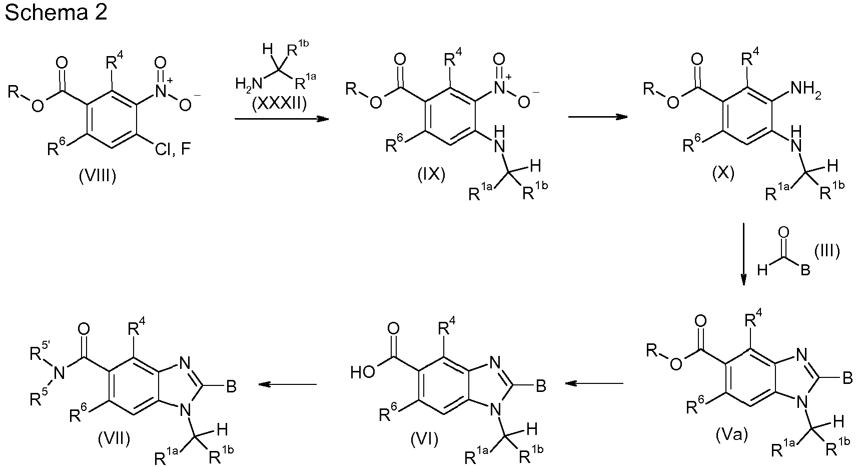

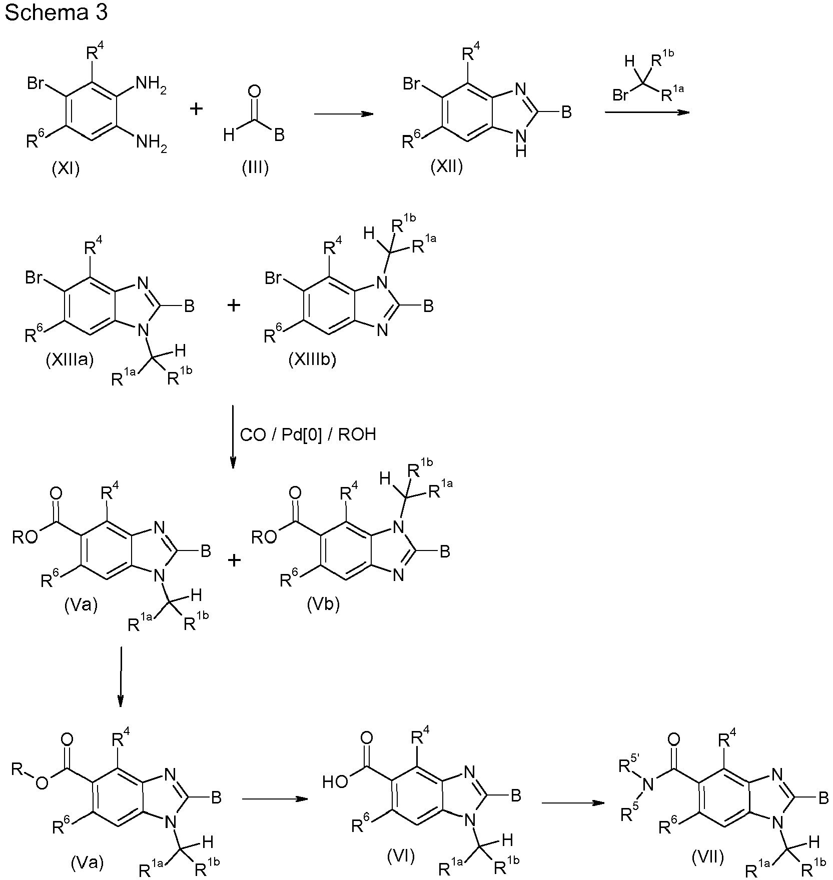

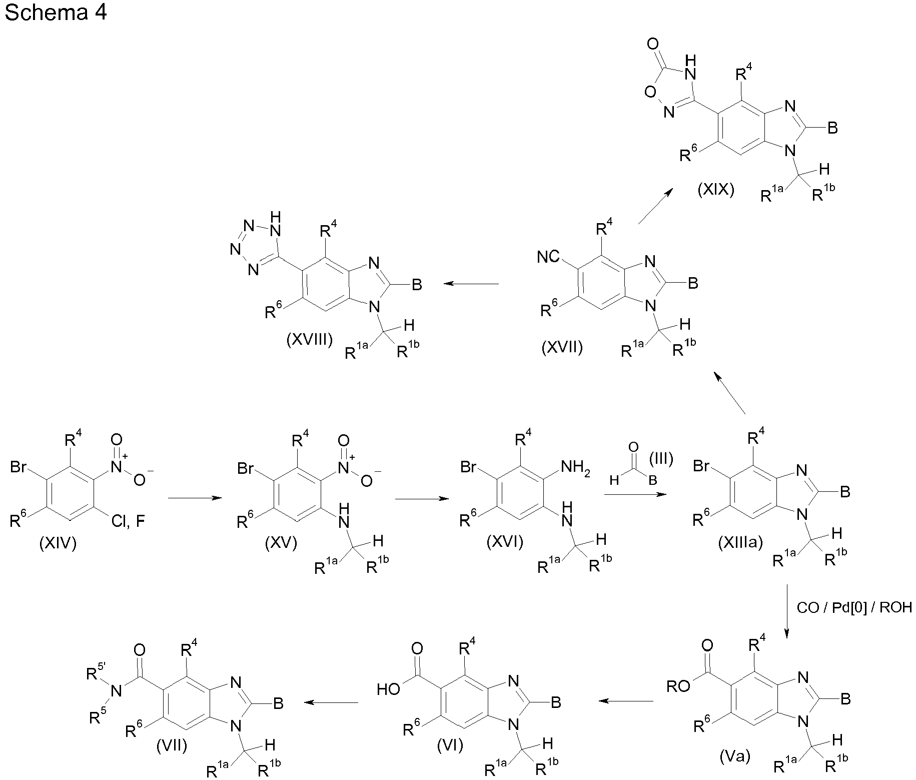

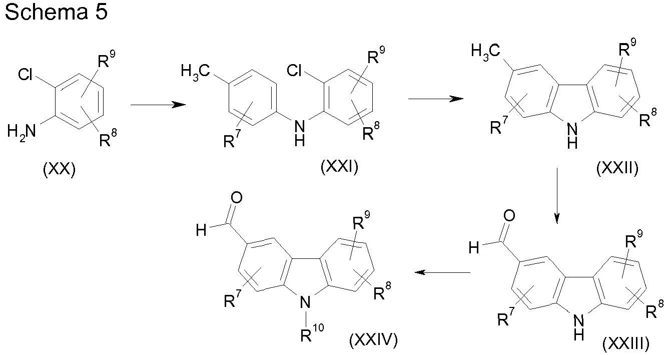

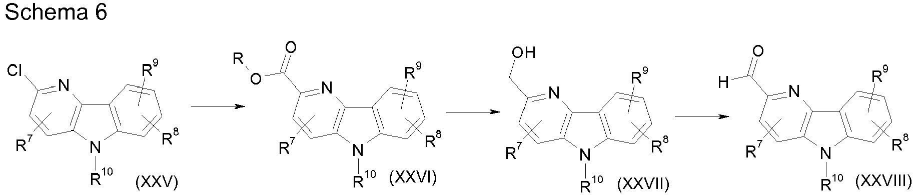

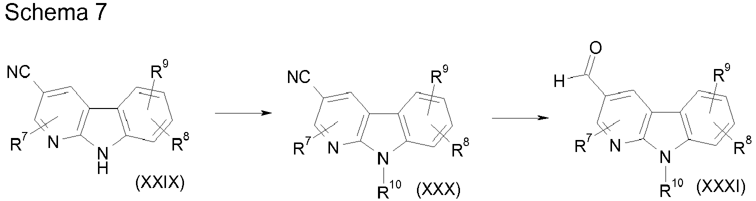

### Herstellung der erfindungsgemäßen Verbindungen

Die nachfolgenden Beispiele erläutern die Herstellung der erfindungsgemäßen Verbindungen der allgemeinen Formel (I), ohne den Umfang der beanspruchten Verbindungen auf diese Beispiele zu beschränken.

Die erfindungsgemäßen Verbindungen der allgemeinen Formel (I) lassen sich wie nachstehend beschrieben herstellen und charakterisieren.

### Abkürzungen

- DCM: Dichlormethan
- DMF: N,N-Dimethylformamid
- DMSO: Dimethylsulfoxid
- h: Stunde(n)
- HPLC: Hochdruck-, Hochleistungsflüssigchromatographie
- M: molar
- min: Minute(n)
- N: normal
- NMR: Kernresonanzspektroskopie
- RT: Raumtemperatur
- THF: Tetrahydrofuran

NMR-Peak Formen sind so angegeben wie sie im Spektrum erscheinen, mögliche Effekte höherer Ordnung wurden nicht berücksichtigt.

### Experimenteller Teil

### Intermediat 1

### Ethyl-4-[(2-methoxyethyl)amino]-3-nitrobenzoat

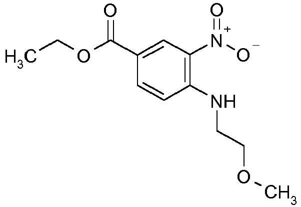

40.0 g (0.17 mol) Ethyl-4-chlor-3-nitrobenzoat wurden in 200 mL DMSO gegeben, 20.9 g (0.28 mol) 2-Methoxyethanamin hinzugefügt, 6 h auf 60 °C erhitzt und dann über Nacht auf RT abgekühlt. Das Reaktionsgemisch wurde auf 200 mL gesättigte Natriumhydrogencarbonatlösung gegossen, der entstandene Niederschlag abfiltriert und mit 100 mL Wasser gewaschen. Der Niederschlag wurde getrocknet. So wurden 45.5g (78%) der Titelverbindung erhalten.
¹H-NMR (300 MHz, DMSO-d6), δ [ppm]= 1.31 (t, 3H), 3.31 - 3.32 (m, 1H), 3.57 - 3.62 (m, 4H), 4.29 (q, 2H), 7.19 (d, 1H), 7.97 (dd, 1H), 8.50 - 8.56 (m, 1H), 8.61 (d, 1H).

### Intermediat 2

### 4-[(2-Methoxyethyl)amino]-3-nitrobenzoesäure

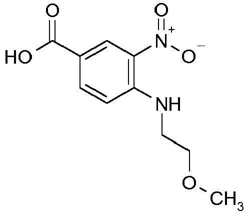

26.0 g (0.097 mol) Ethyl-4-[(2-methoxyethyl)amino]-3-nitrobenzoat wurden in 100 mL Ethanol gegeben, mit 55 mL 2M Natronlauge versetzt und 1 h zum Rückfluss erhitzt. Nach dem Abkühlen wurden 75 mL 2M Salzsäure hinzugefügt und fünfmal mit Dichlormethan extrahiert. Die gesammelten organischen Phasen wurden über Natriumsulfat getrocknet, filtriert und eingeengt. So wurden 21.8 g (93%) der Titelverbindung erhalten.
¹H-NMR (300 MHz, DMSO-d6), δ [ppm]= 3.31 (s, 3H), 3.57 -3.61 (4H), 7.17 (d, 1H), 7.96 (dd, 1H), 8.47 - 8.53 (br. s., 1H), 8.61 (d, 1H), 12.40 - 13.30 (1H).

### Intermediat 3

### 3-Amino-4-[(2-methoxyethyl)amino]benzoesäure

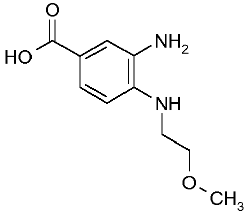

21,8 g (0.09 mol) 4-[(2-Methoxyethyl)amino]-3-nitrobenzoesäure wurden in 500 mL Ethanol gelöst, mit 2,5g Palladium/Kohle (10%) versetzt und bei RT unter Einleitung von Wasserstoff 4 h gerührt. Danach wurden erneut 2,5g Palladium/Kohle (10%) hinzugefügt und weitere 2 h Wasserstoff eingeleitet. Der Katalysator wurde abfiltriert und die Lösung eingeengt. So wurden 19.0 g (82%) der Titelverbindung erhalten.
¹H-NMR (400 MHz, DMSO-d6), δ [ppm]= 3.23 - 3.31 (m, 5H), 3.41 - 3.48 (m, 2H), 5.35 - 5.65 (1H), 5.70 - 6.20 (2H), 6.15 (d, 1H), 6.70 (s, 1H), 7.16 (d, 1H), 11.40 - 12.50 (1H).

### Intermediat 4

### Ethyl-3-amino-4-[(2-methoxyethyl)amino]benzoat

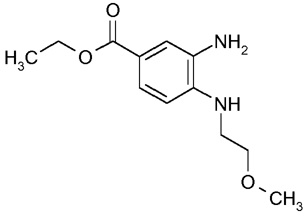

Ethyl-3-amino-4-[(2-methoxyethyl)amino]benzoat wurde analog Intermediat 3 aus Ethyl-4-[(2-methoxyethyl)amino]-3-nitrobenzoat durch Reduktion mit Wasserstoff an Palladium hergestellt.
¹H-NMR (400 MHz, DMSO-d6), δ [ppm]= 1.25 (t, 3H), 3.25 - 3.31 (m, 5H), 3.42 - 3.47 (m, 2H), 4.13 - 4.20 (m, 2H), 6.02 (br. s., 1H), 6.17 (d, 1H), 6.71 (d, 1H), 7.18 (dd, 1H), NH2 nicht angegeben.

### Intermediat 5

### Methyl-3-amino-2-fluor-4-[(2-methoxyethyl)amino]benzoat

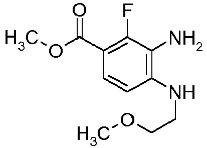

Unter Stickstoff wurden bei -78°C zu einer Lösung von 29.4 g (0.23 mol) Oxalylchlorid in 315 mL DCM innerhalb von 20 min eine Lösung aus 16.0 g (0.21 mol) 2-Methoxyethanol in 315 mL DCM getropft, 1 h gerührt, dann bei fortgesetzter Kühlung 88 mL (0.63 mol) Triethylamin hinzugetropft und anschließend die Kühlung entfernt. Nach Erwärmung auf RT wurde das so erhaltene Reaktionsgemisch zu einer Lösung von 9.5 g (40.4 mmol) 4-Brom-3-fluor-2-nitroanilin in 50 mL DCM gegeben, nacheinander 60 g (0.28 mol) Natriumtriacetoxyborhydrid und langsam 62 mL (0.81 mol) Trifluoressigsäure hinzugegeben und das Reaktionsgemisch danach 17 h bei RT gerührt. Anschließend wurde filtriert, das Filtrat lansam mit 0.5 I Ammoniaklösung (33%ig) versetzt, mehrmals mit DCM extrahiert, die gesammelten organische Phasen bis zur Trockene eingedampft und der Rückstand über Kieselgel aufgereinigt (13,7 g).

13.7 g des erhaltenen Rohprodukts wurden in 500 mL Methanol gelöst, mit 7.6 g (9.3 mmol) 1,1-Bis- (diphenylphosphino)-ferrocen-palladium(II)dichlorid und 18.4 g (0.19 mol) Kaliumacetat versetzt und unter Kohlenmonoxid bei 13 bar und 100 °C für 19 h in einem Autoklaven gerührt. Nach dem Abkühlen auf RT wurde das Gemisch filtriert, das Filtrat im Vakuum eingeengt und das so erhaltene rohe Methyl-3-amino-2-fluor-4-[(2-methoxyethyl)amino]benzoat (9.0 g, 92%) ohne weitere Reinigung in die nächste Stufe eingesetzt.
¹H-NMR (400 MHz, DMSO-d6), δ [ppm]= 3.28 (s, 3H), 3.29 - 3.22 (m, 2H), 3.50 - 3.54 (m, 2H), 3.73 (s, 3H), 4.64 (s, 2H), 5.59 (t, 1H), 6.34 (d, 1H), 7.11 (t, 1H).

### Intermediate 6 und 7

### 8-Chlor-9-ethyl-6-methyl-9H-carbazol-3-carbaldehyd und 5-Chlor-9-ethyl-6-methyl-9H-carbazol-3-carbaldehyd

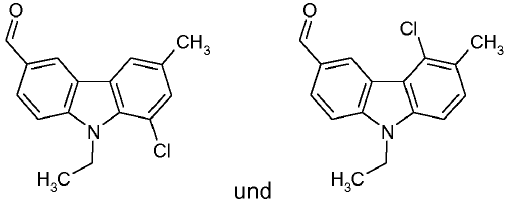

2.0 g (8.43 mmol) 9-Ethyl-6-methyl-9H-carbazol-3-carbaldehyd und 1.1 g (9.27 mmol) N-Chlor-succinimid (NCS) wurden in 29 mL Acetonitril gegeben und 3 h bei 60° C gerührt. Dann wurde das Reaktionsgemisch in Ethylacetat aufgenommen, die organische Phase mit Wasser gewaschen, eingedampft und der Eindampfrückstand chromatographisch an Kieselgel (Hexan/Ethylacetat 9:1 -> 7:3) gereinigt. So wurden 1.2 g (52%) 8-Chlor-9-ethyl-6-methyl-9H-carbazol-3-carbaldehyd und 0.7 g (31%) 5-Chlor-9-ethyl-6-methyl-9H-carbazol-3-carbaldehyd erhalten.

### 8-Chlor-9-ethyl-6-methyl-9H-carbazol-3-carbaldehyd

¹H-NMR (400MHz, DMSO-d6), δ [ppm]= 1.37 (t, 3H), 2.47 (s, 3H), 4.78 (q, 2H), 7.38 (s, 1H), 7.79 (d, 1H), 7.98 - 8.03 (m, 1H), 8.07 (s, 1H), 8.71 (s, 1H), 10.05 (s, 1H).

### 5-Chlor-9-ethyl-6-methyl-9H-carbazol-3-carbaldehyd

¹H-NMR (400 MHz, DMSO-d6), δ [ppm]= 1.32 (t, 3H), 4.51 (q, 2H), 7.51 (d, 1H), 7.63 (d, 1H), 7.81 (d, 1H), 8.03 (dd, 1H), 9.01 (d, 1H), 10.08 (s, 1H).

### Intermediat 8

### Methyl-3-amino-4-[(2-methoxyethyl)amino]-2-methylbenzoat

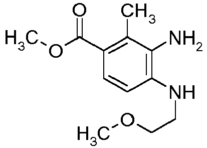

27.5 g (0.12 mol) eines Gemisches aus Methyl-4-chlor-2-methyl-5-nitrobenzoat und Methyl-4-chlor-2-methyl-3-nitrobenzoat, hergestellt nach M. Baumgarth et al., J. Med. Chem. 1997, 40, 2017-2034, wurden in 50 mL DMSO gegeben, mit 31 mL (0.36 mol) 2-Methoxyethanamin versetzt und 25 h bei 80° C gerührt. Dann wurde mit Wasser versetzt, mehrfach mit DCM extrahiert und die gesammelten organischen Phasen eingedampft. Der Rückstand wurde chromatographisch an Kieselgel (Hexan/DCM 1:0 → 0:1) aufgetrennt. Auf diese Weise wurden 9.3 g (29%) Methyl-4-[(2-methoxyethyl)amino]-2-methyl-3-nitrobenzoat und 15.5 g (49%) Methyl-4-[(2-methoxyethyl)amino]-2-methyl-5-nitrobenzoat erhalten.

### Methyl-4-[(2-methoxyethyl)amino]-2-methyl-3-nitrobenzoat

¹H-NMR (400 MHz , DMSO-d6), δ [ppm]= 2.37 (s, 3H), 3.26 (s, 3H), 3.36 (q, 2H), 3.47 (t, 2H), 3.77 (s, 3H), 6.42 (t, 1H), 6.86 (d, 1H), 7.83 (d, 1H).

### Methyl-4-[(2-methoxyethyl)amino]-2-methyl-5-nitrobenzoat

¹H-NMR (400 MHz , DMSO-d6), δ [ppm]= 2.55 (s, 3H), 3.32 (s, 3H), 3.56 - 3.62 (m, 4H), 3.79 (s, 3H), 6.99 (s, 1H), 8.39 - 8.44 (m, 1H), 8.64 (s, 1H).

3.33 g (12.4 mmol) Methyl-4-[(2-methoxyethyl)amino]-2-methyl-3-nitrobenzoat wurden in 80 mL THF/Methanol (1:1) gelöst und unter Normaldruck an Palladium (10%ig auf Kohlenstoff) hydriert. Der Katalysator wurde abfiltriert und das Filtrat eingeengt. So wurden 2.85 g (92%) rohes Methyl-3-amino-4-[(2-methoxyethyl)amino]-2-methylbenzoat erhalten, welches ohne weitere Reinigung in folgende Stufen eingesetzt wurde.
¹H-NMR (400 MHz, DMSO-d6), δ [ppm]= 2.31 (s, 3H), 3.25 - 3.31 (m, 5H), 3.53 (t, 2H), 3.70 (s, 3H), 4.44 (br, 2H), 5.20 (t, 1H), 6.37 (d, 1H), 7.18 (d, 1H).

### Intermediat 9

### 9-Ethyl-8-fluor-6-methyl-9H-carbazol-3-carbonsäure

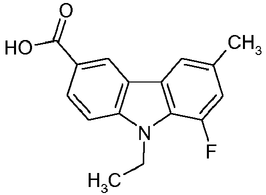

Eine Mischung aus 2.49 g (11.8 mmol) [5-(Ethoxycarbonyl)-2-fluorphenyl]boronsäure, 1.60 g (7.8 mmol) 2-Brom-6-fluor-4-methylanilin, 0.96 g (1.2 mmol) 1,1'-Bis(diphenyl-phosphino)ferrocendichloropalladium(II) und 2.60 g (18.8 mmol) Kaliumcarbonat wurden in 79 mL Tetrahydrofuran 3h zum Rückfluss erhitzt, nach dem Abkühlen mit Wasser versetzt und dann mit Ethylacetat extrahiert. Die gesammelten organischen Phasen wurden mit Wasser neutral gewaschen, mit Natriumsulfat getrocknet, zur Trockene eingedampft und der Rückstand an Kieselgel aufgereinigt. Das erhaltene rohe Ethyl-2'-amino-3',6-difluor-5'-methylbiphenyl-3-carboxylat wurde so in die nächste Stufe eingesetzt.

557 mg (1.91 mmol) rohes Ethyl-2'-amino-3',6-difluor-5'-methylbiphenyl-3-carboxylat wurden in 17 mL DMF gegeben, mit 268 mg (6.69 mmol) Natriumhydrid (60%ige Mineralöldispersion) versetzt und in einem Druckrohr 90 h auf 90°C erhitzt. Nach dem Erkalten wurde vorsichtig auf Eiswasser gegossen und mit DCM extrahiert. Die wässrige Phase wurde auf pH 4 angesäuert, nochmals mit DCM extrahiert und dann mit der zuerst erhaltenen organischen Phase mittels Natriumsulfat getrocknet und bis zur Trockene eingedampft. Auf diese Weise wurde ein Rohgemisch (419 mg) aus Ethyl-8-fluor-6-methyl-9H-carbazol-3-carboxylat und 8-Fluor-6-methyl-9H-carbazol-3-carbonsäure erhalten, welches direkt in die nächste Stufe eingesetzt wurde.

419 mg eines Gemisches aus Ethyl-8-fluor-6-methyl-9H-carbazol-3-carboxylat und 8-Fluor-6-methyl-9H-carbazol-3-carbonsäure wurden in 23 mL Aceton gegeben, 2.8 g (8.6 mmol) Cäsiumcarbonat und 1.0 mL (12.9 mmol) Ethyliodid hinzugefügt und 24 h auf 45° C erhitzt. Nach dem Erkalten wurde filtriert, der Filterrückstand mit Aceton und Ethylacetat nachgewaschen und das Filtrat bis zur Trockene eingedampft. Der Rückstand wurde in 1.5 mL Ethanol aufgenommen, mit 2 mL 2N Natronlauge versetzt und 5 h auf 45°C erhitzt. Dann wurde mit 1M Salzsäure auf pH 2 angesäuert, mit Ethylacetat extrahiert, mit Natriumsulfat getrocknet und anschließend bis zur Trockene eingedampft. Auf diese Weise wurden 435 mg (14%, 4 Stufen) 9-Ethyl-8-fluor-6-methyl-9H-carbazol-3-carbonsäure erhalten.
¹H-NMR (400 MHz, DMSO-d6), δ [ppm]= 1.35 (t, 3H), 2.47 (s, 3H), 4.53 (q, 2H), 7.17 (d, 1H), 7.69 (d, 1H), 7.91 (s, 1H), 8.07 (dd, 1H), 8.74 (d, 1H), 12.61 (s, 1H).

### Intermediat 10

### 9-Ethyl-6-fluor-8-methyl-9H-carbazol-3-carbonsäure

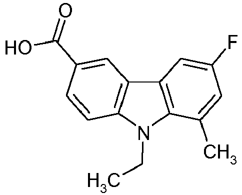

9-Ethyl-6-fluor-8-methyl-9H-carbazol-3-carbonsäure wurde analog der bei Intermediat 35 beschriebenen Synthese für 9-Ethyl-8-fluor-6-methyl-9H-carbazol-3-carbonsäure aus 2.75 (13.5 mmol) 2-Brom-4-fluor-6-methylanilin und 2.6 g (12.3 mmol) [5-(Ethoxycarbonyl)-2-fluorphenyl]boronsäure über 4 Stufen hergestellt (543mg, 20%).
¹H-NMR (400 MHz, DMSO-d6), δ [ppm]= 1.33 (t, 3H), 2.79 (s, 3H), 4.63 (q, 2H), 7.15 (dd, 1H), 7.67 (d, 1H), 7.99 (dd, 1H), 8.05 (dd, 1H), 8.78 (d, 1H), 12.59 (br. s., 1H).

### Beispiel 1

### Methyl-2-(6-brom-9-ethyl-9H-carbazol-3-yl)-1-(2-methoxyethyl)-4-methyl-1H-benzimidazol-5-carboxylat

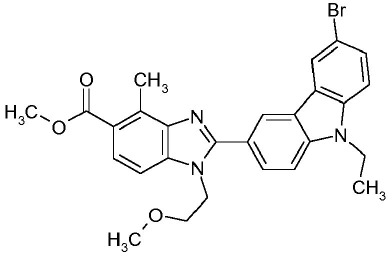

180 mg (0.94 mmol) Natriumdisulfit wurden in 0.9 mL Wasser gegeben, dann 100 mg (0.4 mmol) Methyl-3-amino-4-[(2-methoxyethyl)amino]-2-methylbenzoat (Intermediat 8) und 127 mg (0.4 mmol) 6-Brom-9-ethyl-9H-carbazol-3-carbaldehyd hinzugefügt und dann 1.3 mL THF hinzugegeben. Es wurde zunächst bei Raumtemperatur gerührt und anschließend 1 h zum Rückfluss erhitzt. Nach dem Abkühlen wurde das Reaktionsgemisch eingeengt und der Eindampfrückstand chromatographisch aufgereinigt. So wurden 139 mg (63%) Methyl-2-(6-brom-9-ethyl-9H-carbazol-3-yl)-1-(2-methoxyethyl)-4-methyl-1H-benzimidazol-5-carboxylat als Feststoff erhalten.
¹H-NMR (400 MHz, DMSO-d6), δ [ppm]= 1.36 (t, 3H), 2.89 (s, 3H), 3.09 (s, 3H), 3.66 (t, 2H), 3.87 (s, 3H), 4.48 - 4.61 (m, 4H), 7.59 - 7.72 (m, 3H), 7.80 - 7.86 (m, 2H), 7.97 (dd, 1H), 8.55 (d, 1H), 8.69 (d, 1H).

### Beispiel 2

### 2-(6-Brom-9-ethyl-9H-carbazol-3-yl)-1-(2-methoxyethyl)-4-methyl-1H-benzimidazol-5-carbonsäure

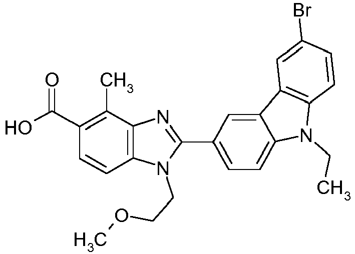

130 mg (0.25 mmol) Methyl-2-(6-brom-9-ethyl-9H-carbazol-3-yl)-1-(2-methoxyethyl)-4-methyl-1H-benzimidazol-5-carboxylat wurden in ein Gemisch aus 2 mL Methanol und 2 mL THF gegeben, mit 2 mL 2.0 M Natronlauge versetzt und für 8 h zum Rückfluss erhitzt. Nach dem Abkühlen auf RT wurde dreimal mit Ethylacetat extrahiert und die organischen Phasen verworfen. Die erhaltene wässrige Phase wurde mit 2 M Salzsäure auf pH 3 angesäuert, mehrmals mit Ethylacetat extrahiert und die so erhaltenen organischen Phasen bis zur Trockene eingeengt. So wurden 96 mg (74%) der Titelverbindung erhalten.
¹H-NMR (400 MHz, DMSO-d6), δ [ppm]= 1.36 (t, 3H), 2.89 (s, 3H), 3.10 (s, 3H), 3.67 (t, 2H), 4.48 - 4.59 (m, 4H), 7.55 (d, 1H), 7.64 (dd, 1H), 7.69 (d, 1H), 7.79 - 7.86 (m, 2H), 7.96 (dd, 1H), 8.54 (d, 1H), 8.69 (d, 1H), 12.20 - 13.00 (br., 1H).

### Beispiel 3

### Methyl-2-(9-ethyl-8-fluor-6-methyl-9H-carbazol-3-yl)-1-(2-methoxyethyl)-4-methyl-1H-benzimidazol-5-carboxylat

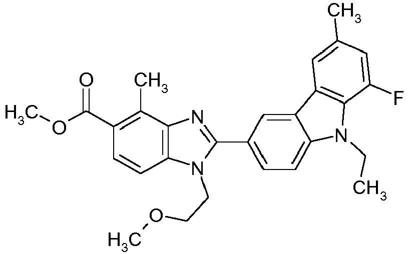

Eine Mischung aus 100 mg (0.42 mmol) Methyl-3-amino-4-[(2-methoxyethyl)amino]-2-methylbenzoat (Intermediat 8), 114 mg (0.42 mmol) 9-Ethyl-8-fluor-6-methyl-9H-carbazol-3-carbonsäure (Intermediat 9) und 1.25 mL (2.1 mmol) Propanphosphonsäureanhydrid-Lösung (50%ig in DMF) in 2 mL Pyridin wurden 90 h in einem Druckrohr auf 100°C erhitzt. Nach dem Erkalten wurde auf Wasser gegossen, mehrmals mit Ethylacetat extrahiert, die gesammelten organischen Phasen mit Natriumsulfat getrocknet, bis zur Trockene eingedampft und der Rückstand mittels präparativer HPLC aufgereinigt. Auf diese Weise wurden 60 mg (30%) Methyl-2-(9-ethyl-8-fluor-6-methyl-9H-carbazol-3-yl)-1-(2-methoxyethyl)-4-methyl-1H-benzimidazol-5-carboxylat erhalten.
¹H-NMR (300MHz, DMSO-d6): δ [ppm]= 1.39 (t, 3H), 2.49 (s, 3H), 2.88 (s, 3H), 3.11 (s, 3H), 3.68 (t, 2H), 3.87 (s, 3H), 4.53 - 4.61 (m, 4H), 7.21 (d, 1H), 7.62 (d, 1H), 7.80 - 7.87 (m, 2H), 7.91 - 7.99 (m, 2H), 8.63 (d, 1H).

### Beispiel 4

### 2-(9-Ethyl-8-fluor-6-methyl-9H-carbazol-3-yl)-1-(2-methoxyethyl)-4-methyl-1H-benzimidazol-5-carbonsäure

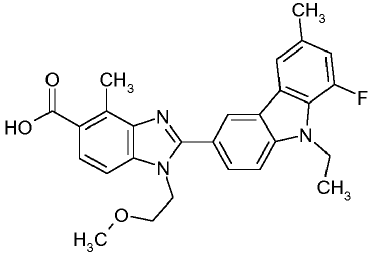

60 mg (0.13 mmol) Methyl-2-(9-ethyl-8-fluor-6-methyl-9H-carbazol-3-yl)-1-(2-methoxyethyl)-4-methyl-1H-benzimidazol-5-carboxylat wurden 10 mL Methanol aufgenommen, mit 0.32 mL (0.63 mmol) 2M Natronlauge versetzt und 17 h auf 60°C erhitzt. Nach dem Erkalten wurde eingedampft, der Rückstand mit 2M Salzsäure auf pH 3 gebracht, mehrmals mit Ethylacetat extrahiert, die gesammelten organischen Phasen eingedampft und an Kieselgel aufgereinigt. Auf diese Weise wurden 40 mg (69%) der Titelverbindung erhalten.
¹H-NMR (400 MHz, DMSO-d6), δ [ppm]= 1.39 (t, 3H), 2.49 (s, 3H), 2.89 (s, 3H), 3.12 (s, 3H) 3.69 (t, 2H), 4.52 - 4.64 (m, 4H), 7.20 (d, 1H), 7.60 (d, 1H), 7.81 - 7.99 (m, 4H), 8.63 (d, 1H), 12.40 - 12.75 (br., 1H).

### Beispiel 5

### Methyl-2-(9-ethyl-6-fluor-8-methyl-9H-carbazol-3-yl)-1-(2-methoxyethyl)-4-methyl-1H-benzimidazol-5-carboxylat

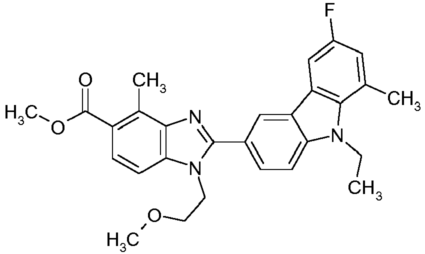

In Analogie zu Beispiel 3 wurden aus 100 mg (0.42 mmol) Methyl-3-amino-4-[(2-methoxyethyl)amino]-2-methylbenzoat (Intermediat 8) und 114 mg (0.42 mmol) 9-Ethyl-6-fluor-8-methyl-9H-carbazol-3-carbonsäure (Intermediat 10) Methyl-2-(9-ethyl-6-fluor-8-methyl-9H-carbazol-3-yl)-1-(2-methoxyethyl)-4-methyl-1H-benzimidazol-5-carboxylat hergestellt.
¹H-NMR (400 MHz, DMSO-d6), δ [ppm]= 1.38 (t, 3H), 2.84 (s, 3H), 2.90 (s, 3H), 3.11 (s, 3H), 3.74 (t, 2H), 3.91 (s, 3H), 4.66 - 4.75 (m, 4H), 7.23 (dd, 1H), 7.90 - 8.07 (m, 5H), 8.78 (d, 1H).

### Beispiel 6

### 2-(9-Ethyl-6-fluor-8-methyl-9H-carbazol-3-yl)-1-(2-methoxyethyl)-4-methyl-1H-benzimidazol-5-carbonsäure

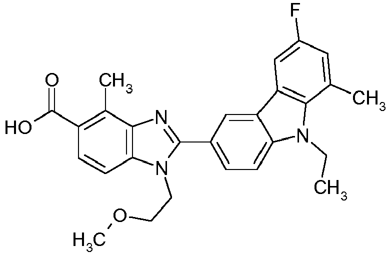

In Analogie zu Beispiel 4 wurde aus Methyl-2-(9-ethyl-6-fluor-8-methyl-9H-carbazol-3-yl)-1-(2-methoxyethyl)-4-methyl-1H-benzimidazol-5-carboxylat die Titelverbindung hergestellt (38 mg, 20% über 2 Stufen).
¹H-NMR (400 MHz, DMSO-d6), δ [ppm]= 1.37 (t, 3H), 2.83 (s, 3H), 2.89 (s, 3H), 3.10 (s, 3H), 3.67 (t, 2H), 4.54 (t, 2H), 4.68 (q, 2H), 7.17 (dd, 1H), 7.56 (d, 1H), 7.80 (d, 1H), 7.84 (d, 1H), 7.93 (dd, 1H), 7.98 (dd, 1H), 8.61 (d, 1H), 12.00 - 12.70 (br., 1H).

### Beispiel 7

### 2-(9-Ethyl-6-fluor-8-methyl-9H-carbazol-3-yl)-4-fluor-1-(2-methoxyethyl)-1H-benzimidazol-5-carbonsäure

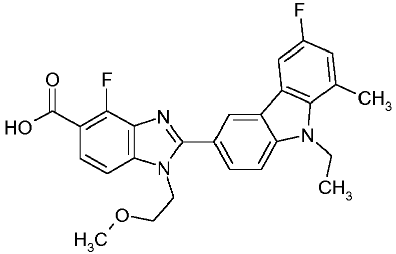

In Analogie zu Beispiel 3 wurden aus 100 mg (0.41 mmol) Methyl-3-amino-2-fluor-4-[(2-methoxyethyl)amino]benzoat (Intermediat 5) und 93 mg (0.34 mmol) 9-Ethyl-6-fluor-8-methyl-9H-carbazol-3-carbonsäure (Intermediat 10) zunächst Methyl-2-(9-ethyl-6-fluor-8-methyl-9H-carbazol-3-yl)-4-fluor-1-(2-methoxyethyl)-1H-benzimidazol-5-carboxylat hergestellt, welches dann analog zu Beispiel 4 zur Titelverbindung umgesetzt wurde (6 mg, 3% über 2 Stufen).
¹H-NMR (400 MHz, DMSO-d6), δ [ppm]= 1.37 (t, 3H), 2.83 (s, 3H), 3.12 (s, 3H), 3.69 (t, 2H), 4.56 (t, 2H), 4.68 (q, 2H), 7.17 (dd, 1H), 7.51 (d, 1H), 7.72 (t, 1H), 7.81 (d, 1H), 7.97 (td, 2H), 8.65 (d, 1H), COOH nicht angegeben.

### Beispiel 8

### 2-(9-Ethyl-8-fluor-6-methyl-9H-carbazol-3-yl)-4-fluor-1-(2-methoxyethyl)-1H-benzimidazol-5-carbonsäure

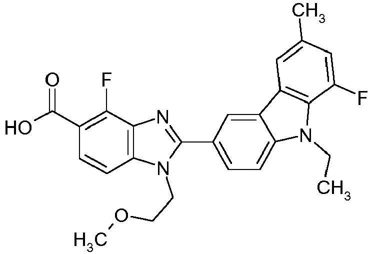

In Analogie zu Beispiel 3 wurden aus 100 mg (0.41 mmol) Methyl-3-amino-2-fluor-4-[(2-methoxyethyl)amino]benzoat (Intermediat 5) und 93 mg (0.34 mmol) 9-Ethyl-8-fluor-6-methyl-9H-carbazol-3-carbonsäure (Intermediat 9) zunächst Methyl-2-(9-ethyl-8-fluor-6-methyl-9H-carbazol-3-yl)-4-fluor-1-(2-methoxyethyl)-1H-benzimidazol-5-carboxylat hergestellt, welches dann analog zu Beispiel 4 zur Titelverbindung umgesetzt wurde (57 mg, 35% über 2 Stufen).
¹H-NMR (400 MHz, DMSO-d6), δ [ppm]= 1.39 (t, 3H), 2.48 (s, 3H), 3.12 (s, 3H), 3.70 (t, 2H), 4.54 - 4.62 (m, 4H), 7.20 (d, 1H), 7.55 (d, 1H), 7.75 (dd, 1H), 7.83 (d, 1H), 7.92 (s, 1H), 7.97 (dd, 1H), 8.65 (d, 1H), COOH nicht angegeben.

### Beispiel 9

### Ethyl-2-(9-ethyl-8-fluor-6-methyl-9H-carbazol-3-yl)-1-(2-methoxyethyl)-1H-benzimidazol-5-carboxylat

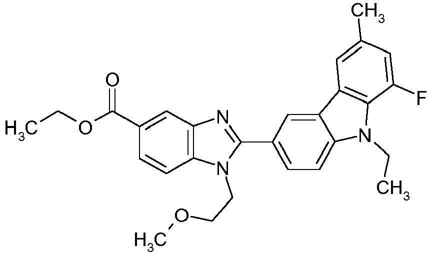

In Analogie zu Beispiel 3 wurden aus 100 mg (0.42 mmol) Ethyl-3-amino-4-[(2-methoxyethyl)amino]benzoat (Intermediat 4) und 95 mg (0.35 mmol) 9-Ethyl-8-fluor-6-methyl-9H-carbazol-3-carbonsäure (Intermediat 9) Ethyl-2-(9-ethyl-8-fluor-6-methyl-9H-carbazol-3-yl)-1-(2-methoxyethyl)-1H-benzimidazol-5-carboxylat hergestellt (41 mg, 20%).
¹H-NMR (400 MHz, DMSO-d6), δ [ppm]= 1.38 (m, 6H), 2.47 (s, 3H), 3.13 (s, 3H), 3.71 (t, 2H), 4.36 (q, 2H), 4.54 - 4.63 (m, 4H), 7.21 (d, 1H), 7.82 (dd, 2H), 7.89 - 7.99 (m, 3H), 8.29 (d, 1H), 8.64 (d, 1H).

### Beispiel 10

### 2-(9-Ethyl-8-fluor-6-methyl-9H-carbazol-3-yl)-1-(2-methoxyethyl)-1H-benzimidazol-5-carbonsäure

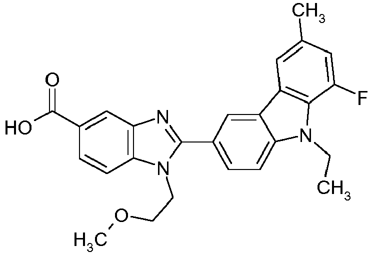

In Analogie zu Beispiel 4 wurde aus Ethyl-2-(9-ethyl-8-fluor-6-methyl-9H-carbazol-3-yl)-1-(2-methoxyethyl)-1H-benzimidazol-5-carboxylat die Titelverbindung hergestellt (23 mg, 59%).
¹H-NMR (400 MHz, DMSO-d6), δ [ppm]= 1.39 (t, 3H), 2.47 (s, 3H), 3.13 (s, 3H), 3.72 (t, 2H), 4.53 - 4.63 (m, 4H), 7.20 (d, 1H), 7.76 (d, 1H), 7.82 (d, 1H), 7.88 - 8.00 (m, 3H), 8.26 (d, 1H), 8.64 (d, 1H), 12.45 - 13.05 (br., 1H).

### Beispiel 11

### Methyl-2-(8-chlor-9-ethyl-6-methyl-9H-carbazol-3-yl)-1-(2-methoxyethyl)-4-methyl-1H-benzimidazol-5-carboxylat

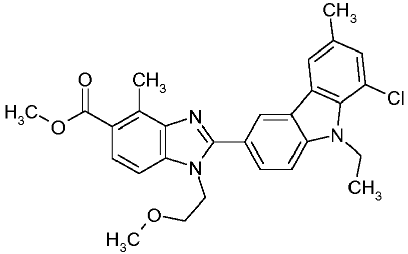

In Analogie zu Beispiel 1 wurden aus 88 mg (0.37 mmol) Methyl-3-amino-4-[(2-methoxyethyl)amino]-2-methylbenzoat (Intermediat 8) und 100 mg (0.37 mmol) 8-Chlor-9-ethyl-6-methyl-9H-carbazol-3-carbaldehyd (Intermediat 6) Methyl-2-(8-chlor-9-ethyl-6-methyl-9H-carbazol-3-yl)-1-(2-methoxyethyl)-4-methyl-1H-benzimidazol-5-carboxylat hergestellt (100 mg, 55%).
¹H-NMR (300 MHz, DMSO-d6), δ [ppm]= 1.40 (t, 3H), 2.47 (s, 3H), 2.88 (s, 3H), 3.11 (s, 3H), 3.68 (t, 2H), 3.87 (s, 3H), 4.56 (t, 2H), 4.82 (q, 2H), 7.39 (s, 1H), 7.60 (d, 1H), 7.81 - 7.86 (m, 2H), 7.97 (dd, 1H), 8.09 (s, 1H), 8.63 (d, 1H).

### Beispiel 12

### 2-(8-Chlor-9-ethyl-6-methyl-9H-carbazol-3-yl)-1-(2-methoxyethyl)-4-methyl-1H-benzimidazol-5-carbonsäure

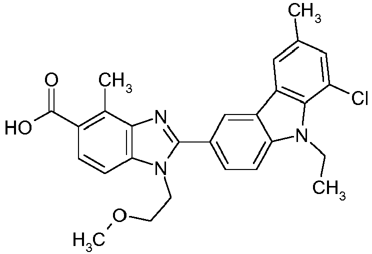

In Analogie zu Beispiel 2 wurde aus Methyl-2-(8-chlor-9-ethyl-6-methyl-9H-carbazol-3-yl)-1-(2-methoxyethyl)-4-methyl-1H-benzimidazol-5-carboxylat die Titelverbindung hergestellt (60 mg, 61 %).
¹H-NMR (300 MHz, DMSO-d6), δ [ppm]= 1.40 (t, 3H), 2.47 (s, 3H), 2.89 (s, 3H), 3.11 (s, 3H), 3.68 (t, 2H), 4.55 (t, 2H), 4.82 (q, 2H), 7.39 (s, 1H), 7.57 (d, 1H), 7.80 - 7.88 (m, 2H), 7.97 (dd, 1H), 8.09 (s, 1H), 8.63 (d, 1H), 12.58 (br. s., 1H).

### Beispiel 13

### 2-(5-Chlor-9-ethyl-6-methyl-9H-carbazol-3-yl)-1-(2-methoxyethyl)-4-methyl-1H-benzimidazol-5-carbonsäure

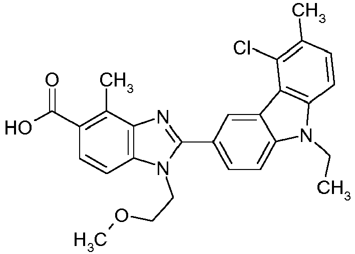

In Analogie zu Beispiel 1 wurden aus 88 mg (0.37 mmol) Methyl-3-amino-4-[(2-methoxyethyl)amino]-2-methylbenzoat (Intermediat 8) und 100 mg (0.37 mmol) 5-Chlor-9-ethyl-6-methyl-9H-carbazol-3-carbaldehyd (Intermediat 7) zunächst Methyl-2-(5-chlor-9-ethyl-6-methyl-9H-carbazol-3-yl)-1-(2-methoxyethyl)-4-methyl-1H-benzimidazol-5-carboxylat hergestellt (111 mg, 61%), welches dann analog Beispiel 2 weiter zur Titelverbindung umgesetzt wurde (95 mg, 94%).
¹H-NMR (300 MHz, DMSO-d6), δ [ppm]= 1.35 (t, 3H), 2.54 (s, 3H), 2.89 (s, 3H), 3.15 (s, 3H), 3.75 (t, 2H), 4.46 - 4.60 (m, 4H), 7.48 - 7.57 (m, 2H), 7.61 - 7.67 (m, 1H), 7.81 - 7.87 (m, 2H), 7.99 (dd, 1H), 8.99 (d, 1H), COOH nicht angegeben.

### Biologische Beispiele:

### 1. Syngenes Maus-Endometriosemodell:

Die syngene Induktion der Endometriose in Mäusen stellt ein gängiges Tiermodell dar, um die Wirksamkeit von Substanzen für die Therapie der Endometriose zu testen. Die Endometriose wird experimentell durch Transplantation von murinen Uterusfragmenten einer Spendermaus desselben Stammes in die Bauchhöhle der Empfänger-Maus induziert. Es wurden weibliche Tiere des balb/c Stammes verwendet. Mittels Vaginalabstrich wird der Zyklus der Maus bestimmt. Es werden ausschließlich Spendertiere verwendet, die sich im Östrus befinden. Die Spendertiere werden getötet und die Uterushörner werden entnommen und anschließend longitudinal aufgeschnitten. Aus den Uteri werden mit einer Stanze 2 mm Bioptate ausgestanzt, die anschließend ins Empfängertier eingenäht werden. Die Empfängertiere werden narkotisiert und einer Laparotomie unterzogen. Während des Eingriffs werden 6 Uterusstanzen einer Donormaus an das parietale Peritoneum der Empfängermaus angenäht. Am Tag nach diesem Eingriff beginnt die 4-wöchige Behandlung mit den zu testenden Substanzen. Nach 28 Tagen werden die Tiere in einer finalen Laparotomie eröffnet und die Läsionsgrößen werden bestimmt. Die angewachsenen Läsionen werden fotografisch festgehalten und die Fläche wird mittels AxioVision Software vermessen. Pro Behandlungsgruppe werden 14 Tiere eingesetzt.

### 2. Durchflusszytometrie:

Zur Gewinnung der Zellen aus dem Peritoneum werden 3 ml kaltes PBS (phosphate buffered saline) in das Peritoneum des toten Tieres injiziert und nach leichter Massage des Bauches wieder entnommen. Die Zellen dieser Peritoneallavage werden bei 1400 UpM 2 min herunterzentrifugiert und in 500 µl PBS mit 2% FCS (fetal calf serum) wieder aufgenommen. Für jede Färbung mit verschiedenen Antikörper-Flourochromkonjugat-Mixen werden 100 µl dieser Zellsuspension verwendet. Die Zellen werden dafür in 96 Lochplatten wieder herunterzentrifugiert und in 50 µl einer Antikörperlösung in der 1:300 verdünnter anti CD11b- Pacific Blue (eBioscience), 1:200 verdünnter Anti F4/80-PE (eBioscience), 1:200 verdünnter Anti Gr1- APC-Cy7 (BD Pharmigen), 1:400 verdünnter Anti CD11c-PE-Cy7 (BD Pharmigen) und 1:400 verdünnter Anti MHCII-FITC (BD Pharmigen) resuspendiert und für 20 min im Dunkeln auf Eis gelagert. Anschließend werden 150 µl PBS mit 2%FCS zugegeben und die Zellen erneut 2 min bei 1400 UpM herunter zentrifugiert. Die 200 µl Überstand werden verworfen und die Zellen mit 200 µl PBS mit 2% FCS gewaschen und erneut abzentrifugiert. Anschließend werden sie in 200 µl PBS mit 2%FCS und 1:5000 verdünntem 5mg/ml DAPI (Sigma) aufgenommen und die Fluoreszenz-Intensität der einzelne Fluorochrome pro Zelle mit einem FACS Canto II Durchflusszytometer gemessen.

### 3. Nachweis des Antagonismus des humanen Prostaglandin E2 (Subtyp EP₄) Rezeptorsignals

### 3. 1 Nachweisprinzip

### Nachweis der Antagonisierung des hEP4-Signals

Die Bindung des Agonisten Prostaglandin E2 (PGE2) an den EP4 Subtyp des humanen hPGE2-R (hEP4-R) induziert die Aktivierung membranständiger Adenylatzyklasen und damit die Bildung von cAMP. In Gegenwart des Phosphodiesteraseinhibitors IBMX akkumuliert dieses cAMP intrazellulär und wird nach Zell-Lyse in einem kompetitiven Nachweisverfahren eingesetzt. Bei diesem Verfahren konkurriert es mit einem fluoreszenzmarkierten cAMP (cAMP-d2) um die Bindung an einen Eu-Kryptat markierten Anti-cAMP Antikörpers. In Abwesenheit zellulären cAMPs bildet sich ein Komplex zwischen dem Eu-Kryptat markierten Anti-cAMP Antikörper und dem cAMP-d2 Molekül aus, welcher nach Anregung bei 337 nm einen auf FRET (fluorescence resonance energy transfer) basierenden Energietransfer zum cAMP-d2 Tracer ermöglicht und zu einem langanhaltenden Fluoreszenzsignal führt (Emission bei 665 und 620 nm). Dieses Signal wird zeitlich aufgelöst, d.h. nach Abklingen der Hintergrundsfluoreszenz in einem geeigneten Messgerät gemessen (time resolved; TR-FRET). Die Well-Ratio Ermittlung (Emision665nm/Emision620nm *10000) ermöglicht es zudem Einzelmessungsunterschiede in den Zugabemengen der Detektionsreagenzien zu normieren.

Durch Prostglandin-E2-Gabe und Anstieg des intrazellulären cAMPs kommt es zu einer Erniedrigung des FRET- Signals, welches im Falle einer antagonistischen Substanzwirkung wieder ansteigt.

### 3.2. Nachweisverfahren

### 3.2.1 Test auf Antagonismus (Angaben pro Vertiefung einer 384-Loch Platte):

Zu den in einer Testplatte vorgelegten Substanzlösungen (50 nl; 100% DMSO) werden 4 µl einer den hEP4 exprimierenden Zellsuspension (2500 Zellen/Well) zugegeben, die außerdem bereits den cAMP-D2 Tracer enthält. Nach einer 20 minütigen Vorinkubation bei Raumtemperatur werden 2 µl einer 3xPGE2 Lösung zugegeben und in Gegenwart einer EC80-Konzentration des Agonisten (0,4 nM) für weitere 60 min bei Raumtemperatur inkubiert (Volumen: ∼6 µl), bevor die Gesamtreaktion anschließend durch Zugabe von 2 µl Lysisbuffer gestoppt wird (Volumen: ∼8 µl). Weitere 20 min bei Raumtemperatur später wird das Zell-Lysat entsprechend den Herstellangaben in einem TR-FRET geeigneten Messgerät gemessen (vergleiche cAMP HTRF-Assay Kit: Cisbio International 62AM6PEJ high range)

### 3.2.2 Test auf Agonismus (Angaben pro Vertiefung einer 384-Loch Platte):

Zu den in einer Testplatte vorgelegten Substanzlösungen (50 nl; 100% DMSO) werden 4 µl einer den hEP4 exprimierenden Zellsuspension (2500 Zellen/Well) zugegeben, die außerdem bereits den cAMP-D2 Tracer enthält. Nach einer 20 minütigen Vorinkubation bei Raumtemperatur werden 2 µl Zellmedium zugegeben und für weitere 60 min bei Raumtemperatur inkubiert (Volumen: ∼6 µl), bevor die Gesamtreaktion anschließend durch Zugabe von 2 µl Lysisbuffer gestoppt wird (Volumen: ∼8 µl). Weitere 20 min bei Raumtemperatur später wird das Zell-Lysat entsprechend den Herstellangaben in einem TR-FRET geeigneten Messgerät gemessen (vgl. cAMP HTRF-Assay Kit: Cisbio International 62AM6PEJ high range)

### 4. Nachweis des Antagonismus des humanen Prostaglandin E2 (Subtyp EP2) Rezeptorsignals

### 4. 1 Nachweisprinzip

Die Bindung von PGE2 an den EP2-Subtyp des humanen PGE2-Rezeptors induziert die Aktivierung membranständiger Adenylatzyklasen und führt zur Bildung von cAMP. In Gegenwart des Phosphodiesteraseinhibitors IBMX wird das aufgrund dieser Stimulation akkumulierte und mittels Zell-Lyse freigesetzte cAMP in einem kompetitiven Nachweisverfahren eingesetzt. In diesem Test konkurriert das im Lysat vorhandene cAMP mit einem fluoreszenzmarkierten cAMP (cAMP-d2) um die Bindung an einen Eu-Kryptat markierten Anti-cAMP Antikörper.

In Abwesenheit von zellulärem cAMP entsteht ein maximales Signal, welches auf die Bindung dieses cAMP-d2 Moleküls an den Antikörpers zurückzuführen ist. Nach Anregung des cAMP-d2 Moleküls bei 337 nm kommt es zu einem Fluoreszenz Resonanz Energie Transfer (FRET) zu den Eu-Kryptat Molekülen des (damit markierten) Anti-cAMP Antikörpers, gefolgt von einem langanhaltenden Emissionssignal bei 665 nm (sowie bei 620 nM). Beide Signale werden in einem geeigneten Messgerät zeitlich versetzt, d.h. nach Abklingen der Hintergrundsfluoreszenz gemessen. Jegliche Erhöhung des durch Prostglandin-E₂-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 4.2. Nachweisverfahren

### 4.2.1. Test auf Antagonismus (Angaben pro Vertiefung einer 384-Loch-Platte):

Zu einer Testplatte mit den bereits vorgelegten Substanzlösungen (0.05 µl; 100 % DMSO, Konzentrationsbereich von 0.8 nM - 16.5 µM) wurden 4 µl einer cAMP-d2/Zellsuspension zugegeben (625000 Zellen/ml). Nach einer 20-minütigen Vorinkubation bei Raumtemperatur wurden 2 µl einer 3xPGE2 Lösung (1.5 nM, in PBS-IBMX) zugegeben und in Gegenwart des Agonisten für weitere 60 min bei Raumtemperatur inkubiert (Volumen: ∼ 6 µl). Anschließend wurde die Reaktion durch Zugabe von 2 µl Lysispuffer gestoppt und vor der eigentlichen Messung für weitere 20 min bei Raumtemperatur inkubiert (Volumen: ∼ 8 µl).

### 5. Nachweis des Antagonismus des humanen Prostaglandin D Rezeptorsignals

### 5. 1 Nachweisprinzip

Die Bindung von Prostaglandin D2 an den humanen PGD-Rezeptor induziert die Aktivierung membranständiger Adenylatzyklasen und führt zur Bildung von cAMP. In Gegenwart des Phosphodiesteraseinhibitors IBMX wird das aufgrund dieser Stimulation akkumulierte und mittels Zell-Lyse freigesetzte cAMP in einem kompetitiven Nachweisverfahren eingesetzt. In diesem Test konkurriert das im Lysat vorhandene cAMP mit einem fluoreszenzmarkierten cAMP (cAMP-d2) um die Bindung an einen Eu-Kryptat markierten Anti-cAMP Antikörper.

In Abwesenheit von zellulärem cAMP entsteht ein maximales Signal, welches auf die Bindung dieses cAMP-d2 Moleküls an den Antikörpers zurückzuführen ist. Nach Anregung des cAMP-d2 Moleküls bei 337 nm kommt es zu einem Fluoreszenz-Resonanz-Energie-Transfer (FRET) zu den Eu-Kryptat-Molekülen des (damit markierten) Anti-cAMP Antikörpers, gefolgt von einem langanhaltenden Emissionssignal bei 665 nm (sowie bei 620 nM). Beide Signale werden in einem geeigneten Messgerät zeitlich versetzt, d.h. nach Abklingen der Hintergrundsfluoreszenz gemessen. Jegliche Erhöhung des durch Prostglandin-E2-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 5.2. Nachweisverfahren

### 5.2.1. Test auf Antagonismus (Angaben pro Vertiefung einer 384-Loch-Platte):

Zu einer Testplatte mit den bereits vorgelegten Substanzlösungen (0.05 µl; 100 % DMSO, Konzentrationsbereich von 0.8 nM - 16.5 µM) wurden 4 µl einer cAMP-d2/Zellsuspension zugegeben (625000 Zellen/ml). Nach einer 20-minütigen Vorinkubation bei Raumtemperatur (RT) wurden 2 µl einer 3xPGD2 Lösung (6 nM, in PBS-IBMX) zugegeben und in Gegenwart des Agonisten für weitere 30 min bei RT inkubiert (Volumen: ∼ 6 µl). Anschließend wurde die Reaktion durch Zugabe von 2 µl Lysispuffer gestoppt und vor der eigentlichen Messung für weitere 20 min bei RT inkubiert (Volumen: ∼ 8 µl).

**Tabelle 1: Antagonisierung der Aktivität des humanen EP4 Rezeptors durch die erfindungsgemäßen Verbindungen**

| Beispiel | Antagonisierung hEP4 IC₅₀ [M] |
|---|---|
| 1 | 3.2E-8 |
| 2 | 1.1E-8 |
| 3 | 4.0E-8 |
| 4 | 2.4E-9 |
| 6 | 2.0E-9 |
| 7 | 2.4E-9 |
| 8 | 1.9E-9 |
| 9 | 6.2E-8 |
| 10 | 1.5E-9 |
| 11 | 4.7E-8 |
| 12 | 2.8E-9 |
| 13 | 2.3E-8 |

### Referenzen

Giudice L C; Endometriosis; N Engl J Med 2010;362:2389-98.
Chishima F, Hayakawa S, Sugita K, Kinukawa N, Aleemuzzaman S, Nemoto N, Yamamoto T, Honda M: Increased expression of cyclooxygenase-2 in local lesions of endometriosis patients. Am J Reprod. Immunol 2002; 48:50-56.
Sales K J and Jabbour H N; Cyclooxygenase enzymes and prostaglandins in pathology of the endometrium. Reproduction (2003) 126, 559-567.
Stratton P and Berkley K J; Chronic pelvic pain and endometriosis: translational evidence of the relationship and implications; Human Reproduction Update, Vol.0, No.0 pp. 1-21, 2010.
Petraglia F, Hornung D, Seitz C, Faustmann T, Gerlinger C, Luisi S, Lazzeri L, Strowitzki T; Reduced pelvic pain in women with endometriosis: efficacy of long-term dienogest treatment; Arch Gynecol Obstet, 2012 Jan;285(1):167-73.

## Patentansprüche

1. Verbindungen der allgemeinen Formel (I) 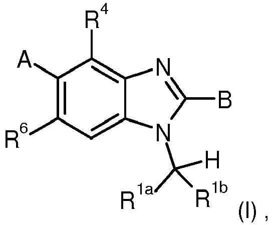 in der
A für Wasserstoff, Brom, Cyano, Formyl, C₁-C₃-Alkyl, 4-6-gliedriges Heterocyclyl, 5-6-gliedriges Heteroaryl, R¹¹O-C(=O)-(CH₂)ₚ-, R⁵R^{5'}N-C(=O)-(CH₂)ₚ-, R¹¹O-S(=O)₂-(CH₂)ₚ-, R⁵R^{5'}N-S(=O)₂-(CH₂)ₚ-, (C₁-C₆-Alkyl)-S(=O)₂-, (C₁-C₆-Alkyl)-S(=O)(=NH)- oder (C₃-C₆-Cycloalkyl)-S(=O)(=NH)- steht,
wobei Heteroaryl bevorzugt aus der Gruppe bestehend aus Pyrazolyl, Imidazolyl, Triazolyl, Tetrazolyl, Oxazolyl, Isoxazolyl und Oxadiazolyl ausgewählt wird,
und
wobei Alkylreste gegebenenfalls einfach oder mehrfach, gleich oder verschieden substituiert sein können mit Halogen oder Hydroxy,
und
wobei Heterocyclylreste gegebenenfalls einfach oder mehrfach, gleich oder verschieden substituiert sein können mit C₁-C₃-Alkyl, Trifluormethyl oder Hydroxy,
und
wobei Heterarylreste gegebenenfalls einfach oder mehrfach, gleich oder verschieden substituiert sein können mit C₁-C₃-Alkyl, Trifluormethyl oder Hydroxy,
B aus den folgenden Strukturen ausgewählt wird, 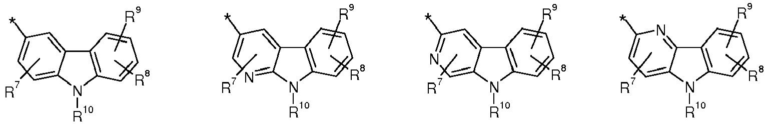 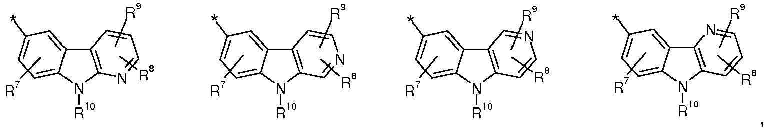 worin * die Verknüpfungsstelle im Molekül bedeutet,
R^{1a}, R^{1b} unabhängig voneinander für Wasserstoff, Cyano, C₁-C₅-Alkyl, C₂-C₅-Alkenyl, C₂-C₅-Alkinyl, (C₃-C₆-Cycloalkyl)-(CH₂)ₘ-, (4-6-gliedriges Heterocyclyl)-(CH₂)ₙ-, (C₁-C₅-Alkoxy)-(C₁-C₃-alkyl)-, (C₃-C₆-Cycloalkoxy)-(C₁-C₃-alkyl)-, H₂N-(C₁-C₃-Alkyl)-, (C₁-C₅-Alkyl)NH-(C₁-C₃-alkyl)- oder (C₁-C₅-Alkyl)₂N-(C₁-C₃-alkyl)- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
und
wobei Alkylreste, Cycloalkylreste und Heterocyclylreste gegebenenfalls einfach oder mehrfach, gleich oder verschieden substituiert sein können mit Halogen, C₁-C₅-Alkyl, Hydroxy, C(=O)OH, HO-C(=O)-(C₁-C₅-Alkyl)-, (C₁-C₅-Alkyl)O-C(=O)-(C₁-C₅-alkyl)- oder (C₁-C₅-Alkyl)-S(=O)₂-,
R⁴ für Wasserstoff, Fluor, Chlor, C₁-C₂-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen oder Hydroxy,
R⁵, R^{5'} unabhängig voneinander für Wasserstoff, C₁-C₇-Alkyl, (C₁-C₇-Alkoxy)-(C₂-C₅-alkyl)-, (4-6-gliedriges Heterocyclyl)-(CH₂)ᵣ-, (C₁-C₇-Alkyl)-C(=O)-, (C₃-C₇-Cycloalkyl)-C(=O)-, Phenyl-(CH₂)ᵣ-C(=O)-, Pyridyl-(CH₂)ᵣ-C(=O)-, (C₁-C₇-Alkyl)-S(=O)₂-, (C₃-C₇-Cycloalkyl)-S(=O)₂-, Phenyl-(CH₂)ᵣ-S(=O)₂- oder Pyridyl-(CH₂)ᵣ-S(=O)₂- stehen,
wobei Heterocyclyl bevorzugt aus der Gruppe bestehend aus Oxetanyl, Tetrahydrofuranyl, Tetrahydropyranyl, 1,4-Dioxanyl, Morpholinyl, Azetidinyl, Pyrrolidinyl, Piperazinyl und Piperidinyl ausgewählt wird,
und
wobei R⁵ und R^{5'} unabhängig voneinander gegebenenfalls einfach oder mehrfach, gleich oder verschieden substituiert sein können mit Halogen, Hydroxy, C₁-C₂-Alkyl, Trifluoromethyl, (C₁-C₅-Alkyl)NH-, (C₁-C₅-Alkyl)₂N-, C₁-C₂-Alkoxy oder Trifluormethoxy,
oder
R⁵, R^{5'} zusammen mit dem Stickstoffatom, an das sie gebunden sind, einen 4-6-gliedrigen heterocyclischen Ring bilden, der optional, einfach oder mehrfach, gleich oder verschieden mit Oxo, Hydroxy, Carboxy, C₁-C₂-Alkyl oder C₁-C₂-Alkoxy substituiert sein kann,
wobei ein 6-gliedriger heterocyclischer Ring optional als weiteres Ringatom ein Heteroatom, das aus der Gruppe bestehend aus O und N ausgewählt wird, enthalten kann,
R⁶ für Wasserstoff, Fluor, Chlor, Methyl, Trifluormethyl, Methoxy oder Trifluormethoxy steht,
R⁷ für Wasserstoff, Fluor, Chlor, Cyano, SF₅, C₁-C₃-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen,
R⁸ für Fluor, Chlor, Brom, Cyano, SF₅, C₁-C₃-Alkyl, C₃-C₅-Cycloalkyl, C₁-C₂-Alkoxy oder (C₃-C₄-Cycloalkyl)-CH₂- steht,
wobei Alkyl- und Cycloalkylreste gegebenenfalls einfach oder mehrfach, gleich oder verschieden substituiert sein können mit Halogen,
R⁹ für Fluor, Chlor, Brom, Cyano, C₁-C₃-Alkyl oder C₁-C₂-Alkoxy steht,
wobei Alkylreste gegebenenfalls einfach oder mehrfach, gleich oder verschieden substituiert sein können mit Halogen,
oder
R⁹ für Brom steht und gleichzeitig R⁸ für Wasserstoff steht,
R¹⁰ für C₁-C₅-Alkyl, C₃-C₅-Alkenyl, C₃-C₅-Alkinyl, (C₃-C₆-Cycloalkyl)-(CH₂)ₙ-, (4-6-gliedriges Heterocyclyl)-(CH₂)ₙ-, oder (C₁-C₇-Alkoxy)-(C₂-C₅-alkyl)- steht,
wobei Heterocyclyl bevorzugt aus der Gruppe bestehend aus Oxetanyl, Tetrahydrofuranyl, Tetrahydropyranyl, Morpholinyl, Pyrrolidinyl und Piperidinyl ausgewählt wird,
und
wobei Alkylreste, Cycloalkylreste und Heterocyclylreste gegebenenfalls einfach oder mehrfach, gleich oder verschieden substituiert sein können mit Halogen, C₁-C₂-Alkyl, C₁-C₂-Alkoxy oder C(=O)OH,
R¹¹ für Wasserstoff, C₁-C₇-Alkyl, C₃-C₇-Cycloalkyl, Phenyl-(CH₂)_{q}- oder (C₁-C₇-Alkoxy)-(C₂-C₅-alkyl)- steht,
wobei Phenyl gegebenenfalls einfach oder mehrfach, gleich oder verschieden substituiert sein kann mit C₁-C₃-Alkyl, Trifluormethyl oder Hydroxy,
m 0, 1, 2 oder 3 ist,
n 0, 1, 2 oder 3 ist,
p 0, 1 oder 2 ist,
q 1, 2 oder 3 ist, und
r 0, 1, 2 oder 3 ist,
sowie deren Diastereomere, Enantiomere, Solvate und Salze oder Cyclodextrin-Clathrate.

2. Verbindungen gemäß Anspruch 1, wobei
A für Cyano, C₁-C₃-Alkyl, 5-gliedriges Heterocyclyl, 5-gliedriges Heteroaryl, R¹¹O-C(=O)-(CH₂)ₚ-, R⁵R^{5'}N-C(=O)-(CH₂)ₚ- oder R⁵R^{5'}N-S(=O)₂-(CH₂)ₚ- steht,
wobei Heteroaryl bevorzugt aus der Gruppe bestehend aus Triazolyl, Tetrazolyl und Oxadiazolyl ausgewählt wird,
und
wobei Alkylreste gegebenenfalls einfach oder mehrfach, gleich oder verschieden substituiert sein können mit Hydroxy,
und
wobei Heterarylreste gegebenenfalls einfach oder mehrfach, gleich oder verschieden substituiert sein können mit C₁-C₃-Alkyl, Trifluormethyl oder Hydroxy,
B für eine Gruppe 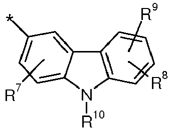 steht,
worin * die Verknüpfungsstelle im Molekül bedeutet,
R^{1a} für Wasserstoff oder C₁-C₅-Alkyl steht,
R^{1b} für Wasserstoff, C₁-C₅-Alkyl, C₂-C₅-Alkenyl, (C₃-C₆-Cycloalkyl)-(CH₂)ₘ-, (4-6-gliedriges Heterocyclyl)-(CH₂)ₙ-, (C₁-C₅-Alkoxy)-(C₁-C₃-Alkyl)- oder (C₁-C₅-Alkyl)₂N-(C₁-C₃-alkyl)- steht,
wobei Heterocyclyl bevorzugt aus der Gruppe bestehend aus Oxetanyl, Tetrahydrofuranyl, 1,4-Dioxanyl, Morpholinyl und Pyrrolidinyl ausgewählt wird,
und
wobei Alkylreste und Cycloalkylreste gegebenenfalls einfach oder mehrfach, gleich oder verschieden substituiert sein können mit C₁-C₅-Alkyl, Hydroxy oder (C₁-C₅-Alkyl)-S(O)₂-,
R⁴ für Wasserstoff, Fluor, Chlor, C₁-C₂-Alkyl oder C₁-C₂-Alkoxy steht,
R⁵, R^{5'} unabhängig voneinander für Wasserstoff, C₁-C₇-Alkyl, (4-6-gliedriges Heterocyclyl)-(CH₂)_{r'}, (C₁-C₇-Alkyl)-S(O)₂-, (C₃-C₇-Cycloalkyl)-S(O)₂-, Phenyl-(CH₂)ᵣ-S(O)₂- oder Pyridyl-(CH₂)ᵣ-S(O)₂- stehen,
wobei Heterocyclyl bevorzugt aus der Gruppe bestehend aus Morpholinyl und Pyrrolidinyl ausgewählt wird,
und
wobei R⁵ und R^{5'} unabhängig voneinander gegebenenfalls einfach oder mehrfach, gleich oder verschieden substituiert sein können mit Halogen, C₁-C₂-Alkyl, Trifluoromethyl, (C₁-C₅-Alkyl)₂N-, C₁-C₂-Alkoxy oder Trifluormethoxy,
oder
R⁵, R^{5'} zusammen mit dem Stickstoffatom, an das sie gebunden sind, einen 4-6-gliedrigen heterocyclischen Ring bilden, der optional einfach mit Oxo oder Hydroxy substituiert sein kann,
wobei ein 6-gliedriger heterocyclischer Ring optional als weiteres Ringatom ein Sauerstoffatom enthalten kann,
R⁶ für Wasserstoff, Fluor, Methyl oder Methoxy steht,
R⁷ für Wasserstoff, Fluor, Chlor, C₁-C₃-Alkyl oder C₁-C₂-Alkoxy steht,
R⁸ für Fluor, Chlor, Brom, C₁-C₃-Alkyl oder C₁-C₂-Alkoxy steht,
R⁹ für Fluor, Chlor, Brom, C₁-C₃-Alkyl oder C₁-C₂-Alkoxy steht,
oder
R⁹ für Brom steht und gleichzeitig R⁸ für Wasserstoff steht,
R¹⁰ für C₁-C₅-Alkyl, C₃-C₅-Alkenyl, C₃-C₅-Alkinyl, (C₃-C₆-Cycloalkyl)-(CH₂)ₙ- oder (C₁-C₇-Alkoy)-(C₂-C₅-Alkyl)- steht,
R¹¹ für Wasserstoff oder C₁-C₇-Alkyl steht,
m 0 oder 1 ist,
n 0 oder 1 ist,
p 0 ist, und
r 0, 1 oder 2 ist,
sowie deren Diastereomere, Enantiomere, Solvate und Salze oder Cyclodextrin- Clathrate.

3. Verbindungen gemäß Anspruch 1, wobei
A für 5-gliedriges Heteroaryl, R¹¹O-C(=O)-(CH₂)ₚ-, R⁵R^{5'}N-C(=O)-(CH₂)ₚ- oder R⁵R^{5'}N-S(=O)₂-(CH₂)ₚ- steht,
wobei Heteroaryl bevorzugt aus der Gruppe bestehend aus Triazolyl, Tetrazolyl und Oxadiazolyl ausgewählt wird,
und
wobei Heterarylreste gegebenenfalls einfach oder mehrfach, gleich oder verschieden substituiert sein können mit C₁-C₃-Alkyl, Trifluormethyl oder Hydroxy,
B für eine Gruppe 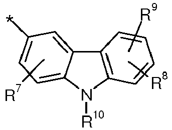 steht,
worin * die Verknüpfungsstelle im Molekül bedeutet,
R^{1a} für Wasserstoff oder Methyl steht,
R^{1b} für Wasserstoff, C₁-C₂-Alkyl, Vinyl, Cyclopropyl-(CH₂)ₘ-, Methoxy-(C₁-C₂-Alkyl)- oder (N,N-Dimethylamino)methyl steht,
wobei Alkylreste und Cycloalkylreste gegebenenfalls einfach oder mehrfach, gleich oder verschieden substituiert sein können mit Methyl, Hydroxy, oder Methylsulfonyl,
R⁴ für Wasserstoff, Fluor, Chlor, Methyl oder Methoxy steht,
R⁵, R^{5'} unabhängig voneinander für Wasserstoff, C₁-C₂-Alkyl oder (5-6-gliedriges Heterocyclyl)-(CH₂)ᵣ- stehen,
wobei Heterocyclyl bevorzugt aus der Gruppe bestehend aus Morpholinyl und Pyrrolidinyl ausgewählt wird,
und
wobei R⁵ und R^{5'} unabhängig voneinander gegebenenfalls einfach oder mehrfach, gleich oder verschieden substituiert sein können mit Chlor, Fluor, Methyl, Trifluoromethyl, N,N-Dimethylamino, Methoxy oder Trifluoromethoxy,
oder
R⁵, R^{5'} zusammen mit dem Stickstoffatom, an das sie gebunden sind, einen 4-6-gliedrigen heterocyclischen Ring bilden, der optional einfach mit Oxo oder Hydroxy substituiert sein kann,
wobei ein 6-gliedriger heterocyclischer Ring optional als weiteres Ringatom ein Sauerstoffatom enthalten kann,
R⁶ für Wasserstoff, Fluor, Methyl oder Methoxy steht,
R⁷ für Wasserstoff, Fluor, Chlor, Methyl oder Methoxy steht,
R⁸ für Fluor, Chlor, Brom, Methyl oder Methoxy steht,
R⁹ für Fluor, Chlor, Brom, Methyl oder Methoxy steht,
oder
R⁹ für Brom steht und gleichzeitig R⁸ für Wasserstoff steht,
R¹⁰ für C₁-C₃-Alkyl, Allyl, Propargyl, (C₃-C₄-Cycloalkyl)-(CH₂)ₙ- oder Methoxyethyl steht,
R¹¹ für Wasserstoff oder C₁-C₃-Alkyl steht,
m 0 oder 1 ist,
n 0 oder 1 ist,
p 0 ist, und
r 0, 1 oder 2 ist,
sowie deren Diastereomere, Enantiomere, Solvate und Salze oder Cyclodextrin-Clathrate.

4. Verbindungen gemäß Anspruch 1, wobei
A für R¹¹O-C(=O)-(CH₂)ₚ- steht,
B für eine Gruppe 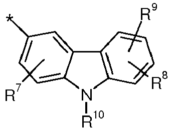 steht,
worin * die Verknüpfungsstelle im Molekül bedeutet,
R^{1a} für Wasserstoff steht,
R^{1b} für Methoxymethyl steht,
R⁴ für Wasserstoff, Fluor oder Methyl steht,
R⁶ für Wasserstoff steht,
R⁷ für Wasserstoff steht,
R⁸ für Fluor, Chlor oder Methyl steht,
R⁹ für Fluor, Chlor, Brom oder Methyl steht,
oder
R⁹ für Brom steht und gleichzeitig R⁸ für Wasserstoff steht,
R¹⁰ für Ethyl steht,
R¹¹ für Wasserstoff, Methyl oder Ethyl steht, und
p 0 ist,
sowie deren Diastereomere, Enantiomere, Solvate und Salze oder Cyclodextrin-Clathrate.

5. Verbindung gemäß Anspruch 1 ausgewählt aus einer Gruppe, die die folgenden Verbindungen enthält:
1. Methyl-2-(6-brom-9-ethyl-9H-carbazol-3-yl)-1-(2-methoxyethyl)-4-methyl-1H-benzimidazol-5-carboxylat ;
2. 2-(6-Brom-9-ethyl-9H-carbazol-3-yl)-1-(2-methoxyethyl)-4-methyl-1H-benzimidazol-5-carbonsäure ;
3. Methyl-2-(9-ethyl-8-fluor-6-methyl-9H-carbazol-3-yl)-1-(2-methoxyethyl)-4-methyl-1H-benzim idazol-5-carboxylat ;
4. 2-(9-Ethyl-8-fluor-6-methyl-9H-carbazol-3-yl)-1-(2-methoxyethyl)-4-methyl-1H-benzimidazol-5-carbonsäure ;
5. Methyl-2-(9-ethyl-6-fluor-8-methyl-9H-carbazol-3-yl)-1-(2-methoxyethyl)-4-methyl-1H-benzim idazol-5-carboxylat ;
6. 2-(9-Ethyl-6-fluor-8-methyl-9H-carbazol-3-yl)-1-(2-methoxyethyl)-4-methyl-1H-benzimidazol-5-carbonsäure ;
7. 2-(9-Ethyl-6-fluor-8-methyl-9H-carbazol-3-yl)-4-fluor-1-(2-methoxyethyl)-1H-benzimidazol-5-carbonsäure ;
8. 2-(9-Ethyl-8-fluor-6-methyl-9H-carbazol-3-yl)-4-fluor-1-(2-methoxyethyl)-1H-benzimidazol-5-carbonsäure ;
9. Ethyl-2-(9-ethyl-8-fluor-6-methyl-9H-carbazol-3-yl)-1-(2-methoxyethyl)-1H-benzimidazol-5-carboxylat ;
10. 2-(9-Ethyl-8-fluor-6-methyl-9H-carbazol-3-yl)-1-(2-methoxyethyl)-1H-benzimidazol-5-carbonsäure ;
11. Methyl-2-(8-chlor-9-ethyl-6-methyl-9H-carbazol-3-yl)-1-(2-methoxyethyl)-4-methyl-1H-benzimidazol-5-carboxylat ;
12. 2-(8-Chlor-9-ethyl-6-methyl-9H-carbazol-3-yl)-1-(2-methoxyethyl)-4-methyl-1H-benzimidazol-5-carbonsäure; und
13. 2-(5-Chlor-9-ethyl-6-methyl-9H-carbazol-3-yl)-1-(2-methoxyethyl)-4-methyl-1H-benzimidazol-5-carbonsäure .

6. Verbindung der Formel (I), wie in einem der Ansprüche 1 bis 5 definiert, zur Behandlung und/oder Prophylaxe von Krankheiten.

7. Verbindung der Formel (I), wie in einem der Ansprüche 1 bis 5 definiert, zur Verwendung in einem Verfahren zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

8. Verwendung einer Verbindung nach Anspruch 1, 2, 3, 4 oder 5 zur Herstellung eines Arzneimittels zur Behandlung und/oder Prophylaxe von Krankheiten.

9. Verwendung einer Verbindung, wie in einem der Ansprüche 1 bis 5 definiert, zur Herstellung eines Arzneimittels zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

10. Arzneimittel enthaltend eine Verbindung, wie in einem der Ansprüche 1 bis 5 definiert, in Kombination mit einem oder mehreren weiteren Wirkstoffen insbesondere mit selektiven Estrogen Rezeptor Modulatoren (SERMs), Estrogenrezeptor (ER) Antagonisten, Aromataseinhibitoren, 17-β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen (Mkk3/6, Mek1/2, Erk1/2), Inhibitoren der Proteinkinase B (PKBα/β/γ; Akt1/2/3), Inhibitoren der Phosphoinositid-3-Kinase (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, Neurokinin1 Rezeptor Antagonisten, Paracetamol, selektiven COX2-Inhibitoren und/oder nicht-selektiven COX1/COX2 Inhibitoren.

11. Arzneimittel enthaltend eine Verbindung der Formel (I) wie in einem der Ansprüche 1 bis 5 definiert, in Kombination mit einem inerten, nichttoxischen, pharmazeutisch geeigneten Hilfsstoff.

12. Arzneimittel nach Anspruch 10 oder 11 zur Behandlung und/oder Prophylaxe von Endometriose, von uterinen Leiomyomen, von uterinen Blutungsbeschwerden, wobei es sich bei den Blutungsbeschwerden um starke und langanhaltende Blutungen, zeitlich irreguläre Blutungen sowie Schmerzen handeln kann, der Dysmenorrhö, des Krebs, wobei es sich bei den Krebserkrankungen um Lungen-, Darm-, Brust-, Haut-, Prostata-, Ösophagus-Krebs und Leukämie handeln kann, von Arteriosklerose und von polyzystischen Nierenerkrankungen.

## Claims

1. Compounds of the general formula (I) 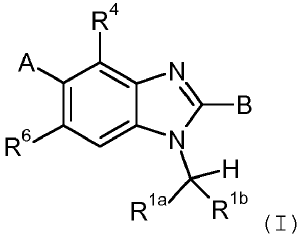 in which
A represents hydrogen, bromine, cyano, formyl, C₁-C₃-alkyl, 4- to 6-membered heterocyclyl, 5- to 6-membered heteroaryl, R¹¹O-C(=O)-(CH₂)ₚ-, R⁵R^{5'}N-C(=O)-(CH₂)ₚ-, R¹¹O-S(=O)₂-(CH₂)ₚ-, R⁵R^{5'}N-S(=O)₂-(CH₂)ₚ-, (C₁-C₆-alkyl)-S(=O)₂-, (C₁-C₆-alkyl)-S(=O)(=NH)- or
(C₃-C₆-cycloalkyl) -S(=O) (=NH)-,
where heteroaryl is preferably selected from the group consisting of pyrazolyl, imidazolyl, triazolyl, tetrazolyl, oxazolyl, isoxazolyl and oxadiazolyl,
and
where alkyl radicals may optionally be mono- or polysubstituted by identical or different halogen or hydroxy substituents,
and
where heterocyclyl radicals may optionally be mono- or polysubstituted by identical or different C₁-C₃-alkyl, trifluoromethyl or hydroxy substituents,
and
where heteroaryl radicals may optionally be mono- or polysubstituted by identical or different C₁-C₃-alkyl, trifluoromethyl or hydroxy substituents,
B is selected from the following structures 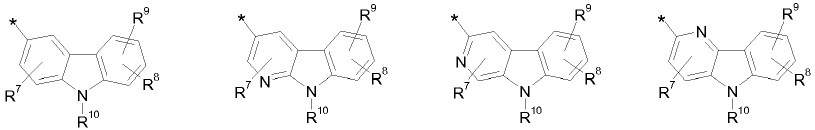 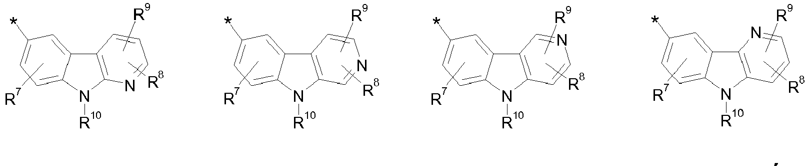 where * denotes the point of attachment in the molecule,
R^{1a}, R^{1b} independently of one another represent hydrogen, cyano, C₁-C₅-alkyl, C₂-C₅-alkenyl, C₂-C₅-alkynyl, (C₃-C₆-cycloalkyl) - (CH₂)ₘ-, (4- to 6-membered heterocyclyl)-(CH₂)ₙ-, (C₁-C₅-alkoxy)-(C₁-C₃-alkyl)-, (C₃-C₆-cycloalkoxy)-(C₁-C₃-alkyl)-, H₂N-(C₁-C₃-alkyl)-, (C₁-C₅-alkyl)NH-(C₁-C₃-alkyl)- or (C₁-C₅-alkyl)₂N-(C₁-C₃-alkyl)-,
where heterocyclyl is preferably selected from the group consisting of oxetanyl, tetrahydrofuranyl, tetrahydropyranyl, 1,4-dioxanyl, morpholinyl, azetidinyl, pyrrolidinyl, piperazinyl and piperidinyl,
and
where alkyl radicals, cycloalkyl radicals and heterocyclyl radicals may optionally be mono- or polysubstituted by identical or different halogen, C₁-C₅-alkyl, hydroxy, C(=O)OH, HO-C(=O)-(C₁-C₅-alkyl) -, (C₁-C₅-alkyl)O-C(=O)-(C₁-C₅-alkyl) - or (C₁-C₅-alkyl)-S(=O)₂- substituents,
R⁴ represents hydrogen, fluorine, chlorine, C₁-C₂-alkyl, C₃-C₅-cycloalkyl, C₁-C₂-alkoxy or (C₃-C₄-cycloalkyl)-CH₂-,
where alkyl and cycloalkyl radicals may optionally be mono- or polysubstituted by identical or different halogen or hydroxy substituents,
R⁵, R^{5'} independently of one another represent hydrogen, C₁-C₇-alkyl, (C₁-C₇-alkoxy)-(C₂-C₅-alkyl)-, (4- to 6-membered heterocyclyl)-(CH₂)ᵣ-, (C₁-C₇-alkyl)-C(=O)-, (C₃-C₇-cycloalkyl)-C(=O)-, phenyl-(CH₂)ᵣ-C(=O)-, pyridyl-(CH₂)ᵣ-C(=O)-, (C₁-C₇-alkyl)-S(=O)₂-, (C₃-C₇-cycloalkyl)-S(=O)₂-, phenyl-(CH₂)ᵣ-S(=O)₂- or pyridyl-(CH₂)ᵣ-S(=O)₂-,
where heterocyclyl is preferably selected from the group consisting of oxetanyl, tetrahydrofuranyl, tetrahydropyranyl, 1,4-dioxanyl, morpholinyl, azetidinyl, pyrrolidinyl, piperazinyl and piperidinyl,
and
where R⁵ and R^{5'} independently of one another may be mono- or polysubstituted by identical or different halogen, hydroxy, C₁-C₂-alkyl, trifluoromethyl, (C₁-C₅-alkyl)NH-, (C₁-C₅-alkyl)₂N-, C₁-C₂-alkoxy or trifluoromethoxy substituents,
or
R⁵, R^{5'} together with the nitrogen atom to which they are attached form a 4- to 6-membered heterocyclic ring which may optionally be mono- or polysubstituted by identical or different oxo, hydroxy, carboxy, C₁-C₂-alkyl or
C₁-C₂-alkoxy substituents,
where a 6-membered heterocyclic ring may optionally contain, as further ring atom, a heteroatom selected from the group consisting of 0 and N,
R⁶ represents hydrogen, fluorine, chlorine, methyl, trifluoromethyl, methoxy or trifluoromethoxy,
R⁷ represents hydrogen, fluorine, chlorine, cyano, SF₅, C₁-C₃-alkyl, C₃-C₅-cycloalkyl, C₁-C₂-alkoxy or (C₃-C₄-cycloalkyl)-CH₂-,
where alkyl and cycloalkyl radicals may optionally be mono- or polysubstituted by identical or different halogen radicals,
R⁸ represents fluorine, chlorine, bromine, cyano, SF₅, C₁-C₃-alkyl, C₃-C₅-cycloalkyl, C₁-C₂-alkoxy or (C₃-C₄-cycloalkyl)-CH₂-,
where alkyl and cycloalkyl radicals may optionally be mono- or polysubstituted by identical or different halogen radicals,
R⁹ represents fluorine, chlorine, bromine, cyano, C₁-C₃-alkyl or C₁-C₂-alkoxy,
where alkyl radicals may optionally be mono- or polysubstituted by identical or different halogen substituents,
or
R⁹ represents bromine and simultaneously R⁸ represents hydrogen,
R¹⁰ represents C₁-C₅-alkyl, C₃-C₅-alkenyl, C₃-C₅-alkynyl, (C₃-C₆-cycloalkyl)-(CH₂)ₙ-, (4- to 6-membered heterocyclyl)-(CH₂)ₙ- or (C₁-C₇-alkoxy)-(C₂-C₅-alkyl)-,
where heterocyclyl is preferably selected from the group consisting of oxetanyl, tetrahydrofuranyl, tetrahydropyranyl, morpholinyl, pyrrolidinyl and piperidinyl
and
where alkyl radicals, cycloalkyl radicals and heterocyclyl radicals may optionally be mono- or polysubstituted by identical or different halogen, C₁-C₂-alkyl, C₁-C₂-alkoxy or C(=O)OH radicals,
R¹¹ represents hydrogen, C₁-C₇-alkyl, C₃-C₇-cycloalkyl, phenyl-(CH₂)_{q}- or (C₁-C₇-alkoxy)-(C₂-C₅-alkyl)-,
where phenyl may optionally be mono- or polysubstituted by identical or different C₁-C₃-alkyl, trifluoromethyl or hydroxy radicals,
m is 0, 1, 2 or 3,
n is 0, 1, 2 or 3,
p is 0, 1 or 2,
q is 1, 2 or 3 and
r is 0, 1, 2 or 3,
and diastereomers, enantiomers, solvates and salts or cyclodextrin clathrates thereof.

2. Compounds according to Claim 1, where
A represents cyano, C₁-C₃-alkyl, 5-membered heterocyclyl, 5-membered heteroaryl, R¹¹O-C(=O)-(CH₂)ₚ-, R⁵R^{5'}N-C(=O)-(CH₂)ₚ- or R⁵R^{5'}N-S(=O)₂-(CH₂)ₚ-,
where heteroaryl is preferably selected from the group consisting of triazolyl, tetrazolyl and oxadiazolyl,
and
where alkyl radicals may optionally be mono- or polysubstituted by identical or different hydroxy substituents,
and
where heteroaryl radicals may optionally be mono- or polysubstituted by identical or different C₁-C₃-alkyl, trifluoromethyl or hydroxy substituents,
B represents a group 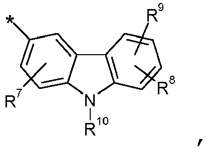 where * denotes the point of attachment in the molecule,
R^{1a} represents hydrogen or C₁-C₅-alkyl,
R^{1b} represents hydrogen, C₁-C₅-alkyl, C₂-C₅-alkenyl, (C₃-C₆-cycloalkyl) - (CH₂)ₘ-, (4- to 6-membered heterocyclyl)-(CH₂)ₙ-, (C₁-C₅-alkoxy)-(C₁-C₃-alkyl)- or (C₁-C₅-alkyl)₂N-(C₁-C₃-alkyl)-,
where heterocyclyl is preferably selected from the group consisting of oxetanyl, tetrahydrofuranyl, 1,4-dioxanyl, morpholinyl and pyrrolidinyl,
and
where alkyl radicals and cycloalkyl radicals may optionally be mono- or polysubstituted by identical or different C₁-C₅-alkyl, hydroxy or (C₁-C₅-alkyl)-S(O)₂- radicals,
R⁴ represents hydrogen, fluorine, chlorine, C₁-C₂-alkyl or C₁-C₂-alkoxy,
R⁵, R^{5'} independently of one another represent hydrogen, C₁-C₇-alkyl, (4- to 6-membered heterocyclyl) - (CH₂)ᵣ-, (C₁-C₇-alkyl)-S(O)₂-, (C₃-C₇-cycloalkyl)-S(O)₂-, phenyl-(CH₂)ᵣ-S(O)₂- or pyridyl-(CH₂)ᵣ-S(O)₂-,
where heterocyclyl is preferably selected from the group consisting of morpholinyl and pyrrolidinyl,
and
where R⁵ and R^{5'} independently of one another may be mono- or polysubstituted by identical or different halogen, C₁-C₂-alkyl, trifluoromethyl, (C₁-C₅-alkyl)₂N-, C₁-C₂-alkoxy or trifluoromethoxy substituents,
or
R⁵, R^{5'} together with the nitrogen atom to which they are attached form a 4- to 6-membered heterocyclic ring which may optionally be substituted by oxo or hydroxy,
where a 6-membered heterocyclic ring may optionally contain, as a further ring atom, an oxygen atom,
R⁶ represents hydrogen, fluorine, methyl or methoxy,
R⁷ represents hydrogen, fluorine, chlorine, C₁-C₃-alkyl or C₁-C₂-alkoxy,
R⁸ represents fluorine, chlorine, bromine, C₁-C₃-alkyl or C₁-C₂-alkoxy,
R⁹ represents fluorine, chlorine, bromine, C₁-C₃-alkyl or C₁-C₂-alkoxy,
or
R⁹ represents bromine and simultaneously R⁸ represents hydrogen,
R¹⁰ represents C₁-C₅-alkyl, C₃-C₅-alkenyl, C₃-C₅-alkynyl, (C₃-C₆-cycloalkyl)- (CH₂)ₙ- or (C₁-C₇-alkoxy)-(C₂-C₅-alkyl) -,
R¹¹ represents hydrogen or C₁-C₇-alkyl,
m is 0 or 1,
n is 0 or 1,
p is 0 and
r is 0, 1 or 2,
and diastereomers, enantiomers, solvates and salts or cyclodextrin clathrates thereof.

3. Compounds according to Claim 1, where
A represents 5-membered heteroaryl, R¹¹O-C(=O)-(CH₂)ₚ-, R⁵R^{5'}N-C(=O)-(CH₂)ₚ- or R⁵R^{5'}N-S(=O)₂-(CH₂)ₚ-,
where heteroaryl is preferably selected from the group consisting of triazolyl, tetrazolyl and oxadiazolyl,
and
where heteroaryl radicals may optionally be mono- or polysubstituted by identical or different C₁-C₃-alkyl, trifluoromethyl or hydroxy substituents,
B represents a group 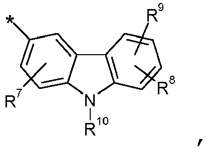 where * denotes the point of attachment in the molecule,
R^{1a} represents hydrogen or methyl,
R^{1b} represents hydrogen, C₁-C₂-alkyl, vinyl, cyclopropyl- (CH₂)ₘ-, methoxy-(C₁-C₂-alkyl)-or (N,N-dimethylamino)methyl,
where alkyl radicals and cycloalkyl radicals may optionally be mono- or polysubstituted by identical or different methyl, hydroxy or methylsulphonyl substituents,
R⁴ represents hydrogen, fluorine, chlorine, methyl or methoxy,
R⁵, R^{5'} independently of one another represent hydrogen, C₁-C₂-alkyl or (5- or 6-membered heterocyclyl)-(CH₂)ᵣ-,
where heterocyclyl is preferably selected from the group consisting of morpholinyl and pyrrolidinyl,
and
where R⁵ and R^{5'} independently of one another may optionally be mono- or polysubstituted by identical or different chlorine, fluorine, methyl, trifluoromethyl, N,N-dimethylamino, methoxy or trifluoromethoxy substituents,
or
R⁵, R^{5'} together with the nitrogen atom to which they are attached form a 4- to 6-membered heterocyclic ring which may optionally be substituted by oxo or hydroxy,
where a 6-membered heterocyclic ring may optionally contain, as a further ring atom, an oxygen atom,
R⁶ represents hydrogen, fluorine, methyl or methoxy,
R⁷ represents hydrogen, fluorine, chlorine, methyl or methoxy,
R⁸ represents fluorine, chlorine, bromine, methyl or methoxy,
R⁹ represents fluorine, chlorine, bromine, methyl or methoxy,
or
R⁹ represents bromine and simultaneously R⁸ represents hydrogen,
R¹⁰ represents C₁-C₃-alkyl, allyl, propargyl, (C₃-C₄-CyCloalkyl)-(CH₂)ₙ- or methoxyethyl,
R¹¹ represents hydrogen or C₁-C₃-alkyl,
m is 0 or 1,
n is 0 or 1,
p is 0 and
r is 0, 1 or 2,
and diastereomers, enantiomers, solvates and salts or cyclodextrin clathrates thereof.

4. Compounds according to Claim 1, where
A represents R¹¹O-C(=O)-(CH₂)ₚ-,
B represents a group 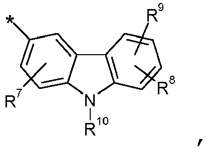 where * denotes the point of attachment in the molecule,
R^{1a} represents hydrogen,
R^{1b} represents methoxymethyl,
R⁴ represents hydrogen, fluorine or methyl,
R⁶ represents hydrogen,
R⁷ represents hydrogen,
R⁸ represents fluorine, chlorine or methyl,
R⁹ represents fluorine, chlorine, bromine or methyl,
or
R⁹ represents bromine and simultaneously R⁸ represents hydrogen,
R¹⁰ represents ethyl,
R¹¹ represents hydrogen, methyl or ethyl, and
p is 0,
and diastereomers, enantiomers, solvates and salts or cyclodextrin clathrates thereof.

5. Compound according to Claim 1, selected from a group comprising the following compounds:
1. methyl 2-(6-bromo-9-ethyl-9H-carbazol-3-yl)-1-(2-methoxyethyl)-4-methyl-1H-benzimidazole-5-carboxylate;
2. 2-(6-bromo-9-ethyl-9H-carbazol-3-yl)-1-(2-methoxyethyl)-4-methyl-1H-benzimidazole-5-carboxylic acid;
3. methyl 2-(9-ethyl-8-fluoro-6-methyl-9H-carbazol-3-yl)-1-(2-methoxyethyl)-4-methyl-1H-benzimidazole-5-carboxylate;
4. 2-(9-ethyl-8-fluoro-6-methyl-9H-carbazol-3-yl)-1-(2-methoxyethyl)-4-methyl-1H-benzimidazole-5-carboxylic acid;
5. methyl 2-(9-ethyl-6-fluoro-8-methyl-9H-carbazol-3-yl)-1-(2-methoxyethyl)-4-methyl-1H-benzimidazole-5-carboxylate;
6. 2-(9-ethyl-6-fluoro-8-methyl-9H-carbazol-3-yl)-1-(2-methoxyethyl)-4-methyl-1H-benzimidazole-5-carboxylic acid;
7. 2-(9-ethyl-6-fluoro-8-methyl-9H-carbazol-3-yl)-4-fluoro-1-(2-methoxyethyl)-1H-benzimidazole-5-carboxylic acid;
8. 2-(9-ethyl-8-fluoro-6-methyl-9H-carbazol-3-yl)-4-fluoro-1-(2-methoxyethyl)-1H-benzimidazole-5-carboxylic acid;
9. ethyl 2-(9-ethyl-8-fluoro-6-methyl-9H-carbazol-3-yl)-1-(2-methoxyethyl)-1H-benzimidazole-5-carboxylate;
10. 2-(9-ethyl-8-fluoro-6-methyl-9H-carbazol-3-yl)-1-(2-methoxyethyl)-1H-benzimidazole-5-carboxylic acid;
11. methyl 2-(8-chloro-9-ethyl-6-methyl-9H-carbazol-3-yl)-1-(2-methoxyethyl)-4-methyl-1H-benzimidazole-5-carboxylate;
12. 2-(8-chloro-9-ethyl-6-methyl-9H-carbazol-3-yl)-1-(2-methoxyethyl)-4-methyl-1H-benzimidazole-5-carboxylic acid;
13. 2-(5-chloro-9-ethyl-6-methyl-9H-carbazol-3-yl)-1-(2-methoxyethyl)-4-methyl-1H-benzimidazole-5-carboxylic acid.

6. Compound of the formula (I) as defined in any of Claims 1 to 5 for the treatment and/or prophylaxis of diseases.

7. Compound of the formula (I) as defined in any of Claims 1 to 5 for use in a method for the treatment and/or prophylaxis of endometriosis, of uterine leiomyomas, of uterine menstrual complaints, where the menstrual complaints may be heavy and prolonged menorrhoea, irregular menorrhoea and pain, of dysmenorrhoea, of cancer, where the cancer may be lung cancer, cancer of the intestine, breast cancer, skin cancer, prostate cancer, cancer of the oesophagus and leukaemia, of arteriosclerosis and of polycystic kidney disorders.

8. Use of a compound according to Claim 1, 2, 3, 4 or 5 for production of a medicament for treatment and/or prophylaxis of diseases.

9. Use of a compound as defined in any of Claims 1 to 5 for production of a medicament for the treatment and/or prophylaxis of endometriosis, of uterine leiomyomas, of uterine menstrual complaints, where the menstrual complaints may be heavy and prolonged menorrhoea, irregular menorrhoea and pain, of dysmenorrhoea, of cancer, where the cancer may be lung cancer, cancer of the intestine, breast cancer, skin cancer, prostate cancer, cancer of the oesophagus and leukaemia, of arteriosclerosis and of polycystic kidney disorders.

10. Medicament comprising a compound as defined in any of Claims 1 to 5 in combination with one or more further active compounds, especially with selective oestrogen receptor modulators (SERMs), oestrogen receptor (ER) antagonists, aromatase inhibitors, 17-β-HSD1 inhibitors, steroid sulphatase (STS) inhibitors, GnRH agonists and antagonists, kisspeptin receptor (KISSR) antagonists, selective androgen receptor modulators (SARMs), androgens, 5α-reductase inhibitors, selective progesterone receptor modulators (SPRMs), gestagens, antigestagens, oral contraceptives, inhibitors of mitogen-activated protein (MAP) kinases and inhibitors of the MAP kinases (Mkk3/6, Mek1/2, Erk1/2), inhibitors of protein kinase B (PKBα/β/γ; Akt1/2/3), inhibitors of phosphoinositide 3-kinase (PI3K), inhibitors of cyclin-dependent kinase (CDK1/2), inhibitors of the hypoxia-induced signalling pathway (HIF1alpha inhibitors, activators of prolylhydroxylases), histone deacetylase (HDAC) inhibitors, prostaglandin F receptor (FP) (PTGFR) antagonists, neurokinin 1 receptor antagonists, paracetamol, selective COX2 inhibitors and/or non-selective COX1/COX2 inhibitors.

11. Medicament comprising a compound of the formula (I) as defined in any of Claims 1 to 5 in combination with an inert, nontoxic, pharmaceutically suitable auxiliary.

12. Medicament according to Claim 10 or 11 for the treatment and/or prophylaxis of endometriosis, of uterine leiomyomas, of uterine menstrual complaints, where the menstrual complaints may be heavy and prolonged menorrhoea, irregular menorrhoea and pain, of dysmenorrhoea, of cancer, where the cancer may be lung cancer, cancer of the intestine, breast cancer, skin cancer, prostate cancer, cancer of the oesophagus and leukaemia, of arteriosclerosis and of polycystic kidney disorders.

## Revendications

1. Composés de formule générale (I) 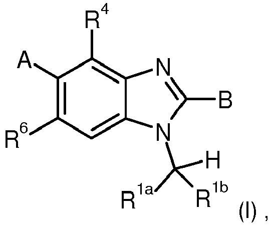 où
A représente hydrogène, brome, cyano, formyle, C₁-C₃-alkyle, hétérocyclyle de 4-6 chaînons, hétéroaryle de 5-6 chaînons, R¹¹O-C(=O)-(CH₂)ₚ-, R⁵R^{5'}N-C(=O)-(CH₂)ₚ-, R¹¹O-S(=O) ₂- (CH₂)ₚ-, R⁵R^{5'}N-S(=O)₂-(CH₂)ₚ-, (C₁-C₆-alkyl)-S(=O)₂-, (C₁-C₆-alkyl)-S(=O)(=NH)- ou (C₃-C₆-cycloalkyl)-S(=O)(=NH)-,
hétéroaryle étant de préférence choisi dans le groupe constitué par pyrazolyle, imidazolyle, triazolyle, tétrazolyle, oxazolyle, isoxazolyle et oxadiazolyle et
les radicaux alkyle pouvant le cas échéant être monosubstitués ou polysubstitués, de manière identique ou différente, par halogène ou hydroxy et
les radicaux hétérocyclyle pouvant le cas échéant être monosubstitués ou polysubstitués, de manière identique ou différente, par C₁-C₃-alkyle, trifluorométhyle ou hydroxy et
les radicaux hétéroaryle pouvant le cas échéant être monosubstitués ou polysubstitués, de manière identique ou différente, par C₁-C₃-alkyle, trifluorométhyle ou hydroxy
B est choisi parmi les structures suivantes, 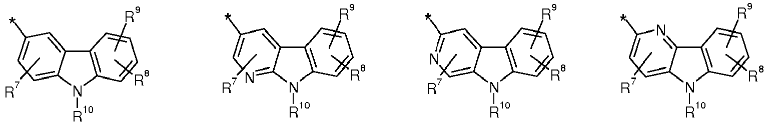 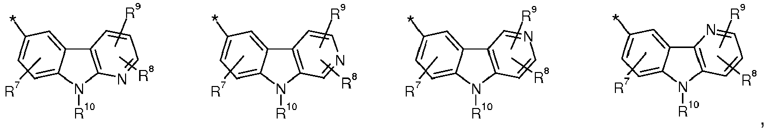 dans laquelle * représente le site de liaison dans la molécule,
R^{1a}, R^{1b} représentent, indépendamment l'un de l'autre, hydrogène, cyano, C₁-C₅-alkyle, C₂-C₅-alcényle, C₂-C₅-alcynyle, (C₃-C₆-cycloalkyl)-(CH₂)ₘ-, (hétérocyclyle de 4-6 chaînons)-(CH₂)ₙ-, (C₁-C₅-alcoxy)-(C₁-C₃-alkyl)-, (C₃-C₆-cycloalcoxy)-(C₁-C₃-alkyl)-, H₂N-(C₁-C₃-alkyl)-, (C₁-C₅-alkyl)NH-(C₁-C₃-alkyl)- ou (C₁-C₅-alkyl)₂N-(C₁-C₃-alkyl)-,
hétérocyclyle étant de préférence choisi dans le groupe constitué par oxétanyle, tétrahydrofurannyle, tétrahydropyrannyle, 1,4-dioxanyle, morpholinyle, azétidinyle, pyrrolidinyle, pipérazinyle et pipéridinyle et
les radicaux alkyle, cycloalkyle et hétérocyclyle pouvant le cas échéant être monosubstitués ou polysubstitués, de manière identique ou différente, par halogène, C₁-C₅-alkyle, hydroxy, C(=O)OH, HO-C(=O)-(C₁-C₅-alkyl)-, (C₁-C₅-alkyl)O-C(=O)-(C₁-C₅-alkyl)- ou (C₁-C₅-alkyl)-S(=O)₂-,
R⁴ représente hydrogène, fluor, chlore, C₁-C₂-alkyle, C₃-C₅-cycloalkyle, C₁-C₂-alcoxy ou (C₃-C₄-cycloalkyl)-CH₂-,
les radicaux alkyle et cycloalkyle pouvant le cas échéant être monosubstitués ou polysubstitués, de manière identique ou différente, par halogène ou hydroxy,
R⁵, R^{5'} représentent, indépendamment l'un de l'autre, hydrogène, C₁-C₇-alkyle, (C₁-C₇-alcoxy)-(C₂-C₅-alkyl)-, (hétérocyclyle de 4-6 chaînons) - (CH₂)ᵣ-, (C₁-C₇-alkyl)-C(=O)-, (C₃-C₇-cycloalkyl)-C(=O)-, phényl-(CH₂)ᵣ-C(=O)-, pyridyl-(CH₂)ᵣ-C(=O)-, (C₁-C₇-alkyl)-S(=O)₂-, (C₃-C₇-cycloalkyl)-S(=O)₂-, phényl-(CH₂)ᵣ-S(=O)₂- ou pyridyl-(CH₂)ᵣ-S(=O)₂-,
hétérocyclyle étant de préférence choisi dans le groupe constitué par oxétanyle, tétrahydrofurannyle, tétrahydropyrannyle, 1,4-dioxanyle, morpholinyle, azétidinyle, pyrrolidinyle, pipérazinyle et pipéridinyle et
R⁵ et R^{5'}, indépendamment l'un de l'autre, pouvant le cas échéant être monosubstitués ou polysubstitués, de manière identique ou différente, par halogène, hydroxy, C₁-C₂-alkyle, trifluorométhyle, (C₁-C₅-alkyl)NH-, (C₁-C₅-alkyl)₂N-, C₁-C₂-alcoxy ou trifluorométhoxy ou
R⁵, R^{5'} ensemble avec l'atome d'azote auquel ils sont liés, forment un cycle hétérocyclique de 4-6 chaînons, qui peut éventuellement être monosubstitué ou polysubstitué, de manière identique ou différente, par oxo, hydroxy, carboxy, C₁-C₂-alkyle ou C₁-C₂-alcoxy,
un cycle hétérocyclique de 6 chaînons pouvant éventuellement contenir, comme autre atome de cycle, un hétéroatome qui est choisi dans le groupe constitué par 0 et N,
R⁶ représente hydrogène, fluor, chlore, méthyle, trifluorométhyle, méthoxy ou trifluorométhoxy,
R⁷ représente hydrogène, fluor, chlore, cyano, SF₅, C₁-C₃-alkyle, C₃-C₅-cycloalkyle, C₁-C₂-alcoxy ou (C₃-C₄-cycloalkyl)-CH₂-,
les radicaux alkyle et cycloalkyle pouvant le cas échéant être monosubstitués ou polysubstitués, de manière identique ou différente, par halogène,
R⁸ représente fluor, chlore, brome, cyano, SF₅, C₁-C₃-alkyle, C₃-C₅-cycloalkyle, C₁-C₂-alcoxy ou (C₃-C₄-cycloalkyl)-CH₂-, les radicaux alkyle et cycloalkyle pouvant le cas échéant être monosubstitués ou polysubstitués, de manière identique ou différente, par halogène,
R⁹ représente fluor, chlore, brome, cyano, C₁-C₃-alkyle ou C₁-C₂-alcoxy,
les radicaux alkyle pouvant le cas échéant être monosubstitués ou polysubstitués, de manière identique ou différente, par halogène ou
R⁹ représente brome et R⁸ représente simultanément hydrogène,
R¹⁰ représente C₁-C₅-alkyle, C₃-C₅-alcényle, C₃-C₅-alcynyle, (C₃-C₆-cycloalkyl)-(CH₂)ₙ-, (hétérocyclyle de 4-6 chaînons)- (CH₂)ₙ-, ou (C₁-C₇-alcoxy)-(C₂-C₅-alkyl)-,
hétérocyclyle étant de préférence choisi dans le groupe constitué par oxétanyle, tétrahydrofurannyle, tétrahydropyrannyle, morpholinyle, pyrrolidinyle et pipéridinyle et
les radicaux alkyle, cycloalkyle et hétérocyclyle pouvant le cas échéant être monosubstitués ou polysubstitués, de manière identique ou différente, par halogène, C₁-C₂-alkyle, C₁-C₂-alcoxy ou C(=O)OH,
R¹¹ représente hydrogène, C₁-C₇-alkyle, C₃-C₇-cycloalkyle, phényl-(CH₂)_{q}- ou (C₁-C₇-alcoxy)-(C₂-C₅-alkyl)-,
phényle pouvant le cas échéant être monosubstitué ou polysubstitué, de manière identique ou différente, par C₁-C₃-alkyle, trifluorométhyle ou hydroxy,
m représente 0, 1, 2 ou 3,
n représente 0, 1, 2 ou 3,
p représente 0, 1 ou 2,
q représente 1, 2 ou 3 et
r représente 0, 1, 2 ou 3,
ainsi que leurs diastéréo-isomères, énantiomères, solvates et sels ou clathrates de cyclodextrine.

2. Composés selon la revendication 1, où
A représente cyano, C₁-C₃-alkyle, hétérocyclyle de 5 chaînons, hétéroaryle de 5 chaînons, R¹¹O-C(=O)-(CH₂)ₚ-, R⁵R^{5'}N-C(=O)-(CH₂)ₚ- ou R⁵R^{5'}N-S(=O)₂-(CH₂)ₚ-, hétéroaryle étant de préférence choisi dans le groupe constitué par triazolyle, tétrazolyle et oxazolyle et
les radicaux alkyle pouvant le cas échéant être monosubstitués ou polysubstitués, de manière identique ou différente, par hydroxy et
les radicaux hétéroaryle pouvant le cas échéant être monosubstitués ou polysubstitués, de manière identique ou différente, par C₁-C₃-alkyle, trifluorométhyle ou hydroxy
B représente un groupe 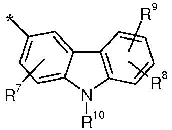 dans lequel * représente le site de liaison dans la molécule,
R^{1a} représente hydrogène ou C₁-C₅-alkyle,
R^{1b} représente hydrogène, C₁-C₅-alkyle, C₂-C₅-alcényle, (C₃-C₆-cycloalkyl) - (CH₂)ₘ-, (hétérocyclyle de 4-6 chaînons) - (CH₂)ₙ-, (C₁-C₅-alcoxy)-(C₁-C₃-alkyl)- ou (C₁-C₅-alkyl)₂N-(C₁-C₃-alkyl)-,
hétérocyclyle étant de préférence choisi dans le groupe constitué par oxétanyle, tétrahydrofurannyle, 1,4-dioxanyle, morpholinyle et pyrrolidinyle et
les radicaux alkyle et cycloalkyle pouvant le cas échéant être monosubstitués ou polysubstitués, de manière identique ou différente, par C₁-C₅-alkyle, hydroxy ou (C₁-C₅-alkyl)-S(O)₂- et
R⁴ représente hydrogène, fluor, chlore, C₁-C₂-alkyle, ou C₁-C₂-alcoxy,
R⁵, R^{5'} représentent, indépendamment l'un de l'autre, hydrogène, C₁-C₇-alkyle, (hétérocyclyle de 4-6 chaînons)-(CH₂)ᵣ-, (C₁-C₇-alkyl)-S(O)₂-, (C₃-C₇-cycloalkyl)-S(O)₂-, phényl-(CH₂)ᵣ-S(O)₂- ou pyridyl-(CH₂)ᵣ-S(O)₂-,
hétérocyclyle étant de préférence choisi dans le groupe constitué par morpholinyle et pyrrolidinyle et
R⁵ et R^{5'}, indépendamment l'un de l'autre, pouvant le cas échéant être monosubstitués ou polysubstitués, de manière identique ou différente, par halogène, C₁-C₂-alkyle, trifluorométhyle, (C₁-C₅-alkyl)₂N-, C₁-C₂-alcoxy ou trifluorométhoxy ou
R⁵, R^{5'} ensemble avec l'atome d'azote auquel ils sont liés, forment un cycle hétérocyclique de 4-6 chaînons, qui peut éventuellement être monosubstitué par oxo ou hydroxy,
un cycle hétérocyclique de 6 chaînons pouvant éventuellement contenir, comme autre atome de cycle, un atome d'oxygène,
R⁶ représente hydrogène, fluor, méthyle ou méthoxy,
R⁷ représente hydrogène, fluor, chlore, C₁-C₃-alkyle, ou C₁-C₂-alcoxy,
R⁸ représente fluor, chlore, brome, C₁-C₃-alkyle ou C₁-C₂-alcoxy,
R⁹ représente fluor, chlore, brome, C₁-C₃-alkyle ou C₁-C₂-alcoxy ou
R⁹ représente brome et R⁸ représente simultanément hydrogène,
R¹⁰ représente C₁-C₅-alkyle, C₃-C₅-alcényle, C₃-C₅-alcynyle, (C₃-C₆-cycloalkyl)-(CH₂)ₙ- ou (C₁-C₇-alcoxy)-(C₂-C₅-alkyl)-,
R¹¹ représente hydrogène ou C₁-C₇-alkyle,
m représente 0 ou 1,
n représente 0 ou 1,
p représente 0 et
r représente 0, 1 ou 2,
ainsi que leurs diastéréo-isomères, énantiomères, solvates et sels ou clathrates de cyclodextrine.

3. Composés selon la revendication 1, où
A représente hétéroaryle de 5 chaînons, R¹¹O-C(=O)-(CH₂)ₚ-, R⁵R^{5'}N-C(=O)-(CH₂)ₚ- ou R⁵R^{5'}N-S(=O)₂-(CH₂)ₚ-, hétéroaryle étant de préférence choisi dans le groupe constitué par triazolyle, tétrazolyle et oxazolyle et
les radicaux hétéroaryle pouvant le cas échéant être monosubstitués ou polysubstitués, de manière identique ou différente, par C₁-C₃-alkyle, trifluorométhyle ou hydroxy,
B représente un groupe 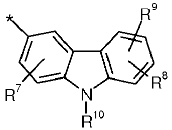 dans lequel * représente le site de liaison dans la molécule,
R^{1a} représente hydrogène ou méthyle,
R^{1b} représente hydrogène, C₁-C₂-alkyle, vinyle, cyclopropyl-(CH₂)ₘ-, méthoxy-(C₁-C₂-alkyl)- ou (N,N-diméthylamino)méthyl-,
les radicaux alkyle et cycloalkyle pouvant le cas échéant être monosubstitués ou polysubstitués, de manière identique ou différente, par méthyle, hydroxy ou méthylsulfonyle,
R⁴ représente hydrogène, fluor, chlore, méthyle ou méthoxy,
R⁵, R^{5'} représentent, indépendamment l'un de l'autre, hydrogène, C₁-C₂-alkyle ou (hétérocyclyle de 5-6 chaînons)-(CH₂)ᵣ -,
hétérocyclyle étant de préférence choisi dans le groupe constitué par morpholinyle et pyrrolidinyle et
R⁵ et R^{5'}, indépendamment l'un de l'autre, pouvant le cas échéant être monosubstitués ou polysubstitués, de manière identique ou différente, par chlore, fluor, méthyle, trifluorométhyle, N,N-diméthylamino, méthoxy ou trifluorométhoxy ou
R⁵, R^{5'} ensemble avec l'atome d'azote auquel ils sont liés, forment un cycle hétérocyclique de 4-6 chaînons, qui peut éventuellement être monosubstitué par oxo ou hydroxy,
un cycle hétérocyclique de 6 chaînons pouvant éventuellement contenir, comme autre atome de cycle, un atome d'oxygène,
R⁶ représente hydrogène, fluor, méthyle ou méthoxy,
R⁷ représente hydrogène, fluor, chlore, méthyle ou méthoxy,
R⁸ représente fluor, chlore, brome, méthyle ou méthoxy,
R⁹ représente fluor, chlore, brome, méthyle ou méthoxy ou
R⁹ représente brome et R⁸ représente simultanément hydrogène,
R¹⁰ représente C₁-C₃-alkyle, allyle, propargyle, (C₃-C₄-cycloalkyl)-(CH₂)ₙ- ou méthoxyéthyle,
R¹¹ représente hydrogène ou C₁-C₃-alkyle,
m représente 0 ou 1,
n représente 0 ou 1,
p représente 0 et
r représente 0, 1 ou 2,
ainsi que leurs diastéréo-isomères, énantiomères, solvates et sels ou clathrates de cyclodextrine.

4. Composés selon la revendication 1, où
A représente R¹¹O-C(=O)-(CH₂)ₚ-,
B représente un groupe 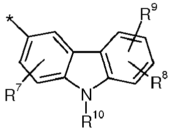 dans lequel * représente le site de liaison dans la molécule,
R^{1a} représente hydrogène,
R^{1b} représente méthoxyméthyle,
R⁴ représente hydrogène, fluor ou méthyle,
R⁶ représente hydrogène,
R⁷ représente hydrogène,
R⁸ représente fluor, chlore ou méthyle,
R⁹ représente fluor, chlore, brome ou méthyle ou
R⁹ représente brome et R⁸ représente simultanément hydrogène,
R¹⁰ représente éthyle,
R¹¹ représente hydrogène, méthyle ou éthyle et
p représente 0,
ainsi que leurs diastéréo-isomères, énantiomères, solvates et sels ou clathrates de cyclodextrine.

5. Composé selon la revendication 1, choisi dans un groupe qui contient les composés suivants :
1. 2-(6-bromo-9-éthyl-9H-carbazol-3-yl)-1-(2-méthoxyéthyl)-4-méthyl-1H-benzimidazole-5-carboxylate de méthyle ;
2. acide 2-(6-bromo-9-éthyl-9H-carbazol-3-yl)-1-(2-méthoxyéthyl)-4-méthyl-1H-benzimidazole-5-carboxylique ;
3. 2-(9-éthyl-8-fluoro-6-méthyl-9H-carbazol-3-yl)-1-(2-méthoxyéthyl)-4-méthyl-1H-benzimidazole-5-carboxylate de méthyle ;
4. acide 2-(9-éthyl-8-fluoro-6-méthyl-9H-carbazol-3-yl)-1-(2-méthoxyéthyl)-4-méthyl-1H-benzimidazole-5-carboxylique ;
5. 2-(9-éthyl-6-fluoro-8-méthyl-9H-carbazol-3-yl)-1-(2-méthoxyéthyl)-4-méthyl-1H-benzimidazole-5-carboxylate de méthyle ;
6. acide 2-(9-éthyl-6-fluoro-8-méthyl-9H-carbazol-3-yl)-1-(2-méthoxyéthyl)-4-méthyl-1H-benzimidazole-5-carboxylique ;
7. acide 2-(9-éthyl-6-fluoro-8-méthyl-9H-carbazol-3-yl)-4-fluoro-1-(2-méthoxyéthyl)-1H-benzimidazole-5-carboxylique ;
8. acide 2-(9-éthyl-8-fluoro-6-méthyl-9H-carbazol-3-yl)-4-fluoro-1-(2-méthoxyéthyl)-1H-benzimidazole-5-carboxylique ;
9. 2-(9-éthyl-8-fluoro-6-méthyl-9H-carbazol-3-yl)-1-(2-méthoxyéthyl)-1H-benzimidazole-5-carboxylate d'éthyle ;
10. acide 2-(9-éthyl-8-fluoro-6-méthyl-9H-carbazol-3-yl)-1-(2-méthoxyéthyl)-1H-benzimidazole-5-carboxylique ;
11. 2-(8-chloro-9-éthyl-6-méthyl-9H-carbazol-3-yl)-1-(2-méthoxyéthyl)-4-méthyl-1H-benzimidazole-5-carboxylate de méthyle ;
12. acide 2-(8-chloro-9-éthyl-6-méthyl-9H-carbazol-3-yl)-1-(2-méthoxyéthyl)-4-méthyl-1H-benzimidazole-5-carboxylique ; et
13. acide 2-(5-chloro-9-éthyl-6-méthyl-9H-carbazol-3-yl)-1-(2-méthoxyéthyl)-4-méthyl-1H-benzimidazole-5-carboxylique.

6. Composé de formule (I), tel que défini dans l'une quelconque des revendications 1 à 5, pour le traitement et/ou la prophylaxie de maladies.

7. Composé de formule (I), tel que défini dans l'une quelconque des revendications 1 à 5, pour une utilisation dans un procédé pour le traitement et/ou la prophylaxie de l'endométriose, de léiomyomes utérins, de troubles hémorragiques utérins, les troubles hémorragiques pouvant être des saignements importants et longs, des saignements temporairement irréguliers ainsi que des douleurs, de la dysménorrhée, du cancer, les maladies cancéreuses pouvant être le cancer des poumons, de l'intestin, du sein, de la peau, de la prostate, de l'oesophage et la leucémie, de l'artériosclérose et de maladies rénales polykystiques.

8. Utilisation d'un composé selon la revendication 1, 2, 3, 4 ou 5 pour la préparation d'un médicament pour le traitement et/ou la prophylaxie de maladies.

9. Utilisation d'un composé, tel que défini dans l'une quelconque des revendications 1 à 5, pour la préparation d'un médicament pour le traitement et/ou la prophylaxie de l'endométriose, de léiomyomes utérins, de troubles hémorragiques utérins, les troubles hémorragiques pouvant être des saignements importants et longs, des saignements temporairement irréguliers ainsi que des douleurs, de la dysménorrhée, du cancer, les maladies cancéreuses pouvant être le cancer des poumons, de l'intestin, du sein, de la peau, de la prostate, de l'oesophage et la leucémie, de l'artériosclérose et de maladies rénales polykystiques.

10. Médicament contenant un composé, tel que défini dans l'une quelconque des revendications 1 à 5, en combinaison avec une ou plusieurs autres substances actives, en particulier avec des modulateurs sélectifs du récepteur des estrogènes (SERM), des antagonistes du récepteur des estrogènes (ER), des inhibiteurs d'aromatases, des inhibiteurs de 17-ß-HSD1, des inhibiteurs de la stéroïde sulfatase (STS), des agonistes et des antagonistes de GnRH, des antagonistes du récepteur de la kisspeptine (KISSR), des modulateurs sélectifs du récepteur des androgènes (SARM), des androgènes, des inhibiteurs de la 5α-réductase, des modulateurs sélectifs du récepteur des progestérones (SPRM), des gestagènes, des antigestagènes, des contraceptifs oraux, des inhibiteurs des protéine kinases activées par des agents mitogènes (MAP) ainsi que des inhibiteurs des MAP kinases (Mkk3/6, Mek1/2, Erk1/2), des inhibiteurs des protéine kinases B (PKBα/ß/γ ; Akt1/2/3), des inhibiteurs des phosphoinositide-3-kinases (PI3K), des inhibiteurs des kinases cycline-dépendantes (CDK1/2), des inhibiteurs de la voie de signalisation induite par l'hypoxie (inhibiteurs HIF1alpha, activateurs des prolylhydroxylases), des inhibiteurs de l'histone désacétylase (HDAC), des antagonistes du récepteur de la prostaglandine F (FP) (PTGFR), des antagonistes du récepteur de la neurokinine-1, le paracétamol, des inhibiteurs sélectifs de COX2 et/ou des inhibiteurs non sélectifs de COX1/COX2.

11. Médicament contenant un composé de formule (I), tel que défini dans l'une quelconque des revendications 1 à 5, en combinaison avec un adjuvant inerte, non toxique, pharmaceutiquement approprié.

12. Médicament selon la revendication 10 ou 11 pour le traitement et/ou la prophylaxie de l'endométriose, de léiomyomes utérins, de troubles hémorragiques utérins, les troubles hémorragiques pouvant être des saignements importants et longs, des saignements temporairement irréguliers ainsi que des douleurs, de la dysménorrhée, du cancer, les maladies cancéreuses pouvant être le cancer des poumons, de l'intestin, du sein, de la peau, de la prostate, de l'oesophage et la leucémie, de l'artériosclérose et de maladies rénales polykystiques.
